# EUROPEAN PATENT SPECIFICATION

(11) **EP 4 499 872 B1**
(45) Date of publication and mention of the grant of the patent: **29.04.2026**
(21) Application number: 23717758.9
(22) Date of filing: 27.03.2023
(51) Int. Cl.: C12Q 1/6874

(54) **SYSTEMS AND METHODS OF SEQUENCING POLYNUCLEOTIDES**
SYSTEME UND VERFAHREN ZUR SEQUENZIERUNG VON POLYNUKLEOTIDEN
SYSTÈMES ET PROCÉDÉS DE SÉQUENÇAGE DE POLYNUCLÉOTIDES

(30) Priority: 29.03.2022 US 202263325057 P
(43) Date of publication of application: 05.02.2025
(73) Proprietor: Illumina, Inc., San Diego, CA 92122 (US)
(72) Inventor: CALLINGHAM, Michael, Cambridge Cambridgeshire CB21 6DF (GB); BUREK, Michael, John, San Diego, California 92122 (US); WINNARD, Christopher, Cambridge Cambridgeshire CB21 6DF (GB); MILLER, Oliver, Jon, Cambridge Cambridgeshire CB21 6DF (GB)
(74) Representative: Robinson, David Edward Ashdown
(86) International application number: PCT/US2023/016388
(87) International publication number: WO 2023/192163

(56) References cited:
- WO-A1-2016/189287
- WO-A1-2018/060482
- WO-A1-2018/114710
- US-A1- 2020 080 142
- US-A1- 2022 049 292

## Description

### BACKGROUND

### Field

The present disclosure relates to DNA sequencing systems and methods. In particular, long Stokes shifted dyes may be used as nucleotide labels for nucleic acid sequencing applications in two-channel DNA sequencing systems and methods.

### Background

Existing DNA sequencing systems and methods may use two or more light sources to excite fluorescent labels conjugated to deoxyribonucleic acid analogs. Different fluorescent labels that are each designed to be efficiently excited by one light source may be subject to cross-talk whereby each label emits light at a wavelength that overlaps with other labels. When uncorrected, this cross-talk can make it difficult for DNA sequencing systems to properly call the correct nucleotide base during a sequencing run. Accordingly, for many biological applications, it is desirable to employ multiple spectrally-distinguishable fluorescent labels with a high enough signal to noise ratio (SNR) to achieve independent detection of a plurality of spatially-overlapping analytes.

However, multiplex fluorescent detection can be problematic and there are a number of important factors that constrain selection of appropriate fluorescent labels. First, it may be difficult to find dye compounds with substantially-resolved absorption and emission spectra in a given application. In addition, when several fluorescent dyes are used together, generating fluorescence signals in distinguishable spectral regions by simultaneous excitation may be complicated because absorption bands of the dyes are usually widely separated, so it is difficult to achieve comparable fluorescence excitation efficiencies even for two dyes. Many excitation methods use high power light sources like lasers and therefore the dye must have sufficient photo-stability to withstand such excitation. A final consideration of particular importance to molecular biology methods is the extent to which the fluorescent dyes must be compatible with reagent chemistries such as, for example, DNA synthesis solvents and reagents, buffers, polymerase enzymes, and ligase enzymes. US 2022/049292 and US 2020/080142 discloses methods for sequencing using multiplex fluorescent detection.

### SUMMARY

In some aspects, the techniques described herein relate to methods of sequencing clusters of labeled polynucleotides bound to a flowcell, including: exciting the clusters of labeled polynucleotides at a first excitation wavelength and detecting fluorescent emissions from the clusters at a first detection wavelength to detect the presence of a first labeled nucleotide; exciting the clusters of labeled polynucleotides at a second excitation wavelength and detecting fluorescent emissions from the clusters at a second detection wavelength to detect the presence of a second labeled nucleotide; exciting the clusters of labeled polynucleotides at a third excitation wavelength and detecting fluorescent emissions from the clusters at a third detection wavelength to detect the presence of a third labeled nucleotide; determining clusters that did not emit fluorescent emissions after excitation at the first, second or third excitation wavelengths to detect the presence of a fourth unlabeled nucleotide; and determining the nucleotide sequence of the labeled polynucleotides based on the detected fluorescent emissions.

With respect to certain two-camera or two-channel methods described herein, nucleic acids can be sequenced utilizing methods and systems described in U.S. Patent Application Publication No. 2013/0079232, As a first example, a nucleic acid can be sequenced by providing a first nucleotide type that is detected in a first channel, a second nucleotide type that is detected in a second channel, a third nucleotide type that is excited with the same wavelength as the first nucleotide type but detected in the second channel. Finally, a fourth nucleotide type that lacks a label that is not, or minimally, detected in either channel.

In some aspects, the techniques described herein relate to a method of sequencing clusters of labeled polynucleotides bound to a flowcell, including: exciting the clusters at a first excitation wavelength and detecting fluorescent emissions at a first detection wavelength from a first labeled nucleotide; exciting the clusters at a second excitation wavelength and detecting fluorescent emissions at a second detection wavelength from a second labeled nucleotide; exciting the clusters at the first excitation wavelength and detecting fluorescent emissions at the second detection wavelength from a third labeled nucleotide; determining clusters that did not emit fluorescent emissions after excitation at the first or second excitation wavelengths to detect the presence of a fourth unlabeled nucleotide; and determining the nucleotide sequence of the polynucleotides based on the detected fluorescent emissions.

Also described herein are chromenoquinoline dyes with long Stokes shifts and improved fluorescent intensity and chemical stability suitable for nucleotide labeling and sequencing applications. These chromenoquinoline dyes have strong fluorescence under both blue and green light excitation (for example, these chromenoquinoline dyes may have an absorption wavelength of from about 340 nm to about 540 nm, from about 350 nm to about 380 nm, or from about 510 nm to about 535 nm). In particular, these dyes are excitable by a blue laser at 350 to about 360 nm. Furthermore, these chromenoquinoline dyes have greater stability in high pH buffer compared to the commercially available dyes used for sequencing by synthesis (SBS).

One aspect of the present disclosure relates to a chromenoquinoline compound of Formula (I), or a salt, or a mesomeric form thereof: 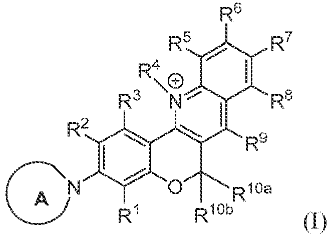
wherein ring A is a 4 to 10 membered heterocyclyl comprising at least one nitrogen atom, and ring A is optionally substituted with one or more R^{N};
each R^{N} is independently carboxyl, C₁-C₆ alkyl, substituted C₁-C₆ alkyl, C₁-C₆ alkoxy, substituted C₁-C₆ alkoxy, C₁-C₆ haloalkyl, C₁-C₆ haloalkoxy. (C₁-C₆ alkoxy)C₁-C₆ alkyl, -O(C₁-C₆ alkoxy)C₁-C₆ alkyl, optionally substituted amino, amino(C₁-C₆ alkyl), halo, cyano, hydroxy, hydroxy(C₁-C₆alkyl), nitro, sulfonyl, sulfo, sulfonate, S-sulfonamido, or N-sulfonamido;
each of R¹, R², R³, R⁵, R⁶, R⁷, R^{R}, R⁹, R^{10a} and R^{10b} is independently H, C₁-C₆ alkyl, substituted C₁-C₆alkyl, C₁-C₆alkoxy, substituted C₁-C₆alkoxy, C₂-C₆alkenyl, C₂-C₆alkynyl, C₁-C₆haloalkyl, C₁-C₆haloalkoxy, (C₁-C₆alkoxy)C₁-C₆alkyl, -O-(C₁-C₆alkoxy)C₁-C₆alkyl, optionally substituted amino, amino(C₁-C₆ alkyl), halo, cyano, hydroxy, hydroxy(C₁-C₆ alkyl), nitro, sulfonyl, sulfo, sulfino, sulfonate, S-sulfonamido, N-sulfonamido, optionally substituted phenyl, optionally substituted 5 to 6 membered heteroaryl, optionally substituted C₃-C₇ cycloalkyl, or optionally substituted 4 to 7 membered heterocyclyl,
R⁴ is C₁-C₆alkyl or substituted C₁-C₆alkyl;
alternatively, R⁵ and R⁶ together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl;
alternatively, R⁶ and R⁷ together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl; and
alternatively, R⁷ and R⁵ together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl;
provided that the compound of Formula (I) comprises a carboxyl group.

### BRIEF DESCRIPTION OF THE DRAWINGS

Features of examples of the present disclosure will become apparent by reference to the following detailed description and drawings, in which like reference numerals correspond to similar, though perhaps not identical, components. For the sake of brevity, reference numerals or features having a previously described function may or may not be described in connection with other drawings in which they appear.
**FIG. 1** is a flowchart illustrating a process of sequencing polynucleotides bound to a flow cell with three detection channels.
**FIG. 2** shows a three-dimensional scatterplot of the potential encoding space of a three-channel sequencing method.
**FIG. 3** shows a two-dimensional scatterplot of an experiment to demonstrate the potential signal-to-noise improvements (in decibels) from increasing the fraction of bright dyes on each labeled base.
**FIG. 4** is a flowchart illustrating a process of sequencing polynucleotides bound to a flow cell using a long Stokes shift dye.
**FIG. 5** shows an illustration of one embodiment of a three-channel sequencing method employing a long Stokes shift dye.
**FIG. 6** shows an illustration of a three-channel sequencing method with two configurations, a step and shoot configuration and a Time Delay Integration (TDI) imager configuration.
**FIG. 7** depicts an embodiment of a system including a memory comprising a three-channel sequencing module.
**FIG. 8A** illustrates the absorption spectra of ffA nucleotides conjugated with chromenoquinoline dyes I-1, I-1A, and I-2 through I-5 as a 1 µM solution in Universal Scan Mix (USM).
**FIG. 8B** illustrates the emission spectra of ffA nucleotides conjugated with chromenoquinoline dyes 1-1, I-1A, and I-2 through I-5 acquired in USM using 350 nm as excitation wavelength.
**FIGs. 9A** and **9B** are the scatterplots obtained at cycle 26 on an Illumina MiSeq^{®} instrument with 1Ex-2Ch mode using the incorporation mix with ffA nucleotide labeled with dye I-1, where the blue laser dosage is at 1x and 10x respectively.
**FIGs. 9C** and **9D** are the scatterplots obtained at cycle 26 on an Illumina MiSeq^{®} instrument with 1Ex-2Ch mode using the incorporation mix with ffA nucleotide labeled with dye 1-2, where the blue laser dosage is at 1x and 10xrespectively.
**FIG. 9E** is a line chart showing percentage of signal decay as a function of blue laser dosage on an Illumina MiSeq^{®} instrument with 1Ex-2Ch mode comparing incorporation mix with ffA nucleotide labeled with dye I-1 or dye 1-2.
**FIG. 9F** is a line chart showing the percent error rate as a function of blue laser dosage for the same sequencing runs, comparing ffA labeled with dye I-1 to ffA labeled with dye 1-2.
**FIGs. 10A** and **10B** are the scatterplots obtained at cycle 26 on an Illumina MiSeq^{®} instrument with 1Ex-2Ch mode using the incorporation mix with ffA nucleotide labeled with dye I-1A, where the blue laser dosage is at 1x and 10x respectively.
**FIGs. 10C** and **10D** are the scatterplots obtained at cycle 26 on an lllumina MiSeq^{®} instrument with 1Ex-2Ch mode using the incorporation mix with ffA nucleotide labeled with dye 1-1, where the blue laser dosage is at 1x and 10x respectively.
**FIG. 10E** is a line chart showing percentage of signal decay as a function of blue laser dosage on an Illumina MiSeq^{®} instrument with 1Ex-2Ch mode comparing incorporation mix with ffA nucleotide labeled with dye 1-1A or dye I-1.
**FIG. 10F** is a line chart showing the percent error rate as a function of blue laser dosage for the same sequencing runs, comparing ffA labeled with dye I-1A to ffA labeled with dye I-1.

### DETAILED DESCRIPTION

One embodiment is a three fluorescent dye system and method for sequencing polynucleotides. This embodiment relates to systems and methods for determining the nucleotide sequence of a polynucleotide by attaching three different fluorescent dyes to three different nucleotides during incorporation. For example, nucleotides A, T, and C may all be labeled with different fluorescent dyes which can emit light at three distinguishable wavelengths in three different channels. A Next Generation Sequencing (NGS) system, such as the ILLUMINA^{®} NEXTSEQ System may be used to detect clusters of polynucleotides, each being labeled by one of the three fluorescent dyes, and one dark state corresponding to a G nucleotide that is unlabeled, to perform a Sequencing by Synthesis (SBS) process. While a typical sequencing platform may employ two channels (colors) to excite and detect nucleotide bases, moving to a three-channel sequencing system allows for improved methods with relatively brighter labeled clusters and a higher Signal-To-Noise (SNR) ratio between the different fluorescent dyes. The brighter labeled clusters and higher SNR is due to the fact that most two-channel sequencing systems rely on using one of the labeled bases that is made to fluoresce in two channels by labeling equal proportions of the base with each of two different dyes. For example, a C nucleotide may be labeled with 50% of a blue dye and 50% of a green dye. Detection of a C nucleotide would then be made by detecting a cluster having both a blue and green fluorescent signature, but with each signature being 50% of the intensity of a cluster labeled with only a single fluorescent dye. Thus, this dual-labeled strategy effectively halves the intensity of the fluorescence for the nucleotide labeled with two fluorescent dyes. Additionally, competition between the two labeled nucleotides onto the base may exacerbate any sequence-dependent noise where one of the labeled nucleotides is preferentially incorporated over the other nucleotide.

In the described embodiment, the third nucleotide is labeled with its own unique fluorescent dye. Labeling the third nucleotide with a separate fluorescent dye molecule, using a separate channel, having, for example, a different excitation/emission detection, allows for each of the three nucleotide bases to be conjugated to their own fluorescent dye, with the fourth base being a "dark" base which is detected by having no fluorescent emission. This scheme allows each base to be labelled more brightly than in prior systems where one base was labeled with a mixture of two dyes. Thus, one aspect of the disclosure is directed to three-channel methods of sequencing polynucleotides where nucleic acids are sequenced using three differentially labeled nucleotide bases in clusters in an array format bound to a flow cell. The NGS system can emit light at the excitation wavelength of the fluorescent dyes attached to the three different labeled nucleotide bases within each cluster and a detector can be configured with the proper filters to create a channel for detecting the emission spectra from each fluorescent dye. These fluorescent images can be captured by a variety of different sensor types, including charge-coupled devices (CCDs) or other CMOS type sensors.

A second aspect of the disclosure is the use of three different fluorescent dyes, as mentioned above, but wherein one of the fluorescent dyes has a relatively long Stokes shift. By using a long-Stokes shift fluorescent dye, the system may require only two emitters and two detection channels, as opposed to three color detection channels mentioned above. For example, in this aspect, a first nucleotide A may be labeled by a first fluorescent dye which is excited at a first wavelength and emits light at a blue color of between 472nm to 520nm, which is detected by a first detector. A second nucleotide T may be labeled by a second fluorescent dye which is excited at a second wavelength and emits light at a green color, for example 540nm to 640nm, which is detected by a second detector. The third nucleotide C may be labeled with a long Stokes shift dye, such as described below, so that it is excited at the first blue wavelength, but emits at a green color, for example 540nm to 640nm, which is detected by the second detector. The fourth molecule G may be unlabeled, and detected by its lack of any fluorescence during an SBS cycle. The use of the long Stokes shift dye allowed a conventional two emitter/two detector SBS sequencing system to be used with a three-color process which can provide all the advantages of high SNR, for example, as mentioned above as compared to prior systems. Of course, while the above example uses various colors for the specific A/T/C/G nucleotides, it should be realized that each nucleotide could be matched with any particular fluorescent dye, or be unlabeled, and still be within the scope of the present invention.

Still another aspect of the present disclosure relates to chromenoquinoline dyes with enhanced fluorescent intensity, long Stokes shift and improved chemical stability, particularly in high pH aqueous environment. These chromenoquinoline dyes also have a wide excitation wavelength and may be excited by both blue and green light sources. In some embodiments, the chromenoquinoline dyes described herein may be used in Illumina's MiSeq^{®} platform using a single blue light excitation at about 350 nm to 360 nm, and two-channel detection (blue channel at about 372 to 520nm, and green channel at about 540-640nm).

### Definitions

The section headings used herein are for organizational purposes only and are not to be construed as limiting the subject matter described.

It is noted that, as used in this specification and the appended claims, the singular forms "a", "an" and "the" include plural referents unless expressly and unequivocally limited to one referent. It will be apparent to those skilled in the art that various modifications and variations can be made to various embodiments described herein without departing from the spirit or scope of the present teachings. Thus, it is intended that the various embodiments described herein cover other modifications and variations within the scope of the appended claims and their equivalents.

Unless defined otherwise, all technical and scientific terms used herein have the same meaning as is commonly understood by one of ordinary skill in the art. The use of the term "including" as well as other forms, such as "include", "includes," and "included," is not limiting. The use of the term "having" as well as other forms, such as "have", "has," and "had," is not limiting. As used in this specification, whether in a transitional phrase or in the body of the claim, the terms "comprise(s)" and "comprising" are to be interpreted as having an open-ended meaning. That is, the above terms are to be interpreted synonymously with the phrases "having at least" or "including at least." For example, when used in the context of a process, the term "comprising" means that the process includes at least the recited steps but may include additional steps. When used in the context of a compound, composition, or device, the term "comprising" means that the compound, composition, or device includes at least the recited features or components, but may also include additional features or components.

As used herein, common organic abbreviations are defined as follows:
- °C: Temperature in degrees Centigrade
- dATP: Deoxyadenosine triphosphate
- dCTP: Deoxycytidine triphosphate
- dGTP: Deoxyguanosine triphosphate
- dTTP: Deoxythymidine triphosphate
- ddNTP: Dideoxynucleotide triphosphate
- ffA: Fully functionalized A nucleotide
- ffC: Fully functionalized C nucleotide
- ffG: Fully functionalized G nucleotide
- ffN: Fully functionalized nucleotide
- ffT: Fully functionalized T nucleotide
- h: Hour(s)
- RT: Room temperature
- SBS: Sequencing by Synthesis
- A: Adenosine (may refer to nucleotide base calls)
- C: Cytosine
- G: Guanine
- T: Thymine

As used herein, the term "array" refers to a population of different probe molecules that are attached to one or more substrates such that the different probe molecules can be differentiated from each other according to relative location. An array can include different probe molecules that are each located at a different addressable location on a substrate. Alternatively, or additionally, an array can include separate substrates each bearing a different probe molecule, wherein the different probe molecules can be identified according to the locations of the substrates on a surface to which the substrates are attached or according to the locations of the substrates in a liquid. Exemplary arrays in which separate substrates are located on a surface include, without limitation, those including beads in wells as described, for example, in U.S. Patent No. 6,1055,331 B1, US 2002/0102578 and PCT Publication No. WO 00/63437. Exemplary formats that can be used in the invention to distinguish beads in a liquid array, for example, using a microfluidic device, such as a fluorescent activated cell sorter (FACS), are described, for example, in US Pat. No. 6,524,793. Further examples of arrays that can be used in the invention include, without limitation, those described in U.S. Pat Nos. 5,329,807; 5,336,1027; 5,561,071; 5,583,911; 5,658,734; 5,837,858; 5,874,919; 5,919,523; 6,836,969; 6,987,768; 6,987,776; 6,988,920; 6,997,006; 6,991,893; 6,1046,313; 6,316,949; 6,382,591; 6,514,751 and 6,610,382; and WO 93/17126; WO 95/11995; WO 95/35505; EP 742 987; and EP 799 897.

As used herein, the term "covalently attached" or "covalently bonded" refers to the forming of a chemical bonding that is characterized by the sharing of pairs of electrons between atoms. For example, a covalently attached polymer coating refers to a polymer coating that forms chemical bonds with a functionalized surface of a substrate, as compared to attachment to the surface via other means, for example, adhesion or electrostatic interaction. It will be appreciated that polymers that are attached covalently to a surface can also be bonded via means in addition to covalent attachment.

The term "halogen" or "halo," as used herein, means any one of the radio-stable atoms of column 7 of the Periodic Table of the Elements, e.g., fluorine, chlorine, bromine, or iodine, with fluorine and chlorine being preferred.

As used herein, "Cₐ to C_{b}" in which "a" and "b" are integers refer to the number of carbon atoms in an alkyl, alkenyl or alkynyl group, or the number of ring atoms of a cycloalkyl or aryl group. That is, the alkyl, the alkenyl, the alkynyl, the ring of the cycloalkyl, and ring of the aryl can contain from "a" to "b", inclusive, carbon atoms. For example, a "Ci to C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)- and (CH₃)₃C-; a C₃ to C₄ cycloalkyl group refers to all cycloalkyl groups having from 3 to 4 carbon atoms, that is, cyclopropyl and cyclobutyl. Similarly, a "4 to 6 membered heterocyclyl" group refers to all heterocyclyl groups with 4 to 6 total ring atoms, for example, azetidine, oxetane, oxazoline, pyrrolidine, piperidine, piperazine, morpholine, and the like. If no "a" and "b" are designated with regard to an alkyl, alkenyl, alkynyl, cycloalkyl, or aryl group, the broadest range described in these definitions is to be assumed. As used herein, the term "C₁-C₆" includes C₁, C₂, C₃, C₄, C₅ and C₆, and a range defined by any of the two numbers. For example, C₁-C₆ alkyl includes C₁, C₂, C₃, C₄, C₅ and C₆ alkyl, C₂-C₆ alkyl, C₁-C₃ alkyl, etc. Similarly, C₂-C₆ alkenyl includes C₂, C₃, C₄, C₅ and C₆ alkenyl, C₂-C₅ alkenyl, C₃-C₄ alkenyl, etc., and C₂-C₆ alkynyl includes C₂, C₃, C₄, C₅ and C₆ alkynyl, C₂-C₅ alkynyl, C₃-C₄ alkynyl, etc. C₃-C₈ cycloalkyl each includes hydrocarbon ring containing 3, 4, 5, 6, 7 and 8 carbon atoms, or a range defined by any of the two numbers, such as C₃-C₇ cycloalkyl or C₅-C₆ cycloalkyl.

As used herein, "alkyl" refers to a straight or branched hydrocarbon chain that is fully saturated (i.e., contains no double or triple bonds). The alkyl group may have 1 to 20 carbon atoms (whenever it appears herein, a numerical range such as "1 to 20" refers to each integer in the given range, *e.g.,* "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 9 carbon atoms. The alkyl group could also be a lower alkyl having 1 to 6 carbon atoms. By way of example only, "C₁-₆ alkyl" or "C₁-C₆ alkyl" indicates that there are one to six carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl, and the like.

As used herein, "alkoxy" refers to the formula -OR wherein R is an alkyl as is defined above, such as ʺʺC₁-₉alkoxy" or "C₁-C₉ alkoxy", including but not limited to methoxy, ethoxy, n-propoxy, 1-methylethoxy (isopropoxy), n-butoxy, iso-butoxy, sec-butoxy, and tert-butoxy, and the like.

As used herein, "alkenyl" refers to a straight or branched hydrocarbon chain containing one or more double bonds. The alkenyl group may have 2 to 20 carbon atoms, although the present definition also covers the occurrence of the term "alkenyl" where no numerical range is designated. The alkenyl group may also be a medium size alkenyl having 2 to 9 carbon atoms. The alkenyl group could also be a lower alkenyl having 2 to 6 carbon atoms. By way of example only, "C₂₋C₆ alkenyl" or "C₂₋₆ alkenyl" indicates that there are two to six carbon atoms in the alkenyl chain, i.e., the alkenyl chain is selected from the group consisting of ethenyl, propen-1-yl, propen-2-yl, propen-3-yl, buten-1-yl, buten-2-yl, buten-3-yl, buten-4-yl, 1-methyl-propen-1-yl, 2-methyl-propen-1-yl, 1-ethyl-ethen-1-yl, 2-methyl-propen-3-yl, buta-1.3-dienyl, buta-1,2,-dienyl, and buta-1,2-dien-4-yl. Typical alkenyl groups include, but are in no way limited to, ethenyl, propenyl, butenyl, pentenyl, and hexenyl, and the like.

As used herein, "alkynyl" refers to a straight or branched hydrocarbon chain containing one or more triple bonds. The alkynyl group may have 2 to 20 carbon atoms, although the present definition also covers the occurrence of the term "alkynyl" where no numerical range is designated. The alkynyl group may also be a medium size alkynyl having 2 to 9 carbon atoms. The alkynyl group could also be a lower alkynyl having 2 to 6 carbon atoms. By way of example only, "C₂₋₆ alkynyl" or "C₂-C₆ alkenyl" indicates that there are two to six carbon atoms in the alkynyl chain, i.e., the alkynyl chain is selected from the group consisting of ethynyl, propyn-1-yl, propyn-2-yl, butyn-1-yl, butyn-3-yl, butyn-4-yl, and 2-butynyl. Typical alkynyl groups include, but are in no way limited to, ethynyl, propynyl, butynyl, pentynyl, and hexynyl, and the like.

The term "aromatic" refers to a ring or ring system having a conjugated pi electron system and includes both carbocyclic aromatic (e.g., phenyl) and heterocyclic aromatic groups (e.g., pyridine). The term includes monocyclic or fused-ring polycyclic (i.e., rings which share adjacent pairs of atoms) groups provided that the entire ring system is aromatic.

As used herein, "aryl" refers to an aromatic ring or ring system (i.e., two or more fused rings that share two adjacent carbon atoms) containing only carbon in the ring backbone. When the aryl is a ring system, every ring in the system is aromatic. The aryl group may have 6 to 18 carbon atoms, although the present definition also covers the occurrence of the term "aryl" where no numerical range is designated. In some embodiments, the aryl group has 6 to 10 carbon atoms. The aryl group may be designated as "C₆-C₁₀ aryl," "C₆ or C₁₀ aryl," or similar designations. Examples of aryl groups include, but are not limited to, phenyl, naphthyl, azulenyl, and anthracenyl.

An "aralkyl" or "arylalkyl" is an aryl group connected, as a substituent, via an alkylene group, such as "C₇₋₁₄ aralkyl" and the like, including but not limited to benzyl, 2-phenylethyl, 3-phenylpropyl, and naphthylalkyl. In some cases, the alkylene group is a lower alkylene group (i.e., a C₁₋₆ alkylene group).

As used herein, "heteroaryl" refers to an aromatic ring or ring system (i.e., two or more fused rings that share two adjacent atoms) that contain(s) one or more heteroatoms, that is, an element other than carbon, including but not limited to, nitrogen, oxygen and sulfur, in the ring backbone. When the heteroaryl is a ring system, every ring in the system is aromatic. The heteroaryl group may have 5-18 ring members (i.e., the number of atoms making up the ring backbone, including carbon atoms and heteroatoms), although the present definition also covers the occurrence of the term "heteroaryl" where no numerical range is designated. In some embodiments, the heteroaryl group has 5 to 10 ring members or 5 to 7 ring members. The heteroaryl group may be designated as "5-7 membered heteroaryl," "5-10 membered heteroaryl," or similar designations. Examples of heteroaryl rings include, but are not limited to, furyl, thienyl, phthalazinyl, pyrrolyl, oxazolyl, thiazolyl, imidazolyl, pyrazolyl, isoxazolyl, isothiazolyl, triazolyl, thiadiazolyl, pyridinyl, pyridazinyl, pyrimidinyl, pyrazinyl, triazinyl, quinolinyl, isoquinlinyl, benzimidazolyl, benzoxazolyl, benzothiazolyl, indolyl, isoindolyl, and benzothienyl.

A "heteroaralkyl" or "heteroarylalkyl" is heteroaryl group connected, as a substituent, via an alkylene group. Examples include but are not limited to 2-thienylmethyl, 3-thienylmethyl, furylmethyl, thienylethyl, pyrrolylalkyl, pyridylalkyl, isoxazollylalkyl, and imidazolylalkyl. In some cases, the alkylene group is a lower alkylene group (i.e., a C₁₋₆ alkylene group).

As used herein, "carbocyclyl" means a non-aromatic cyclic ring or ring system containing only carbon atoms in the ring system backbone. When the carbocyclyl is a ring system, two or more rings may be joined together in a fused, bridged or spiro-connected fashion. Carbocyclyls may have any degree of saturation provided that at least one ring in a ring system is not aromatic. Thus, carbocyclyls include cycloalkyls, cycloalkenyls, and cycloalkynyls. The carbocyclyl group may have 3 to 20 carbon atoms, although the present definition also covers the occurrence of the term "carbocyclyl" where no numerical range is designated. The carbocyclyl group may also be a medium size carbocyclyl having 3 to 10 carbon atoms. The carbocyclyl group could also be a carbocyclyl having 3 to 6 carbon atoms. The carbocyclyl group may be designated as "C₃₋₆ carbocyclyl", "C₃-C₆ carbocyclyl" or similar designations. Examples of carbocyclyl rings include, but are not limited to, cyclopropyl, cyclobutyl, cyclopentyl, cyclohexyl, cyclohexenyl, 2,3-dihydro-indene, bicycle[2.2.2]octanyl, adamantyl, and spiro[4.4]nonanyl.

As used herein, "cycloalkyl" means a fully saturated carbocyclyl ring or ring system. Examples include cyclopropyl, cyclobutyl, cyclopentyl, and cyclohexyl.

As used herein, "heterocyclyl" means a non-aromatic cyclic ring or ring system containing at least one heteroatom in the ring backbone. Heterocyclyls may be joined together in a fused, bridged or spiro-connected fashion. Heterocyclyls may have any degree of saturation provided that at least one ring in the ring system is not aromatic. The heteroatom(s) may be present in either a non-aromatic or aromatic ring in the ring system. The heterocyclyl group may have 3 to 20 ring members (i.e., the number of atoms making up the ring backbone, including carbon atoms and heteroatoms), although the present definition also covers the occurrence of the term "heterocyclyl" where no numerical range is designated. The heterocyclyl group may also be a medium size heterocyclyl having 3 to 10 ring members. The heterocyclyl group could also be a heterocyclyl having 3 to 6 ring members. The heterocyclyl group may be designated as "3-6 membered heterocyclyl" or similar designations. In preferred six membered monocyclic heterocyclyls, the heteroatom(s) are selected from one up to three of O, N or S, and in preferred five membered monocyclic heterocyclyls, the heteroatom(s) are selected from one or two heteroatoms selected from O, N, or S. Examples of heterocyclyl rings include, but are not limited to, azepinyl, acridinyl, carbazolyl, cinnolinyl, dioxolanyl, imidazolinyl, imidazolidinyl, morpholinyl, oxiranyl, oxepanyl, thiepanyl, piperidinyl, piperazinyl, dioxopiperazinyl, pyrrolidinyl, pyrrolidonyl, pyrrolidionyl, 4-piperidonyl, pyrazolinyl, pyrazolidinyl, 1,3-dioxinyl, 1,3-dioxanyl, 1,4-dioxinyl, 1,4-dioxanyl, 1,3-oxathianyl, 1,4-oxathiinyl, 1,4-oxathianyl, 2*H*-1,2-oxazinyl, trioxanyl, hexahydro-1,3,5-triazinyl, 1,3-dioxolyl, 1,3-dioxolanyl, 1,3-dithiolyl, 1,3-dithiolanyl, isoxazolinyl, isoxazolidinyl, oxazolinyl, oxazolidinyl, oxazolidinonyl, thiazolinyl, thiazolidinyl, 1,3-oxathiolanyl, indolinyl, isoindolinyl, tetrahydrofuranyl, tetrahydropyranyl, tetrahydrothiophenyl, tetrahydrothiopyranyl, tetrahydro-1,4-thiazinyl, thiamorpholinyl, dihydrobenzofuranyl, benzimidazolidinyl, and tetrahydroquinoline.

As used herein, "alkoxyalkyl" or "(alkoxy)alkyl" refers to an alkoxy group connected via an alkylene group, such as C₂-C₈alkoxyalkyl, or (C₁-C₆ alkoxy)C₁-C₆alkyl, for example, -(CH₂)₁₋₃-OCH₃.

An "O-carboxy" group refers to a "-OC(=O)R" group in which R is selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein.

A "C-carboxy" group refers to a "-C(=O)OR" group in which R is selected from the group consisting of hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein. A non-limiting example includes carboxyl (i.e., -C(=O)OH).

A "sulfonyl" group refers to an "-SO₂R" group in which R is selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein.

A "sulfino" group refers to a "-S(=O)OH" group.

A "sulfo" group refers to a"-S(=O)₂OH" or "-SO₃H" group.

A "sulfonate" group refers to a "-SO₃⁻" group.

A "sulfate" group refers to "-SO₄⁻" group.

A "S-sulfonamido" group refers to a "-SO₂NR_{A}R_{B}" group in which R_{A} and R_{B} are each independently selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein.

An "N-sulfonamido" group refers to a "-N(R₄)SO₂R_{B}" group in which R_{A} and R_{b} are each independently selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein.

A "C-amido" group refers to a "-C(=O)NR_{A}R_{B}" group in which R_{A} and R_{B} are each independently selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein.

An "N-amido" group refers to a "-N(R_{A})C(=O)R_{B}" group in which R_{A} and R_{B} are each independently selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein.

An "amino" group refers to a "-NR_{A}R_{B}" group in which R_{A} and R_{B} are each independently selected from hydrogen, C₁₋₆ alkyl, C₂₋₆ alkenyl, C₂₋₆ alkynyl, C₃₋₇ carbocyclyl, C₆₋₁₀ aryl, 5-10 membered heteroaryl, and 3-10 membered heterocyclyl, as defined herein. A non-limiting example includes free amino (i.e., -NH₂).

An "aminoalkyl" group refers to an amino group connected via an alkylene group.

An "alkoxyalkyl" group refers to an alkoxy group connected via an alkylene group, such as a "C₂-C₈ alkoxyalkyl" and the like.

When a group is described as "optionally substituted" it may be either unsubstituted or substituted. Likewise, when a group is described as being "substituted", the substituent may be selected from one or more of the indicated substituents. As used herein, a substituted group is derived from the unsubstituted parent group in which there has been an exchange of one or more hydrogen atoms for another atom or group. Unless otherwise indicated, when a group is deemed to be "substituted," it is meant that the group is substituted with one or more substituents independently selected from C₁-C₆ alkyl, C₁-C₆ alkenyl, C₁-C₆ alkynyl, C₃-C₇ carbocyclyl (optionally substituted with halo, C₁-C₆ alkyl, C₁-C₆ alkoxy, C₁-C₆ haloalkyl, and C₁-C₆ haloalkoxy), C₃-C₇-carbocyclyl-C₁₋C₆-alkyl (optionally substituted with halo, C₁-C₆ alkyl, C₁-C₆ alkoxy, C₁-C₆ haloalkyl, and C₁-C₆ haloalkoxy), 3-10 membered heterocyclyl (optionally substituted with halo, C₁-C₆ alkyl, C₁-C₆ alkoxy, C₁-C₆ haloalkyl, and C₁-C₆ haloalkoxy), 3-10 membered heterocyclyl-C₁-C₆-alkyl (optionally substituted with halo, C₁-C₆ alkyl, C₁-C₆ alkoxy, C₁-C₆ haloalkyl, and C₁-C₆ haloalkoxy), aryl (optionally substituted with halo, C₁-C₆ alkyl, C₁-C₆ alkoxy, C₁-C₆ haloalkyl, and C₁-C₆ haloalkoxy), aryl(C₁-C₆)alkyl (optionally substituted with halo, C₁-C₆ alkyl, C₁-C₆ alkoxy, C₁-C₆ haloalkyl, and C₁-C₆ haloalkoxy), 5-10 membered heteroaryl (optionally substituted with halo, C₁-C₆ alkyl, C₁-C₆ alkoxy, C₁-C₆ haloalkyl, and C₁-C₆ haloalkoxy), 5-10 membered heteroaryl(C₁-C₆)alkyl (optionally substituted with halo, C₁-C₆ alkyl, C₁-C₆ alkoxy. C₁-C₆ haloalkyl, and C₁-C₆ haloalkoxy), halo, -CN, hydroxy, C₁-C₆ alkoxy, C₁-C₆ alkoxy(C₁-C₆)alkyl (i.e., ether), aryloxy, sulfhydryl (mercapto), halo(C₁-C₆)alkyl (e.g., -CF₃), halo(C₁-C₆)alkoxy (e.g., -OCF₃), C₁-C₆ alkylthio, arylthio, amino, amino(C₁-C₆)alkyl, nitro, O-carbamyl, N-carbamyl, O-thiocarbamyl, N-thiocarbamyl, C-amido, N-amido, S-sulfonamido, N-sulfonamido, C-carboxy, O-carboxy, acyl, cyanato, isocyanato, thiocyanato, isothiocyanato, sulfinyl, sulfonyl, -SO₃H, sulfonate, sulfate, sulfino, -OSO₂C₁-C₄alkyl, and oxo (=O). Wherever a group is described as "optionally substituted" that group can be substituted with the above substituents. In some embodiments, when an alkyl, alkenyl, alkynyl, aryl, heteroaryl, carbocyclyl or heterocyclyl group is substituted, each is independently substituted with one or more substituents selected from the group consisting of halo, -CN. -SO₃⁻, -OSO₃⁻, -SO₃H, -SR^{A}, -OR^{A}, -NR^{B}R^{C}, oxo, -CONR^{B}R^{C}, -SO₂NR^{B}R^{C}, -COOH, and -COOR^{B}, where R^{A}, R^{B} and R^{C} are each independently selected from H, alkyl, substituted alkyl, alkenyl, substituted alkenyl, alkynyl, substituted alkynyl, aryl, and substituted aryl.

As understood by one of ordinary skill in the art, a compound described herein may exist in ionized form, e.g., the compound may comprise -CO₂⁻, -SO₃⁻ or -O-SO₃⁻, or a pyridinium moiety 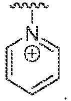 If a compound contains a positively charged group such as pyridinium, it may also contain a negatively charged group (such as -SO₃⁻) or a counterion such that the compound as a whole is neutral. Similarly, if a compound contains negatively charged group, for example, -SO₃⁻, it may also contain a positively charged group (such as a pyridinium moiety) or a counterion such that the compound as a whole is neutral. The compound may exist in a salt form, where the counterion is provided by a conjugate acid or base. The counterion may not expressly shown in the compound structure.

It is to be understood that certain radical naming conventions can include either a mono-radical or a di-radical, depending on the context. For example, where a substituent requires two points of attachment to the rest of the molecule, it is understood that the substituent is a di-radical. For example, a substituent identified as alkyl that requires two points of attachment includes di-radicals such as -CH₂-, -CH₂CH₂-, -CH₂CH(CH₃)CH₂-, and the like. Other radical naming conventions clearly indicate that the radical is a di-radical such as "alkylene" or "alkenylene."

When two "adjacent" R groups are said to form a ring "together with the atom to which they are attached," it is meant that the collective unit of the atoms, intervening bonds, and the two R groups are the recited ring. For example, when the following substructure is present: 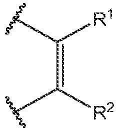 and R¹ and R² are defined as selected from the group consisting of hydrogen and alkyl, or R¹ and R² together with the atoms to which they are attached form an aryl or carbocyclyl, it is meant that R¹ and R² can be selected from hydrogen or alkyl, or alternatively, the substructure has structure: 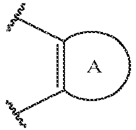 where A is an aryl ring or a carbocyclyl containing the depicted double bond.

Wherever a substituent is depicted as a di-radical (*i.e.,* has two points of attachment to the rest of the molecule), it is to be understood that the substituent can be attached in any directional configuration unless otherwise indicated. Thus, for example, a substituent depicted as -AE- or 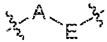 includes the substituent being oriented such that the A is attached at the leftmost attachment point of the molecule as well as the case in which A is attached at the rightmost attachment point of the molecule. In addition, if a group or substituent is depicted as 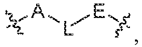 and L is defined an optionally present linker moiety; when L is not present (or absent), such group or substituent is equivalent to

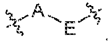

In each instance where a single mesomeric form of a compound described herein is shown, the alternative mesomeric forms are equally contemplated.

As used herein, a "nucleotide" includes a nitrogen containing heterocyclic base, a sugar, and one or more phosphate groups. They are monomeric units of a nucleic acid sequence. In RNA, the sugar is a ribose, and in DNA a deoxyribose, *i.e.* a sugar lacking a hydroxyl group that is present in ribose. The nitrogen containing heterocyclic base can be purine, deazapurine, or pyrimidine base. Purine bases include adenine (A) and guanine (G), and modified derivatives or analogs thereof, such as 7-deaza adenine or 7-deaza guanine. Pyrimidine bases include cytosine (C), thymine (T), and uracil (U), and modified derivatives or analogs thereof. The C-1 atom of deoxyribose is bonded to N-1 of a pyrimidine or N-9 of a purine.

As used herein, a "nucleoside" is structurally similar to a nucleotide, but is missing the phosphate moieties. An example of a nucleoside analogue would be one in which the label is linked to the base and there is no phosphate group attached to the sugar molecule. The term "nucleoside" is used herein in its ordinary sense as understood by those skilled in the art. Examples include, but are not limited to, a ribonucleoside comprising a ribose moiety and a deoxyribonucleoside comprising a deoxyribose moiety. A modified pentose moiety is a pentose moiety in which an oxygen atom has been replaced with a carbon and/or a carbon has been replaced with a sulfur or an oxygen atom. A "nucleoside" is a monomer that can have a substituted base and/or sugar moiety. Additionally, a nucleoside can be incorporated into larger DNA and/or RNA polymers and oligomers.

The term "purine base" is used herein in its ordinary sense as understood by those skilled in the art, and includes its tautomers. Similarly, the term "pyrimidine base" is used herein in its ordinary sense as understood by those skilled in the art, and includes its tautomers. A non-limiting list of optionally substituted purine-bases includes purine, adenine, guanine, deazapurine, 7-deaza adenine, 7-deaza guanine. hypoxanthine, xanthine, alloxanthine, 7-alkylguanine (e.g., 7-methylguanine), theobromine, caffeine, uric acid and isoguanine. Examples of pyrimidine bases include, but are not limited to, cytosine, thymine, uracil, 5,6-dihydrouracil and 5-alkylcytosine (e.g., 5-methylcytosine).

As used herein, when an oligonucleotide or polynucleotide is described as "comprising" a nucleoside or nucleotide described herein, it means that the nucleoside or nucleotide described herein forms a covalent bond with the oligonucleotide or polynucleotide. Similarly, when a nucleoside or nucleotide is described as part of an oligonucleotide or polynucleotide, such as "incorporated into" an oligonucleotide or polynucleotide, it means that the nucleoside or nucleotide described herein forms a covalent bond with the oligonucleotide or polynucleotide. In some such embodiments, the covalent bond is formed between a 3' hydroxy group of the oligonucleotide or polynucleotide with the 5' phosphate group of a nucleotide described herein as a phosphodiester bond between the 3' carbon atom of the oligonucleotide or polynucleotide and the 5' carbon atom of the nucleotide.

As used herein, the term "cleavable linker" is not meant to imply that the whole linker is required to be removed. The cleavage site can be located at a position on the linker that ensures that part of the linker remains attached to the detectable label and/or nucleoside or nucleotide moiety after cleavage.

As used herein, "derivative" or "analog" means a synthetic nucleotide or nucleoside derivative having modified base moieties and/or modified sugar moieties. Such derivatives and analogs are discussed in, e.g., Scheit, Nucleotide Analogs (John Wiley & Son, 1980) and Uhlman et al., Chemical Reviews 90:543-584, 1990. Nucleotide analogs can also comprise modified phosphodiester linkages, including phosphorothioate, phosphorodithioate, alkyl-phosphonate, phosphoranilidate and phosphoramidate linkages. "Derivative", "analog" and "modified" as used herein, may be used interchangeably, and are encompassed by the terms "nucleotide" and "nucleoside" defined herein.

As used herein, the term "phosphate" is used in its ordinary sense as understood by those skilled in the art, and includes its protonated forms (for example, 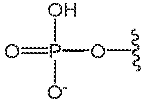 and 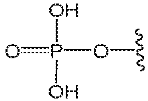 ). As used herein, the terms "monophosphate," "diphosphate," and "triphosphate" are used in their ordinary sense as understood by those skilled in the art, and include protonated forms.

As used herein, the term "phasing" refers to a phenomenon in SBS that is caused by incomplete removal of the 3' terminators and fluorophores, and/or failure to complete the incorporation of a portion of DNA strands within clusters by polymerases at a given sequencing cycle. Prephasing is caused by the incorporation of nucleotides without effective 3' terminators, wherein the incorporation event goes 1 cycle ahead due to a termination failure. Phasing and prephasing cause the measured signal intensities for a specific cycle to consist of the signal from the current cycle as well as noise from the preceding and following cycles. As the number of cycles increases, the fraction of sequences per cluster affected by phasing and prephasing increases, hampering the identification of the correct base. Prephasing can be caused by the presence of a trace amount of unprotected or unblocked 3'-OH nucleotides during sequencing by synthesis (SBS). The unprotected 3'-OH nucleotides could be generated during the manufacturing processes or possibly during the storage and reagent handling processes. Accordingly, the discovery of nucleotide analogues which decrease the incidence of prephasing is surprising and provides a great advantage in SBS applications over existing nucleotide analogues. For example, the nucleotide analogues provided can result in faster SBS cycle time, lower phasing and prephasing values, and longer sequencing read lengths.

### Methods of Sequencing

### Three Channel Base Calling

One embodiment includes systems and methods which utilize three-channel base calling. A "channel" may be used to describe an emission/detection process for a labeled base that is specific for a particular excitation wavelength and detection wavelength pair. Thus, a channel may include a particular first range of wavelengths of light used to excite a fluorescent dye and a second range of wavelengths of light used to detect the fluorescent emissions from the excited dyes. In three-channel base calling systems and methods, each base may be excited and/or detected in turn such that each labeled base may be considered in a different channel. Isolated signals from each channel may reduce cross-talk between bases and also allow for brighter dyes and/or brighter clusters that will improve SNR for base calling.

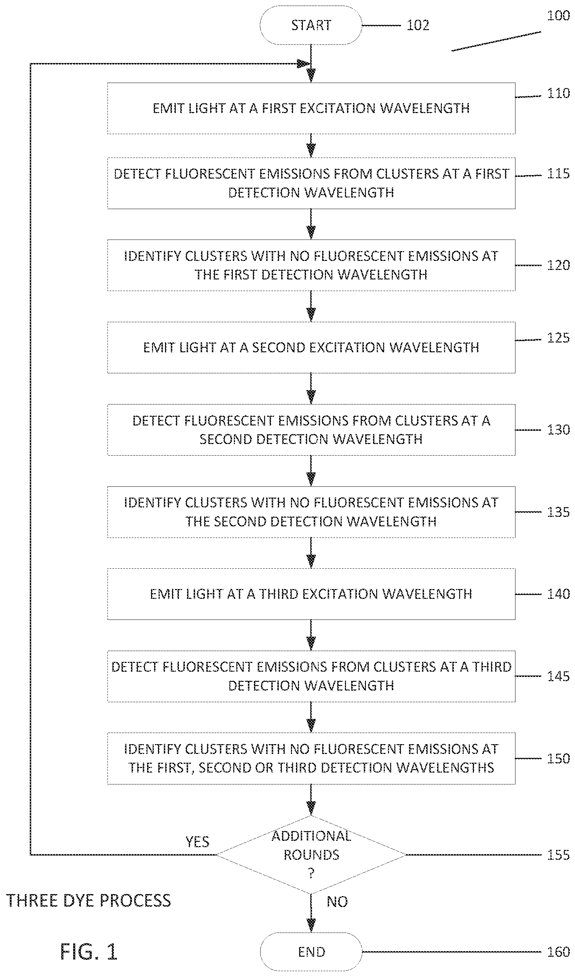

Figure 1 shows a flowchart of a method 100 of sequencing clusters of labeled polynucleotides bound to a flowcell according to one embodiment. The method may begin at a start step 102, by, for example, gathering DNA samples, and putting the sample into a sequencing system. Sequencing systems according to the disclosure may include flow cell sequencers such as those produced by ILLUMINA^{®}, INC. (San Diego, CA). For example, when the method 100 begins at step 102, a flowcell may have been prepared by embedding the flowcell with fragmented polynucleotides (e.g., fragmented single- or double-stranded polynucleotide fragments). Fragmented polynucleotides may be generated from a deoxyribonucleic acid (DNA) sample. DNA samples may be from various sources, for example, a biological sample, a cell sample, an environmental sample, or any combination thereof. The lengths of fragmented polynucleotide fragments may range from 900 bases to 1000 bases.

Polynucleotide fragments may be bridge-amplified into clusters of polynucleotide fragments attached to the inside surface of one or more channels of a flowcell. An inside surface of the one or more flowcell channels may include two types of primers, for example a first primer type (P1) and a second primer type (P2) and the DNA fragments may be amplified by well-known methods.

After generating clusters within the flowcell, the method 100 may begin a Sequencing by Synthesis process. During each sequencing cycle, four or more types of nucleotide analogs are added and incorporated onto the growing primer-polynucleotides. The four types of nucleotide analogs may have different modifications. For example, a first type of nucleotide may be an analog of deoxyguanosine triphosphate (dGTP), and not be conjugated with any fluorescent label. A second type of nucleotide may be an analog of deoxythymidine triphosphate (dTTP), which is conjugated with the first type of fluorescent label that can be excited at a first emission wavelength via a linker. A third type of nucleotide may be an analog of deoxycytidine triphosphate (dCTP), which is conjugated with the second type fluorescent label that can be excited at a second excitation wavelength via a linker. A fourth type of nucleotide may be an analog of deoxyadenosine triphosphate (dATP), which is conjugated with a third type of fluorescent label that can be excited at a third excitation wavelength via a linker. The linkers may include one or more cleavage groups. Prior to the subsequent sequencing cycle, the fluorescent labels may be removed from the nucleotide analogs. For example, a linker attaching a fluorescent label to a nucleotide analog may include an azide and/or an alkoxy group, for example on the same carbon, such that the linker may be cleaved after each incorporation cycle by a phosphine reagent, thereby releasing the fluorescent label from subsequent sequencing cycles.

Once the clusters are created on the flowcell, the method 100 moves to a step 110 of emitting light at a first excitation wavelength. For example, step 110 may include exciting all of the clusters of labeled polynucleotides on a flowcell at a first excitation wavelength. The method 100 then moves to a step 115 to detect any fluorescent emissions from the clusters at a first detection wavelength to detect the presence of a first labeled nucleotide. A single light source such as a laser or an LED source may excite a fluorescent label at the predetermined excitation wavelength. In some embodiments, the single laser or the LED source may be non-tunable.

Image processing yields base calling, described in further detail below, where the complementary nucleotides added to the molecules in a cluster during a cycle are identified. In some embodiments, the fluorescent images may be stored for later processing offline. In some embodiments, the fluorescent images may be processed to determine the sequence of the growing primer-polynucleotides in each cluster in real time. After detecting the fluorescence emissions from the clusters at step 115, the method 100 moves to a step 120, where the method identifies clusters with no fluorescent emissions at the first detection wavelength.

Once the clusters which did not have any fluorescent emissions were identified in step 120, the method 100 moves to a step 125, wherein the system emits light at a second excitation wavelength. For example, at step 125, systems according to the disclosure may proceed by exciting the clusters of labeled polynucleotides on the flowcell at a second excitation wavelength. Once the clusters have been excited at the second excitation wavelength, the method 100 moves to a step 130 wherein the system detects fluorescent emissions from the clusters at the second detection wavelength to detect the presence of a second labeled nucleotide. The method 100 then moves to a step 135, wherein the system may identify clusters which had no fluorescent emissions after excitation at the second excitation wavelength.

The method 100 may then move to a step 140, wherein the system emits light at a third excitation wavelength. The method 100 can then move to a step 145 to detect fluorescent emissions from clusters at a third detection wavelength. For example, at steps 140 and 145, systems of the disclosure may excite the clusters of labeled polynucleotides at a third excitation wavelength and detect fluorescent emissions from the clusters at a third detection wavelength to detect the presence of a third labeled nucleotide. According to the disclosure, systems and methods disclosed herein may determine the nucleotide sequence of the labeled polynucleotides based on the detected fluorescent emissions.

The method 100 may then move to a step 150 wherein the system identifies clusters which had no fluorescent emissions following excitation at the third excitation wavelength. The method 100 then moves to a decision step 155 to determine if the method 100 should repeat for an additional cycle of reading sequence data. A determination may be made at decision step 155 whether to detect more nucleotides based on, for example, the quality of the signal or after a predetermined number of bases. If more nucleotides are to be detected, then the method 100 may loop back to the step 110 to start a next sequencing cycle.

In some embodiments, prior to the next sequencing cycle, the fluorescent labels attached to each nucleotide may be removed from the incorporated nucleotide analogs, and the reversible 3' blocks may be removed so that another nucleotide analog may be added onto each extending primer-polynucleotide. If a determination is made at the decision step 155 that there are no more additional rounds of sequencing necessary, the method 100 then moves to an end step 160 and terminates the method 100. If the system is employing offline fluorescent imaging processing, when there is no additional nucleotide to be detected at decision block 155, the fluorescent images comprising the fluorescent signals detected may be processed after step 160, and the bases of the nucleotides incorporated into the fragmented polynucleotide may be determined remotely. For each nucleotide base determined, a quality score may be determined. After all the fluorescent images are processed, the method 100 may terminate at the step 160.

Sequencing nucleotides of a polynucleotide sequence may involve hundreds of cycles. Accordingly, sequencing a molecule of 350 nucleotides by SBS can involve 300 or more processing cycles in a run. In some embodiments, each cycle may involve simultaneous delivery of all the different nucleotide types to a flow cell and some or all of the nucleotide types may be labelled with a spectrally distinct label. Three images can then be obtained, each using a detection channel that is selective for one of the three different labels. Alternatively, different nucleotide types can be added sequentially and an image of the array of clusters can be obtained between each addition step. In such embodiments each image will show nucleic acid features that have incorporated nucleotides of a particular type. Different features will be present or absent in the different images due the different sequence content of each feature. However, the relative position of the features will remain unchanged in the images. Images obtained from such reversible terminator-SBS methods can be stored, processed and analyzed as set forth herein.

As mentioned above, a cluster may be identified by the lack of any fluorescence after excitation at either the first, second, and/or third excitation wavelengths. Because the lack of fluorescent emissions is dark or "missing" on an image, the system may track the position of each cluster on a flow cell. Once a cluster has been identified at a particular position, the system may then note in subsequence sequencing rounds whether there is a fluorescent emission at the position of the cluster. If no fluorescent emissions are noted at a known position of a cluster, the system may determine that the nucleotide added in the latest sequencing round was the unlabeled nucleotide. For example, in some cases the unlabeled nucleotide may be a "G". However, it should be realized that any nucleotide could be chosen as the one which is unlabeled and still be within the scope of the invention.

An aspect of the disclosure is directed to providing a sequencing system with increased brightness or intensity as compared to prior systems which may have had relatively dim clusters because a nucleotide was labeled with two dyes at approximately 50-50% proportions. The increase in brightness may be quantified as an absolute measurement in terms of photon flux or counts per second. The increase in brightness may also be quantified as relative measurement for a cluster labeled with a single nucleotide as compared to a cluster labeled with two different nucleotides. For SNR optimization reasons, some sequencing systems employ a square constraint on a scatterplot of a two-channel detection, wherein a cluster labeled with a single nucleotide is constrained to have a similar intensity as a cluster labelled with two nucleotides (e.g. base C in a corner cloud). Such constraints in intensity/brightness of a cluster may be controlled by diluting labeled nucleotides with a non-fluorescing tag, such that all clusters are emitting at approximately half of the potential brightness. Removing this constraint according to this disclosure may result in a two-fold increase in intensity, which in turn may increase signal to noise ratio (SNR) by a factor of 6dB.

It should be realized that on any flowcell, the different clusters may have varying brightness. For example, some clusters can be bright, and some clusters can be dim in comparison to each other. In embodiments, the intensity values vary between base calling cycles and thus the classification of bright and dim may also change between cycles. Some examples of intensity value ratios of emissions between bright and dim clusters include 0.55:0.45, 0.60:0.40, 0.65:0.35, 0.70:0.30, 0.75:0.25, 0.80:0.20, 0.85:0.15, 0.90:0.10, and 0.95:0.05. During each sampling event (e.g., each illumination stage or each image acquisition stage), a detector may image clusters with different intensities or clusters generating different types of signals.

The brightness of any cluster may also be affected by fragment length distribution of the sample. The varying brightness of the cluster population can have the effect of elongating the 'on' populations in the base calling scatterplot. By way of example, without normalizing the level of amplification before trying to increase the brightness of all the clusters, some over-amplified AT rich sequences may be much brighter than similar GC rich sequences, and become even more 'over amplified' than the GC rich clusters. In some embodiments, it may be advantageous to normalize each cluster's intensity by its mean intensity in the first 10 cycles to reduce population intensity variation. For example, in the first ten cycles, for every non-guanine(G) base call, two radii can be calculated: the distance of the population intensity from the origin, and the distance of the corresponding Gaussian mean from the origin. Cluster scaling can include normalizing to the mean of the ratio of these two radii averaged over, for example, the first 10 cycles. All cluster intensities can be normalized by this scaling factor before phase correction and base calling are performed. Cluster scaling can advantageously increase throughput and decrease error rates, for example, for samples with large fragment length distributions.

It is also possible to improve the brightness of all clusters, for example, by carrying out a higher number of amplification cycles, or by changing the chemistry/fraction of the dyes, or by changing the detection scheme (two to three-channel detection as described herein).

In some embodiments, a method according to this disclosure may allow for each cluster to be labeled with a single dye, and that dye, or faction of that dye, may be chosen to increase the brightness of the cluster. In some embodiments, methods and systems may not require clusters to be labeled with two nucleotides. Some embodiments may result in an increase in the brightness of a cluster relative to an intensity of fluorescent emissions from a labeled nucleotide labelled with two fluorescent dyes, wherein the two fluorescent dyes are detected in two different channels. Some embodiments may result in an increase in the brightness of a cluster relative to an intensity of fluorescent emissions from a nucleotide forming a corner cloud in a two-excitation, two-channel detection method.

Some embodiments may include a first labeled nucleotide that is labelled with one fluorescent dye at a first intensity, wherein the first intensity is at least 10% higher than an intensity of fluorescent emissions from a labeled nucleotide labelled with at least two labels. In some embodiments, the first intensity may be at least 20% higher relative to a doubly labelled nucleotide. In some embodiments, the first intensity may be at least 30% higher relative to a doubly labelled nucleotide. In some embodiments, the first intensity may be at least 40% higher relative to a doubly labelled nucleotide. In some embodiments, the first intensity may be at least 50% higher relative to a doubly labelled nucleotide. In some embodiments, the first intensity may be at least 800% higher relative to a doubly labelled nucleotide. In some embodiments, the first intensity may be at least 810% higher relative to a doubly labelled nucleotide. In some embodiments, the first intensity may be at least 820% higher relative to a doubly labelled nucleotide.

In some embodiments, the increase in brightness for a cluster may be quantified by absolute or relative improvements in SNR. In general, SNR for a two channel two excitation sequencing system may be quantified as approximately 10 dB of discrimination between a dark base (e.g. unlabeled G) and a base with only one dye (e.g. T or A). In some embodiments, systems and methods according to the disclosure may provide improved SNR quantified as approximately 15 dB of discrimination between a dark base and a base with only one dye. In some embodiments, the improved SNR may be quantified as one of approximately 13, 13.5, 14, 14.5, 15, 15.5, 16, 16.5, and 17 dB. In some embodiments, the noise in the measure may be assumed to be the same for a two-channel or a three-channel system, and improvements to SNR may be attributed to increase in signal. In some embodiments, one or both of the signal and noise of the measurement may increase. For example, moving to a three-channel system and removing issues with preferential absorption of nucleotides may reduce noise in the measurement.

In some embodiments, the increase in brightness for a cluster may be quantified by a relative improvement in SNR of +1, +2, +3, +4, +5, +6 and/or +7 dB, relative to a two channel two excitation sequencing system. The SNR between the systems of this disclosure with a single label may be compared relative to an intensity of fluorescent emissions from a labeled nucleotide labelled with two fluorescent dyes (where one of the two labels is label to be compared. The SNR between the systems of this disclosure with a single label may be compared relative to a nucleotide with two fluorescent dyes that are detected in two different channels. The SNR between the systems of this disclosure with a single label may be compared relative to an intensity of fluorescent emissions from a nucleotide forming a corner cloud in a two-excitation, two-channel detection method.

Also presented herein are methods for evaluating the quality of a base call from a sequencing read, and methods of producing high quality base calls with efficient changes to existing sequencing systems. Quality scoring refers to the process of assigning a quality score to each base call. In some embodiments where four different nucleotides are detected using fewer than four different labels, base calling may employ different analytical approaches compared to systems using traditional four-label detection. As an example, SBS can be performed utilizing two-channel methods and systems described in U.S. Patent Application Publication No. 2013/0079232, By way of example, in embodiments that make use of two-channel detection, base calling may be performed by extracting image data from two images, rather than four. Similarly, in embodiments that make use of three-channel detection, base calling may be performed by extracting image data from three images, rather than four.

In general, dye chemistry, illumination, camera design and the number of images captured may impact overall base calling quality. Quality scoring is widely-used in DNA sequencing to determine the confidence in the correctness of a base call. Percentage of bases above Q30 (also written as % Q>30) implies the percentage of base calls that have a quality score of 30 or higher. A quality score of 30 in turn represents a three nines accuracy of base calls, or indicating a base call accuracy of 99.9%. Likewise, a quality score of 20 means a 99% accuracy of base calls.

In some aspects, the techniques described herein relate to a method, wherein a labeled nucleotide has a signal intensity extracted for each cluster found in the image. For example, for a given cluster location, the weighted average of the intensity of pixels in the cluster location can be determined to extract signal strength. For example, a weighted average of the center pixel and neighboring pixels can be performed. In some embodiments, the intensity or brightness is selected from the group consisting of brightness relative to neighboring pixels, brightness relative to a noise estimate, absolute brightness, brightness as a percentile, and extracted (bilinear) brightness. In preferred aspects, the brightness is brightness relative to neighboring pixels. In some such aspects, the neighboring pixels may be present within a defined radius. In some preferred aspects, the defined radius may be lowered in response to increased signal density. In such preferred aspects, the defined radius can be, for example, 2.0 pixels, 1.5 pixels, 1.0 pixel or less than 1.0 pixel. In certain aspects, ordering the signals comprises ordering the signals by detection count then ordering the signals by intensity. However, it will be apparent to one of skill in the art that other suitable methods of determining the brightness of a signal can be used.

In some aspects, the techniques described herein relate to a method, wherein the signal to noise ratio (SNR) between the first labeled nucleotide and the fourth unlabeled nucleotide is at least about 10 dB. In some aspects, the techniques described herein relate to a method, wherein the SNR between the first labeled nucleotide and the fourth unlabeled nucleotide is at least about 15 dB. In some aspects, the techniques described herein relate to a method, wherein the SNR between the second labeled nucleotide and the fourth unlabeled nucleotide is at least about 10 dB. In some aspects, the techniques described herein relate to a method, wherein the SNR between the second labeled nucleotide and the fourth unlabeled nucleotide is at least about 15 dB. In some aspects, the techniques described herein relate to a method, wherein the SNR between the third labeled nucleotide and the fourth unlabeled nucleotide is at least about 10 dB. In some aspects, the techniques described herein relate to a method, wherein the SNR between the third labeled nucleotide and the fourth unlabeled nucleotide is at least about 15 dB.

The error profile presented by three-channel data may be different from the error profile of two-channel data or four-channel data. Accordingly, presented herein are methods and systems for evaluating the quality of a base call from a sequencing read. In some embodiments, the sequencing read utilizes three-channel base calling. In some embodiments, the sequencing read utilizes two-channel base calling. The predictor values for each base call can be any suitable aspect that may indicate or predict the quality of the base call in a given sequencing run. For example, some suitable predictors are set forth in U.S. Patent Application Publication No. 2012/0020537 filed on Jan. 13, 2011 and entitled, "DATA PROCESSING SYSTEM AND METHODS." Suitable predictor values may include, for example: online overlap, purity, phasing, start, hexamer score, motif accumulation, endiness, approximate homopolymer, intensity decay, penultimate chastity, signal overlap with background (SOWB), and shifted purity G adjustment. Any suitable combination of the above predictor values can be used to quantify the quality of the improved base calling methods presented herein.

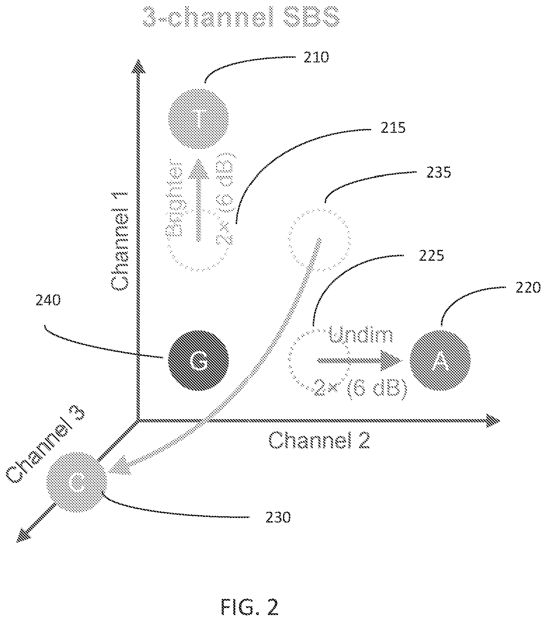

An aspect of the disclosure is directed to increasing the encoding space for base calls from two channels to three-channels. Fig. 2 shows a three-dimensional scatterplot of the potential encoding space of a three-channel sequencing method. The X and Y axes in Fig. 2 correspond to the X-Y axis for a traditional two excitation two channel sequencing system. For example, in a traditional two excitation two channel scatterplot, base T may be clustered at position 215, base A may be clustered at position 225, base C may be clustered at position 235, and base G may be unlabeled and clustered at position 240. When the corner base position 235 (in the top right corner of a traditional two excitation two channel scatterplot, but in the middle of Fig. 2) is shifted into a new third channel (Z-axis, coming out of the page) at position 230, the remaining bases are able to be undimmed. Compare brightness (intensity) of position 215 to the brightness of at position 210 for base T, and the brightness at position 220 to the brightness at position 225 for base A. In particular, a doubling in the intensity of the fluorescent emissions is feasible, which may yield a potential increase in SNR of about 6 dB for each of the two bases in the first and second channels. In some embodiments, the third base in the third channel may also have a relatively high intensity emission. In some embodiments, three bases have increased emission intensity and the SNR for the whole system is creased by 6 dB overall.

As described herein, an aspect of the disclosure is directed to increasing the SNR in base calls by increasing the intensity or brightness in base calls corresponding to the side clouds in comparison to traditional two excitation two channel sequencing methods and systems. Intensity or brightness may alternatively refer to absolute or relative improvements in SNR, intensity, pixel values, counts per second in a fluorimeter, when integrated over a wavelength range. A higher intensity or brightness may also be implemented high quantum yield of a fluorescent dye, and/or by increasing the size of cluster to be integrated.

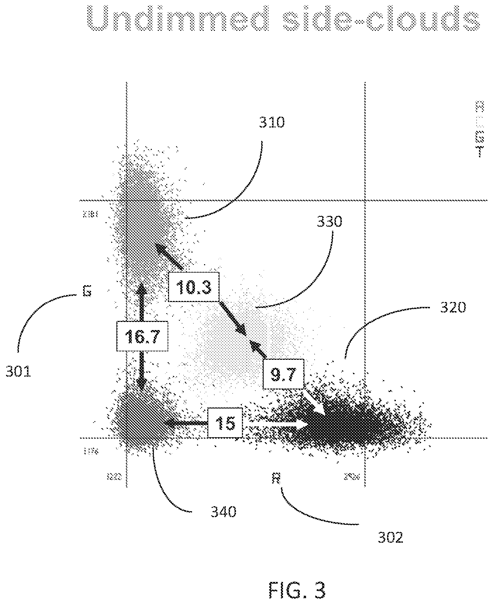

Fig. 3 shows a two-dimensional scatterplot of an experiment that demonstrates the potential signal to noise improvements (in decibels) from increasing the fraction of bright dyes on each base. The proof of concept data shown in this scatterplot was obtained using a NextSeq 2000 sequencing system. A standard set of fully functional nucleotides (ffNs) typically used in NextSeq 2000 systems were used here with the following modifications: i) the A and C bases were swapped, ii) the A dark ffA base was removed, thereby increasing the percentage of ffA bases incorporated with a blue dye from 50% to 800%, and iii) the green ffT was replaced with a brighter green dye that was approximately twice as bright as the standard dye. The results of the experiment are summarized in Fig. 3 itself, where the amount of SNR between each base is indicated in the numerical values shown between each cloud. The axes in Fig. 3 are a green channel 301, and a red channel 302. Fig. 3 shows that the SNR as compared between A (top left, cloud at position 310) and G (unlabeled, bottom left, cloud at position 340) is 16.7 dB. The SNR between T (bottom right, cloud at position 320) and G (unlabeled, bottom left, cloud at position 340) is 15 dB. In this experiment, C corresponding to cloud at position 330, is shown approximately in the middle of the scatterplot; however, base C or any other base may be gathered from another channel as illustrated in Fig. 2 at position 230.

### Long Stokes Shift Embodiments

In one embodiment, the sequencing system and method may use a long Stokes shift fluorescent dye to create a new, separate channel wherein an excitation using the first excitation wavelength is detected at a *second* detection wavelength (not the first detection wavelength). Thus, the system may use two conventional fluorescent dyes and one long Stokes shift dye. The first fluorescent dye is excited at the first excitation wavelength and emits light at the first detection wavelength. The second fluorescent dye is excited at the second excitation wavelength and emits light at the second detection wavelength. The third long Stokes shift dye is also excited at the first excitation wavelength, but emits light at the second detection wavelength. Thus, the first and third dyes are both excited by the same color light, but emit light at two different wavelengths that can be detected by two different detectors. Accordingly, each base may be excited and detected in turn by isolating the excitation and detection signals from each base, and each labeled base may be considered in a different channel. In some embodiments described herein, channel and detection wavelength may be used interchangeably, in some embodiments the two terms may be identical, however, a person of skill in the art will be able to distinguish the use of the two, where, for example, two detectors are configured to enable three channels.

In some embodiments, the techniques described herein relate to a method wherein the first detection wavelength is imaged by a first charge coupled device, and the second detection wavelength is imaged by a second charge coupled device. In some embodiments, the techniques described herein relate to a method, wherein exciting at the first excitation wavelength and detecting fluorescent emissions at the first emission wavelength is performed separately from exciting at the first excitation wavelength and detecting fluorescent emissions at the second emission wavelength. In some embodiments, the techniques described herein relate to a method, wherein exciting at the first excitation wavelength and detecting fluorescent emissions at the first emission wavelength is performed at the same time as exciting at the first excitation wavelength and detecting fluorescent emissions at the second emission wavelength.

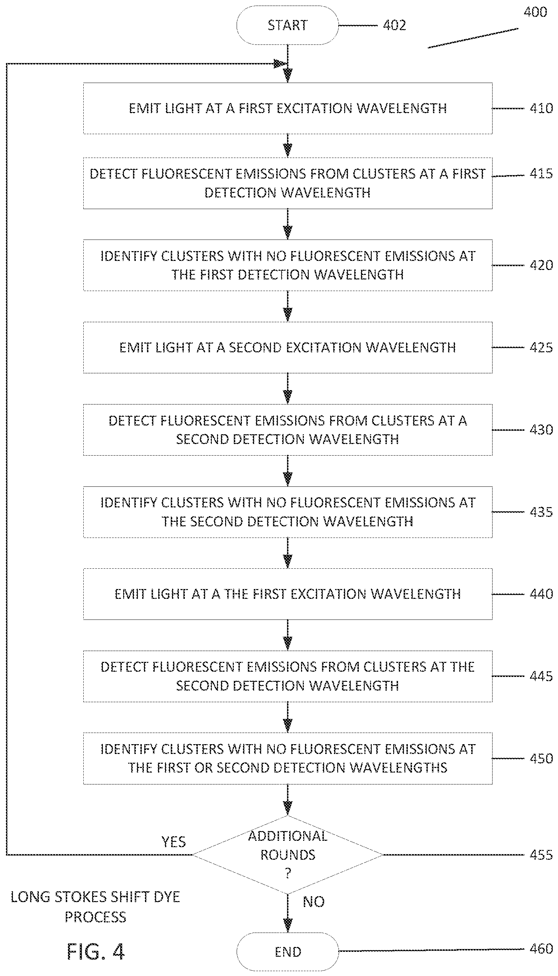

Fig. 4 is a flowchart of an example method sequencing clusters of labeled polynucleotides bound to a flowcell using long Stokes shift dyes. The method 400 may begin at a start step 402 by preparing a sequencing system with a sample of polynucleotides. In some embodiments, at step 410, method 400 may proceed to emit light at a first excitation wavelength. For example, at step 410, method 400 may proceed by exciting the clusters at a first excitation wavelength and detecting, at step 415, fluorescent emissions at a first detection wavelength from a first labeled nucleotide.

Method 400 then moves to a step 420, wherein the method 400 may proceed to identify clusters with no fluorescent emissions at the first detection wavelength. This step 420 may occur at any stage in the method, but may in general occur after excitations and emissions for the three or more other labelled nucleotides are detected. For example, step 420 may be repeated at step 435 and/or at step 450. A cluster may be identified by an emission from a previous step or iteration of method 400, but otherwise appear blank in a fluorescent image after the first, second, and/or third excitations. In some embodiments, at step 425, method 400 may proceed to emit light at a second excitation wavelength. For example, at step 425 method 400 may proceed by exciting the clusters at a second excitation wavelength and detecting, at step 430, fluorescent emissions at a second detection wavelength from a second labeled nucleotide. The method 400 then moves to step 435 wherein clusters with no fluorescent emissions can be detected in order to determine clusters which have incorporated an unlabeled nucleotide.

The method 400 can then move to step 440 to emit light at a first excitation wavelength. For example, at step 440, systems according to the disclosure may proceed by exciting the clusters at the first excitation wavelength. The method 400 then moves to a state 445 to detect any fluorescent emissions from a long Stokes shift fluorescent dye at the second detection wavelength. According to the disclosure, systems and methods disclosed herein may determine the nucleotide sequence of the labeled polynucleotides based on the detected fluorescent emissions.

In some embodiments, at step 450, the method may proceed by determining clusters that did not emit fluorescent emissions after excitation at the first, second and/or third excitation wavelengths to detect the presence of a fourth unlabeled nucleotide.

The method 400 can then move to a decision step 455, to determine if additional read cycles are required. If they are required, then the method 400 may loop to step 410 to emit light at the first excitation wavelength again. If a determination is made at the decision step 455 that no additional rounds are necessary, the method 400 may terminate at the end state 460.

In some aspects, the techniques described herein relate to a method, wherein the first excitation wavelength and the second excitation wavelength are different wavelengths. In some embodiments, the techniques described herein relate to a method wherein the first detection wavelength and the second detection wavelength are different wavelengths. In some embodiments, the techniques described herein relate to a method wherein the first labeled nucleotide, the second labeled nucleotide, and the third labeled nucleotide are each labeled with a single fluorescent dye.

In some embodiments, the techniques described herein relate to a method, wherein the third labeled nucleotide is labeled with a long Stokes shift dye. A long Stokes shift dye may be characterized by the shift in energy (e.g., in meV) or in the relative shift in nanometers. In some embodiments, a long Stokes shift dye has a Stokes shift ranging from about 340 meV to 850 meV. When described using nanometers, a person of skill in the art will realize that the energy shift of 50nm for a Stokes shift dye is different for an excitation wavelength at 300 nm versus an excitation wavelength at 600 nm. However, two emission bands may be distinguished by the resolution of filters or detectors, and can be reported in nanometers. For example, with respect to large Stokes shift dyes, in some embodiments, a suitable large Stokes shift for one of the dyes may range between 50-150 nm (e.g., if excited at 400 nm, a resulting emission max would be at 450-550 nm).

An aspect of the disclosure is directed to techniques employing nucleotides labeled with a chromenoquinoline dye. In some embodiments, the techniques described herein relate to a method wherein the first labeled nucleotide is labeled with dye NR455BoC.

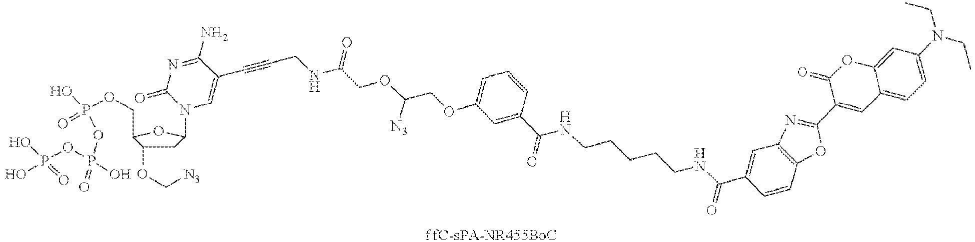

In some embodiments, the techniques described herein relate to a method to 20, wherein the second labeled nucleotide is labeled with dye NR550S0.

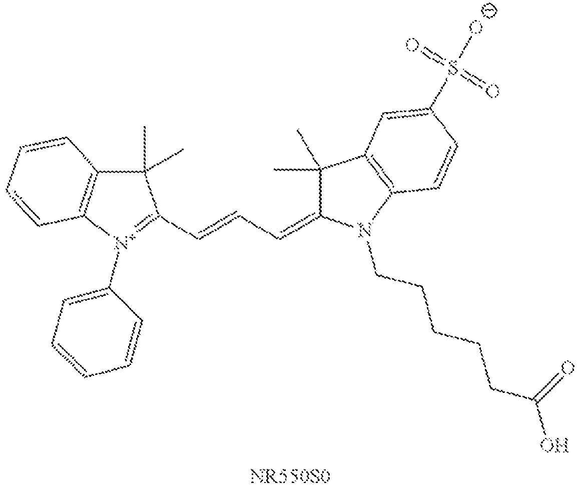

In some embodiments, the techniques described herein relate to a method, wherein the third labeled nucleotide is labeled with dye MC485CQ-O.

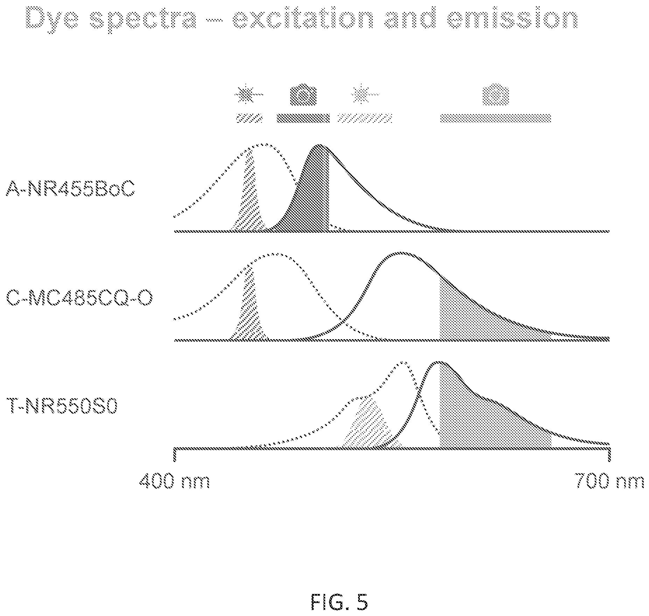

FIG. 5 shows an illustration of the emission spectra of a three-channel sequencing method employing a long Stokes shift dye. In some embodiments, a three channel SBS with a long Stokes shift dye may be implemented on Illumina's non-CMOS platforms. For example, Fig. 5 illustrates a full two-excitation, three-emission scheme with a long Stokes shift dye and other existing ffN dyes. In Fig. 5, a first nucleotide (ffA-NR455BoC) is fluorescent with blue excitation and blue imaging and can be assigned to a channel 1. Then a second nucleotide (ffT-NR550SO) may be fluorescent with green excitation with green imaging (channel 2), and a third nucleotide (ffC-MC485CQ-O) may be fluorescent with blue excitation and green imaging (channel 3). As in two-channel systems, a fourth nucleotide (for example, here ffG) may be dark and not fluoresce in any of the channels.

Making use of a long Stokes Shift dye enables benefits of three-channel systems, potentially without significant changes to existing sequencing platforms. Some modifications to step and shoot systems/methods, may include longer turnaround time for step and shoot systems, because a third image would be captured. For TDI line scanners, a third camera and associated optics may be added. For example, Step and shoot platforms may image three separate images, but may only require two cameras and may also only employ two excitation sources. In some embodiments, three excitation sources and two cameras may be employed.

In some embodiments, the techniques described herein relate to a method, wherein the detecting fluorescent emissions from the first labeled nucleotide, the second labeled nucleotide and the third labeled nucleotide are performed using a Time Delay Integration (TDI) imaging method. In some embodiments, the techniques described herein relate to a method, wherein the detecting fluorescent emissions from the first labeled nucleotide, the second labeled nucleotide and the third labeled nucleotide are performed using a step and shoot imaging method. Imaging steps may include moving a point focal region of light in a raster pattern across areas of interest in a flow cell.

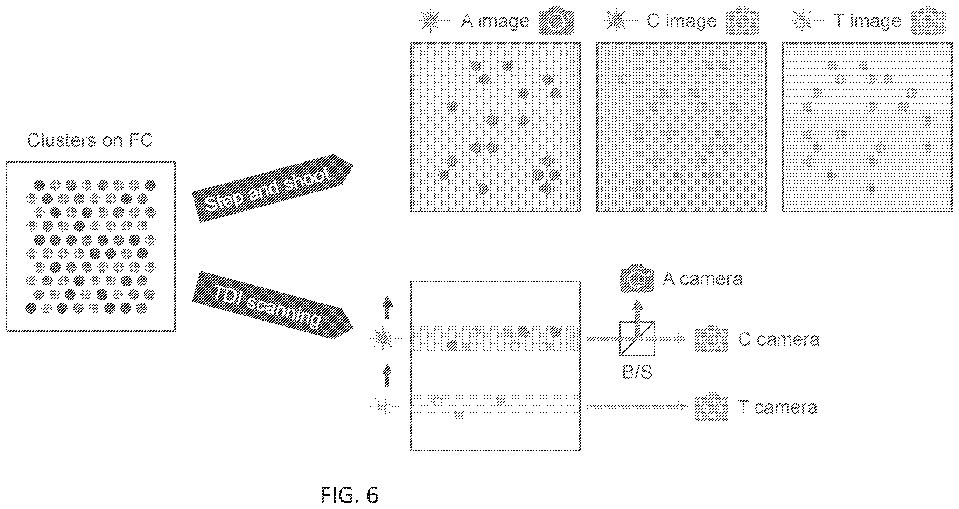

FIG. 6 shows an illustration of a three-channel sequencing method with two of many possible configurations, a step and shoot configuration and a Time Delay Integration (TDI) imager configuration. For example, as shown in the top of Fig. 6, one or more regions of the flow cell may be illuminated at one time in a "step and shoot" manner, where regions of a sample area may be individually imaged. In some embodiments, as shown in the bottom of Fig. 6, techniques may be used to effectively dwell on each sub-diffraction area being imaged long enough to detect sufficient photons to achieve a targeted or desirable signal to noise ratio.

TDI-like data acquisition may be employed to achieve long exposure time for points on a sample while simultaneously moving the sample. For example, an object may move past a series of sensors (e.g., where an individual sensor can be a pixel on a CCD chip, an individual photodiode, an individual PMT, or the like). As the object moves, photons may be collected on one of the sensors, and as the object moves from one sensor to the next, the accumulated signal may be shifted along the series of sensors. In some embodiments, TDI can be carried out by scanning a sample such that a frame transfer device produces a continuous video image of the sample by means of a stack of linear arrays aligned with and synchronized to the apparent movement of the sample, whereby as the image moves from one line to the next, the stored charge moves along with it. Exemplary TDI designs and methods that can be used in the invention are described in U.S. Pat. No. 5,754,991,

### General Methods

Nucleotides comprising a dye compound according to the present disclosure may be used in any method of analysis such as methods that include detection of a fluorescent label attached to such nucleotide, whether on its own or incorporated into or associated with a larger molecular structure or conjugate. In this context the term "incorporated into a polynucleotide" can mean that the 5' phosphate is joined in phosphodiester linkage to the 3' hydroxyl group of a second nucleotide, which may itself form part of a longer polynucleotide chain. The 3' end of a nucleotide set forth herein may or may not be joined in phosphodiester linkage to the 5' phosphate of a further nucleotide. Thus, in one non-limiting embodiment, the disclosure provides a method of detecting a labeled nucleotide incorporated into a polynucleotide which comprises: (a) incorporating at least one labeled nucleotide of the disclosure into a polynucleotide and (b) determining the identity of the nucleotide(s) incorporated into the polynucleotide by detecting the fluorescent signal from the dye compound attached to said nucleotide(s). Particular embodiments of the method of sequencing utilizes a one-excitation, two-channel detection system (also known as 1Ex-2Ch). Detailed disclosures are provided in WO 2018/165099 and U.S. Ser. No. 17/338590, In particular, the 1Ex-2Ch may contain a blue excitation light source having a wavelength between about 350 nm to about 360 nm, and two separate collection channels at both the blue and green regions (e.g., at a blue region with a wavelength ranging from about 372 to about 520 nm, and at a green region with a wavelength ranging from about 540 nm to about 640nm). However, 1Ex-2Ch and a two-excitation, two-channel detection system (2Ex-2Ch) configurations are not considered mutually exclusive and can be used in various combinations. For example, dyes described herein that used in 1Ex-2Ch may be used in 2Ex-2Ch configurations. For example, a fluorescent-based SBS method that uses a first nucleotide type that is detected in a first channel (e.g. dATP having a label that is detected in the first channel when excited by a first excitation wavelength), a second nucleotide type that is detected in a second channel (e.g. dTTP having a label that is detected in the second channel when excited by a second excitation wavelength), may employ a third nucleotide type according to the disclosure that is detected in one or both of the first and the second channel (e.g. dCTP having a label that is detected in at least one of the first, second or a third channel when excited by the first and/or second excitation wavelength) and a fourth nucleotide type that lacks a label that is not, or minimally, detected in either channel (e.g. dGTP having no label).

This method can include: a synthetic step (a) in which one or more labeled nucleotides according to the disclosure are incorporated into a polynucleotide and a detection step (b) in which one or more labeled nucleotide(s) incorporated into the polynucleotide are detected by detecting or quantitatively measuring their fluorescence.

Some embodiments of the present application are directed to a method for determining the sequences of a plurality of different target polynucleotides, comprising:
(a) contacting a solid support with a solution comprising sequencing primers under hybridization conditions, wherein the solid support comprises a plurality of different target polynucleotides immobilized thereon; and the sequencing primers are complementary to at least a portion of the target polynucleotides;
(b) contacting the solid support with an aqueous solution comprising DNA polymerase and one more of four different types of nucleotides (e.g., dATP, dGTP, dCTP and dTTP or dUTP), under conditions suitable for DNA polymerase-mediated primer extension, and incorporating one type of nucleotides into the sequencing primers to produce extended copy polynucleotides, wherein at least one type of nucleotide is a chromenoquinoline labeled nucleotide described herein, and wherein each of the four types of nucleotides comprises a 3' blocking group;
(c) imaging the solid support and performing one or more fluorescent measurements of the extended copy polynucleotides; and
(d) removing the 3' blocking group of the incorporated nucleotides. In some embodiments, step (d) also removes the labels of the incorporated nucleotides (if the incorporated nucleotides are labeled). In some such embodiments, the labels and the 3' blocking groups of the incorporated nucleotides are removed in a single chemical reaction. In some further embodiments, the method may also comprises (e) washing the solid support with an aqueous wash solution (e.g., washing the removed label moiety and the 3' blocking group away from the extended copy polynucleotides). In some embodiments, steps (b) through (e) are repeated at least 30, 40, 50, 60, 70, 80, 90, 800, 810, 820, 830, 840, 850, 860, 870, 880, 890, 900, 950, or 1000 cycles to determine the target polynucleotide sequences. In some embodiments, the four types of nucleotides comprise dATP, dCTP, dGTP and d'ITP or dUTP, or non-natural nucleotide analogs thereof. In some embodiments, the sequence determination is conducted after the completion of repeated cycles of the sequencing steps described herein. In some embodiments, the chromenoquinoline dyes described herein may be used as any one of the first, the second or the third label described in the method.

In some further embodiments, the method is performed on an automated sequencing instrument, and wherein the automated sequencing instrument comprises a single light source operating with a blue laser at about 350 nm to about 360 nm. The incorporation of the first type of the nucleotide is determined by detection in the one of the blue or green channel/region (e.g., at a blue region with a wavelength ranging from about 372 to about 520 nm, or at a green region with a wavelength ranging from about 540 nm to about 640nm). The incorporation of the second type of nucleotide is determined by detection in the other one of the blue or green detection channel/region. The incorporation of the third type of nucleotide is determined by detection in both the blue and green channels/regions. The incorporation of the fourth type of nucleotide is determined by no detection in either the blue or the green channel/region.

In other embodiments, the automatic sequencing instrument may comprise two light sources operating at different wavelengths (e.g., at 350-360 nm and 520-530 nm). The incorporation of the first type of the nucleotide conjugates is determined by a signal state in the first imaging event and a dark state in the second imaging event. The incorporation of the second type of the nucleotide conjugates is determined by a dark state in the first imaging event and a signal state in the second imaging event. The incorporation of the third type of the nucleotide conjugates is determined by a signal state in both the first imaging event and the second imaging event. The incorporation of the fourth type of the nucleotide conjugates is determined by a dark state in both the first imaging event and the second imaging event.

In some embodiments, at least one nucleotide is incorporated into a polynucleotide (such as a single stranded primer polynucleotide described herein) in the synthetic step by the action of a polymerase enzyme. However, other methods of joining nucleotides to polynucleotides, such as, for example, chemical oligonucleotide synthesis or ligation of labeled oligonucleotides to unlabeled oligonucleotides, can be used. Therefore, the term "incorporating," when used in reference to a nucleotide and polynucleotide, can encompass polynucleotide synthesis by chemical methods as well as enzymatic methods.

In a specific embodiment, a synthetic step is carried out and may optionally comprise incubating a template or target polynucleotide strand with a reaction mixture comprising fluorescently labeled nucleotides of the disclosure. A polymerase can also be provided under conditions which permit formation of a phosphodiester linkage between a free 3' hydroxyl group on a polynucleotide strand annealed to the template or target polynucleotide strand and a 5' phosphate group on the labeled nucleotide. Thus, a synthetic step can include formation of a polynucleotide strand as directed by complementary base pairing of nucleotides to a template/target strand.

In all embodiments of the methods, the detection step may be carried out while the polynucleotide strand into which the labeled nucleotides are incorporated is annealed to a template/target strand, or after a denaturation step in which the two strands are separated. Further steps, for example chemical or enzymatic reaction steps or purification steps, may be included between the synthetic step and the detection step. In particular, the polynucleotide strand incorporating the labeled nucleotide(s) may be isolated or purified and then processed further or used in a subsequent analysis. By way of example, polynucleotide strand incorporating the labeled nucleotide(s) as described herein in a synthetic step may be subsequently used as labeled probes or primers. In other embodiments, the product of the synthetic step set forth herein may be subject to further reaction steps and, if desired, the product of these subsequent steps purified or isolated.

Suitable conditions for the synthetic step will be well known to those familiar with standard molecular biology techniques. In one embodiment, a synthetic step may be analogous to a standard primer extension reaction using nucleotide precursors, including the labeled nucleotides as described herein, to form an extended polynucleotide strand (primer polynucleotide strand) complementary to the template/target strand in the presence of a suitable polymerase enzyme. In other embodiments, the synthetic step may itself form part of an amplification reaction producing a labeled double stranded amplification product comprised of annealed complementary strands derived from copying of the primer and template polynucleotide strands. Other exemplary synthetic steps include nick translation, strand displacement polymerization, random primed DNA labeling, etc. A particularly useful polymerase enzyme for a synthetic step is one that is capable of catalyzing the incorporation of the labeled nucleotides as set forth herein. A variety of naturally occurring or mutant/modified polymerases can be used. By way of example, a thermostable polymerase can be used for a synthetic reaction that is carried out using thermocycling conditions, whereas a thermostable polymerase may not be desired for isothermal primer extension reactions. Suitable thermostable polymerases which are capable of incorporating the labeled nucleotides according to the disclosure include those described in WO 2005/024010 or WO06120433, In synthetic reactions which are carried out at lower temperatures such as 37 °C, polymerase enzymes need not necessarily be thermostable polymerases, therefore the choice of polymerase will depend on a number of factors such as reaction temperature, pH, strand-displacing activity and the like.

In specific non-limiting embodiments, the disclosure encompasses methods of nucleic acid sequencing, re-sequencing, whole genome sequencing, single nucleotide polymorphism scoring, any other application involving the detection of the modified nucleotide or nucleoside labeled with dyes set forth herein when incorporated into a polynucleotide.

A particular embodiment of the disclosure provides use of labeled nucleotides comprising dye moiety according to the disclosure in a polynucleotide sequencing-by-synthesis reaction. Sequencing-by-synthesis generally involves sequential addition of one or more nucleotides or oligonucleotides to a growing polynucleotide chain in the 5' to 3' direction using a polymerase or ligase in order to form an extended polynucleotide chain complementary to the template/target nucleic acid to be sequenced. The identity of the base present in one or more of the added nucleotide(s) can be determined in a detection or "imaging" step. The identity of the added base may be determined after each nucleotide incorporation step. The sequence of the template may then be inferred using conventional Watson-Crick base-pairing rules. The use of the nucleotides labeled with dyes set forth herein for determination of the identity of a single base may be useful, for example, in the scoring of single nucleotide polymorphisms, and such single base extension reactions are within the scope of this disclosure.

In an embodiment of the present disclosure, the sequence of a template/target polynucleotide is determined by detecting the incorporation of one or more nucleotides into a nascent strand complementary to the template polynucleotide to be sequenced through the detection of fluorescent label(s) attached to the incorporated nucleotide(s). Sequencing of the template polynucleotide can be primed with a suitable primer (or prepared as a hairpin construct which will contain the primer as part of the hairpin), and the nascent chain is extended in a stepwise manner by addition of nucleotides to the 3' end of the primer in a polymerase-catalyzed reaction.

In particular embodiments, each of the different nucleotide triphosphates (A, T, G and C) may be labeled with a unique fluorophore and also comprises a blocking group at the 3' position to prevent uncontrolled polymerization. Alternatively, one of the four nucleotides may be unlabeled (dark). The polymerase enzyme incorporates a nucleotide into the nascent chain complementary to the template/target polynucleotide, and the blocking group prevents further incorporation of nucleotides. Any unincorporated nucleotides can be washed away and the fluorescent signal from each incorporated nucleotide can be "read" optically by suitable means, such as a charge-coupled device using light source excitation and suitable emission filters. The 3' blocking group and fluorescent dye compounds can then be removed (deprotected) (simultaneously or sequentially) to expose the nascent chain for further nucleotide incorporation. Typically, the identity of the incorporated nucleotide will be determined after each incorporation step, but this is not strictly essential. Similarly, U.S. Pat. No. 5,1002,509 discloses a method to sequence polynucleotides immobilized on a solid support.

The method, as exemplified above, utilizes the incorporation of fluorescently labeled, 3'-blocked nucleotides A, G, C, and T into a growing strand complementary to the immobilized polynucleotide, in the presence of DNA polymerase. The polymerase incorporates a base complementary to the target polynucleotide but is prevented from further addition by the 3'-blocking group. The label of the incorporated nucleotide can then be determined, and the blocking group removed by chemical cleavage to allow further polymerization to occur. The nucleic acid template to be sequenced in a sequencing-by-synthesis reaction may be any polynucleotide that it is desired to sequence. The nucleic acid template for a sequencing reaction will typically comprise a double stranded region having a free 3' hydroxyl group that serves as a primer or initiation point for the addition of further nucleotides in the sequencing reaction. The region of the template to be sequenced will overhang this free 3' hydroxyl group on the complementary strand. The overhanging region of the template to be sequenced may be single stranded but can be double-stranded, provided that a "nick is present" on the strand complementary to the template strand to be sequenced to provide a free 3' OH group for initiation of the sequencing reaction. In such embodiments, sequencing may proceed by strand displacement. In certain embodiments, a primer bearing the free 3' hydroxyl group may be added as a separate component (e.g., a short oligonucleotide) that hybridizes to a single-stranded region of the template to be sequenced. Alternatively, the primer and the template strand to be sequenced may each form part of a partially self-complementary nucleic acid strand capable of forming an intra-molecular duplex, such as for example a hairpin loop structure. Hairpin polynucleotides and methods by which they may be attached to solid supports are disclosed in PCT Publication Nos. WO0157248 and WO2005/047301, Nucleotides can be added successively to a growing primer, resulting in synthesis of a polynucleotide chain in the 5' to 3' direction. The nature of the base which has been added may be determined, particularly but not necessarily after each nucleotide addition, thus providing sequence information for the nucleic acid template. Thus, a nucleotide is incorporated into a nucleic acid strand (or polynucleotide) by joining of the nucleotide to the free 3' hydroxyl group of the nucleic acid strand via formation of a phosphodiester linkage with the 5' phosphate group of the nucleotide.

The nucleic acid template to be sequenced may be DNA or RNA, or even a hybrid molecule comprised of deoxynucleotides and ribonucleotides. The nucleic acid template may comprise naturally occurring and/or non-naturally occurring nucleotides and natural or non-natural backbone linkages, provided that these do not prevent copying of the template in the sequencing reaction.

In certain embodiments, the nucleic acid template to be sequenced may be attached to a solid support via any suitable linkage method known in the art, for example via covalent attachment. In certain embodiments template polynucleotides may be attached directly to a solid support (e.g., a silica-based support). However, in other embodiments of the disclosure the surface of the solid support may be modified in some way so as to allow either direct covalent attachment of template polynucleotides, or to immobilize the template polynucleotides through a hydrogel or polyelectrolyte multilayer, which may itself be non-covalently attached to the solid support.

Arrays in which polynucleotides have been directly attached to a support (for example, silica-based supports such as those disclosed in WO00/06770 wherein polynucleotides are immobilized on a glass support by reaction between a pendant epoxide group on the glass with an internal amino group on the polynucleotide. In addition, polynucleotides can be attached to a solid support by reaction of a sulfur-based nucleophile with the solid support, for example, as described in WO2005/047301 A still further example of solid-supported template polynucleotides is where the template polynucleotides are attached to hydrogel supported upon silica-based or other solid supports, for example, as described in WO00/31148, WOO01/01143, WO02/12566, WO03/014392, U.S. Pat. No. 6,365,878 and WO00/53812,

A particular surface to which template polynucleotides may be immobilized is a polyacrylamide hydrogel. Polyacrylamide hydrogels are described in the references cited above and in WO2005/065814, Specific hydrogels that may be used include those described in WO2005/065814 and U.S. Pub. No. 2014/0079923. In one embodiment, the hydrogel is PAZAM (poly(N-(5-azidoacetamidylpentyl) acrylamide-co-acrylamide)).

DNA template molecules can be attached to beads or microparticles, for example, as described in U.S. Pat. No. 6,872,918 Attachment to beads or microparticles can be useful for sequencing applications. Bead libraries can be prepared where each bead contains different DNA sequences. Exemplary libraries and methods for their creation are described in Nature, 337, 1076-1080 (2005); Science, 1009, 5741, 1728-1732 (2005), Sequencing of arrays of such beads using nucleotides set forth herein is within the scope of the disclosure.

Template(s) that are to be sequenced may form part of an "array" on a solid support, in which case the array may take any convenient form. Thus, the method of the disclosure is applicable to all types of high-density arrays, including single-molecule arrays, clustered arrays, and bead arrays. Nucleotides labeled with dye compounds of the present disclosure may be used for sequencing templates on essentially any type of array, including but not limited to those formed by immobilization of nucleic acid molecules on a solid support.

However, nucleotides labeled with dye compounds of the disclosure are particularly advantageous in the context of sequencing of clustered arrays. In clustered arrays, distinct regions on the array (often referred to as sites, or features) comprise multiple polynucleotide template molecules. Generally, the multiple polynucleotide molecules are not individually resolvable by optical means and are instead detected as an ensemble. Depending on how the array is formed, each site on the array may comprise multiple copies of one individual polynucleotide molecule (e.g., the site is homogenous for a particular single- or double-stranded nucleic acid species) or even multiple copies of a small number of different polynucleotide molecules (e.g., multiple copies of two different nucleic acid species). Clustered arrays of nucleic acid molecules may be produced using techniques generally known in the art. By way of example, WO 98/44151 and WO00/1 8957, describe methods of amplification of nucleic acids wherein both the template and amplification products remain immobilized on a solid support in order to form arrays comprised of clusters or "colonies" of immobilized nucleic acid molecules. The nucleic acid molecules present on the clustered arrays prepared according to these methods are suitable templates for sequencing using nucleotides labeled with dye compounds of the disclosure.

Nucleotides labeled with dye compounds of the present disclosure are also useful in sequencing of templates on single molecule arrays. The term "single molecule array" or "SMA" as used herein refers to a population of polynucleotide molecules, distributed (or arrayed) over a solid support, wherein the spacing of any individual polynucleotide from all others of the population is such that it is possible to individually resolve the individual polynucleotide molecules. The target nucleic acid molecules immobilized onto the surface of the solid support can thus be capable of being resolved by optical means in some embodiments. This means that one or more distinct signals, each representing one polynucleotide, will occur within the resolvable area of the particular imaging device used.

Single molecule detection may be achieved wherein the spacing between adjacent polynucleotide molecules on an array is at least 800 nm, more particularly at least 950 nm, still more particularly at least 1000 nm, even more particularly at least 1050 nm. Thus, each molecule is individually resolvable and detectable as a single molecule fluorescent point, and fluorescence from said single molecule fluorescent point also exhibits single step photobleaching.

The terms "individually resolved" and "individual resolution" are used herein to specify that, when visualized, it is possible to distinguish one molecule on the array from its neighboring molecules. Separation between individual molecules on the array will be determined, in part, by the particular technique used to resolve the individual molecules. The general features of single molecule arrays will be understood by reference to published applications WO00/06770 and WO 01/57248, Although one use of the labeled nucleotides of the disclosure is in sequencing-by-synthesis reactions, the utility of such nucleotides is not limited to such methods. In fact, the labeled nucleotides described herein may be used advantageously in any sequencing methodology which requires detection of fluorescent labels attached to nucleotides incorporated into a polynucleotide.

In particular, nucleotides labeled with dye compounds of the disclosure may be used in automated fluorescent sequencing protocols, particularly fluorescent dye-terminator cycle sequencing based on the chain termination sequencing method of Sanger and co-workers. Such methods generally use enzymes and cycle sequencing to incorporate fluorescently labeled dideoxynucleotides in a primer extension sequencing reaction. So-called Sanger sequencing methods, and related protocols (Sanger-type), utilize randomized chain termination with labeled dideoxynucleotides.

Thus, the present disclosure also encompasses nucleotides labeled with dye compounds which are dideoxynucleotides lacking hydroxyl groups at both of the 3' and 2' positions, such modified dideoxynucleotides being suitable for use in Sanger type sequencing methods and the like.

Nucleotides labeled with dye compounds of the present disclosure incorporating 3' blocking groups, it will be recognized, may also be of utility in Sanger methods and related protocols since the same effect achieved by using dideoxy nucleotides may be achieved by using nucleotides having 3' OH blocking groups: both prevent incorporation of subsequent nucleotides. Where nucleotides according to the present disclosure, and having a 3' blocking group are to be used in Sanger-type sequencing methods it will be appreciated that the dye compounds or detectable labels attached to the nucleotides need not be connected via cleavable linkers, since in each instance where a labeled nucleotide of the disclosure is incorporated; no nucleotides need to be subsequently incorporated and thus the label need not be removed from the nucleotide.

Alternatively, the sequencing methods described herein may also be carried out using unlabeled nucleotides and affinity reagents containing a fluorescent dye described herein. For example, one, two, three or each of the four different types of nucleotides (e.g., dATP, dCTP, dGTP and dTTP or dUTP) in the incorporation mixture of step (a) may be unlabeled. Each of the four types of nucleotides (e.g., dNTPs) has a 3' blocking group to ensure that only a single base can be added by a polymerase to the 3' end of the primer polynucleotide. After incorporation of an unlabeled nucleotide in step (b), the remaining unincorporated nucleotides are washed away. An affinity reagent is then introduced that specifically recognizes and binds to the incorporated dNTP to provide a labeled extension product comprising the incorporated dNTP. Uses of unlabeled nucleotides and affinity reagents in sequencing by synthesis have been disclosed in WO 2018/129214 and WO 2020/097607. A modified sequencing method of the present disclosure using unlabeled nucleotides may include the following steps:
(a') contacting a solid support with a solution comprising sequencing primers under hybridization conditions, wherein the solid support comprises a plurality of different target polynucleotides immobilized thereon; and the sequencing primers are complementary to at least a portion of the target polynucleotides;
(b') contacting the solid support with an aqueous solution comprising DNA polymerase and one more of four different types of unlabeled nucleotides (e.g., dATP, dCTP, dGTP, and dTTP or dUTP) under conditions suitable for DNA polymerase-mediated primer extension, and incorporating one type of nucleotides into the sequencing primers to produce extended copy polynucleotides, and wherein each of the four types of nucleotides comprises a 3' blocking group;
(c') contacting the extended copy polynucleotides with a set of affinity reagents under conditions wherein one affinity reagent binds specifically to the incorporated unlabeled nucleotides to provide labeled extended copy polynucleotides;
(d') imaging the solid support and performing one or more fluorescent measurements of the extended copy polynucleotides; and
(e') removing the 3' blocking group of the incorporated nucleotides.

In some embodiments of the modified sequencing method described herein, the method further comprises removing the affinity reagents from the incorporated nucleotides. In still further embodiments, the 3' blocking group and the affinity reagent are removed in the same reaction. In some embodiments, the method further comprises a step (f') washing the solid support with an aqueous wash solution. In further embodiments, steps (b') through (f') are repeated at least 50, 800, 850, 900, 950 or 1000 cycles to determine the target polynucleotide sequences. In some embodiments, the set of affinity reagents may comprise a first affinity reagent that binds specifically to the first type of nucleotide, a second affinity reagent that binds specifically to the second type of nucleotide, and a third affinity reagent that binds specifically to the third type of nucleotide. In some further embodiments, each of the first, second and the third affinity reagents comprises a detectable labeled that is spectrally distinguishable. In some embodiments, the affinity reagents may include protein tags, antibodies (including but not limited to binding fragments of antibodies, single chain antibodies, bispecific antibodies, and the like), aptamers, knottins, affimers, or any other known agent that binds an incorporated nucleotide with a suitable specificity and affinity. In one embodiment, at least one affinity reagent is an antibody or a protein tag. In another embodiment, at least one of the first type, the second type, and the third type of affinity reagents is an antibody or a protein tag comprising one or more detectable labels (e.g., multiple copies of the same detectable label), wherein the detectable label is or comprises a chromenoquinoline dye moiety described herein.

### Systems

Disclosed herein are embodiments of a system for determining the nucleotide sequence of polynucleotides. In one embodiment, the system includes, or is in communication with, a single light source, two light sources and/or three light sources. Light sources may be any source of light such as a laser or a LED light source and may be configured to produce light at a pre-determined wavelength. The system can include, or is in communication with, at least one detector configured to detect two or more substantially different fluorescent emissions off different fluorophores attached to nucleotides. The system can cause the light source to generate light onto a nucleotide. The nucleotide may be identified as a first type when a first fluorescent emission is detected by the at least one detector. The nucleotide may be identified as a second type when a second fluorescent emission is detected by the at least one detector. The nucleotide can be identified as a third type when a third fluorescent emission is detected by the at least one detector. The nucleotide can be identified as a fourth type when a fourth fluorescent emission is detected by the at least one detector. At least two of the first fluorescent emission, the second fluorescent emission, the third fluorescent emission, and the fourth fluorescent emissions may have substantially different wavelengths.

In one embodiment, the system includes, or is in communication with, two light sources. In one embodiment, the system includes, or is in communication with, three light sources. In some embodiments, he three light sources may have excitation wavelengths independently selected from violet, blue, green, orange, red and infra-red. In some embodiments, a first excitation wavelength may be a blue excitation light source having a wavelength between about 350 nm to about 360 nm, and two separate collection channels at both the blue and green regions (e.g., at a blue region with a wavelength ranging from about 372 to about 520 nm, and at a green region with a wavelength ranging from about 540 nm to about 640nm). In some embodiments, the first excitation wavelength is between 300 nm to 540 nm. In some embodiments, the first detection wavelength is between 310 nm and 600 nm. In some embodiments, the second excitation wavelength is between 350 nm to 600 nm. In some embodiments, the first detection wavelength is between 360 nm to 660 nm. In some embodiments, the third excitation wavelength is between 520 nm to 700 nm. In some embodiments, the first detection wavelength is between 530 nm to 760 nm.

In some embodiments, the system may include only two light sources or additional light sources as needed. The light source can include lasers, light emitting diodes, or lamps. In one embodiment, a first light source may be a red laser, a second light source may be an infrared laser, and a third light source may be a green laser. In one embodiment, a first light source may be a red laser, a second light source may be an orange laser, and a third light source may be a green laser. In one embodiment, a light source has a wavelength from about 1090 nm to about 780 nm. A light source may have a wavelength from about 936 nm to about 1340 nm. A light source may have a wavelength from about 1090 nm to about 780 nm.

In some embodiments, each light source may be independently blocked or directed to a specific area on a flow cell. For example, a third light source can be blocked so that only the first light source and the second light source are directed to the desired point at a given point in time. Alternatively, all three light sources may be directed to the desired point at the same time. Additional components can be used for adjusting or controlling the light source such as filters, collimators, polarizers and density filters.

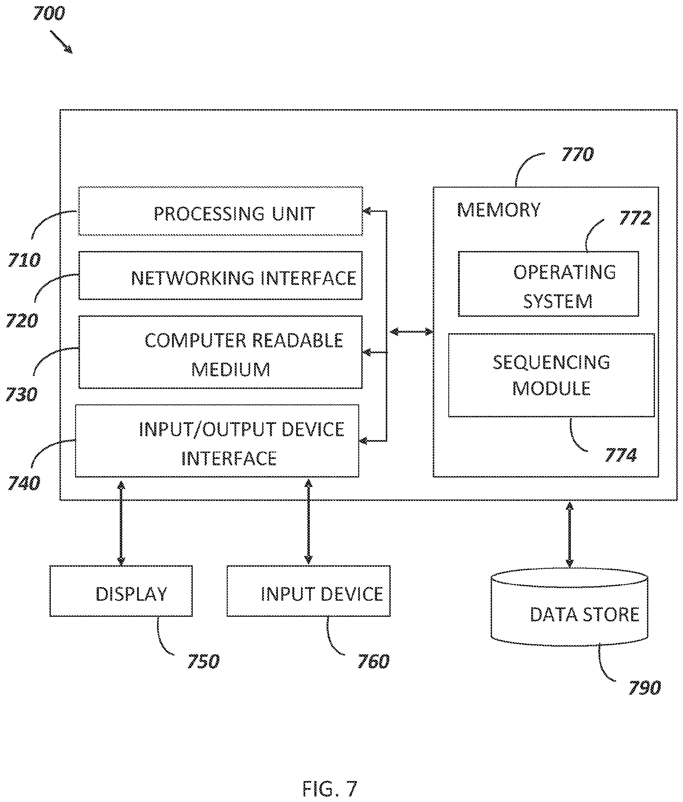

Embodiments of the present disclosure may also include a system for analyzing and assembling sequences of polynucleotides. Fig. 7 is a block diagram of an exemplary computing system 700 that may be used in connection with an illustrative sequencing system. The computing system 700 may be configured to determine a DNA sequence by using the sequencing and assembly methods disclosed herein. The general architecture of the computing system 700 depicted in Fig. 7 includes an arrangement of computer hardware and software components. The computing system 700 may include many more (or fewer) elements than those shown in Fig. 7. It is not necessary, however, that all of these generally conventional elements be shown in order to provide an enabling disclosure.

As illustrated, the computing system 700 includes a processing unit 710, a network interface 720, a computer-readable medium drive 730, an input/output device interface 740, a display 750, and an input device 760, all of which may communicate with one another by way of a communication bus. The network interface 770 may provide connectivity to one or more networks or computing systems. The processing unit 710 may thus receive information and instructions from other computing systems or services via a network. The processing unit 710 may also communicate to and from memory 770 and further provide output information for an optional display 750 via the input/output device interface 740. The input/output device interface 740 may also accept input from the optional input device 760, such as a keyboard, mouse, digital pen, microphone, touch screen, gesture recognition system, voice recognition system, gamepad, accelerometer, gyroscope, or other input device.

The memory 770 may contain computer program instructions (grouped as modules or components in some embodiments) that the processing unit 710 executes in order to implement one or more embodiments. The memory 770 generally includes RAM, ROM and/or other persistent, auxiliary or non-transitory computer-readable media. The memory 770 may store an operating system 772 that provides computer program instructions for use by the processing unit 710 in the general administration and operation of the computing device 700. The memory 770 may further include computer program instructions and other information for implementing aspects of the present disclosure.

For example, in one embodiment, the memory 770 includes a three-channel sequencing module 774 for analyzing and assembling sequences of polynucleotides. The three-channel sequencing module 774 can perform the methods disclosed herein, including the method described with respect to the flow diagrams of Figs. 1 or 4. In addition, memory 770 may include or communicate with the data store 790 and/or one or more other data stores that store one or more inputs, one or more outputs, and/or one or more results (including intermediate results) of determining a DNA sequence and providing an assembly process according to the present disclosure.

In some embodiments, the disclosed systems and methods may involve approaches for shifting or distributing certain sequence data analysis features and sequence data storage to a cloud computing environment or cloud-based network. User interaction with sequencing data, genome data, or other types of biological data may be mediated via a central hub that stores and controls access to various interactions with the data. In some embodiments, the cloud computing environment may also provide sharing of protocols, analysis methods, libraries, sequence data as well as distributed processing for sequencing, analysis, and reporting. In some embodiments, the cloud computing environment facilitates modification or annotation of sequence data by users. In some embodiments, the systems and methods may be implemented in a computer browser, on-demand or on-line.

In some embodiments, software written to perform the methods as described herein is stored in some form of computer readable medium, such as memory, CD-ROM, DVD-ROM, memory stick, flash drive, hard drive, SSD hard drive, server, mainframe storage system and the like.

In some embodiments, the methods may be written in any of various suitable programming languages, for example compiled languages such as C, C#, C++, Fortran, and Java. Other programming languages could be script languages, such as Perl, MatLab, SAS, SPSS, Python, Ruby, Pascal, Delphi, R and PHP. In some embodiments, the methods are written in C, C#, C++, Fortran, Java, Perl, R, Java or Python. In some embodiments, the method may be an independent application with data input and data display modules. Alternatively, the method may be a computer software product and may include classes wherein distributed objects comprise applications including computational methods as described herein.

In some embodiments, the methods may be incorporated into pre-existing data analysis software, such as that found on sequencing instruments. Software comprising computer implemented methods as described herein are installed either onto a computer system directly, or are indirectly held on a computer readable medium and loaded as needed onto a computer system. Further, the methods may be located on computers that are remote to where the data is being produced, such as software found on servers and the like that are maintained in another location relative to where the data is being produced, such as that provided by a third party service provider.

An assay instrument, desktop computer, laptop computer, or server which may contain a processor in operational communication with accessible memory comprising instructions for implementation of systems and methods. In some embodiments, a desktop computer or a laptop computer is in operational communication with one or more computer readable storage media or devices and/or outputting devices. An assay instrument, desktop computer and a laptop computer may operate under a number of different computer based operational languages, such as those utilized by Apple based computer systems or PC based computer systems. An assay instrument, desktop and/or laptop computers and/or server system may further provide a computer interface for creating or modifying experimental definitions and/or conditions, viewing data results and monitoring experimental progress. In some embodiments, an outputting device may be a graphic user interface such as a computer monitor or a computer screen, a printer, a hand-held device such as a personal digital assistant (i.e., PDA, Blackberry, iPhone), a tablet computer (for example, iPAD), a hard drive, a server, a memory stick, a flash drive and the like.

A computer readable storage device or medium may be any device such as a server, a mainframe, a supercomputer, a magnetic tape system and the like. In some embodiments, a storage device may be located onsite in a location proximate to the assay instrument, for example adjacent to or in close proximity to, an assay instrument. For example, a storage device may be located in the same room, in the same building, in an adjacent building, on the same floor in a building, on different floors in a building, etc. in relation to the assay instrument. In some embodiments, a storage device may be located off-site, or distal, to the assay instrument. For example, a storage device may be located in a different part of a city, in a different city, in a different state, in a different country, etc. relative to the assay instrument. In embodiments where a storage device is located distal to the assay instrument, communication between the assay instrument and one or more of a desktop, laptop, or server is typically via Internet connection, either wireless or by a network cable through an access point. In some embodiments, a storage device may be maintained and managed by the individual or entity directly associated with an assay instrument, whereas in other embodiments a storage device may be maintained and managed by a third party, typically at a distal location to the individual or entity associated with an assay instrument. In embodiments as described herein, an outputting device may be any device for visualizing data.

An assay instrument, desktop, laptop and/or server system may be used itself to store and/or retrieve computer implemented software programs incorporating computer code for performing and implementing computational methods as described herein, data for use in the implementation of the computational methods, and the like. One or more of an assay instrument, desktop, laptop and/or server may comprise one or more computer readable storage media for storing and/or retrieving software programs incorporating computer code for performing and implementing computational methods as described herein, data for use in the implementation of the computational methods, and the like. Computer readable storage media may include, but is not limited to, one or more of a hard drive, a SSD hard drive, a CD-ROM drive, a DVD-ROM drive, a floppy disk, a tape, a flash memory stick or card, and the like. Further, a network including the Internet may be the computer readable storage media. In some embodiments, computer readable storage media refers to computational resource storage accessible by a computer network via the Internet or a company network offered by a service provider rather than, for example, from a local desktop or laptop computer at a distal location to the assay instrument.

In some embodiments, computer readable storage media for storing and/or retrieving computer implemented software programs incorporating computer code for performing and implementing computational methods as described herein, data for use in the implementation of the computational methods, and the like, is operated and maintained by a service provider in operational communication with an assay instrument, desktop, laptop and/or server system via an Internet connection or network connection.

In some embodiments, a hardware platform for providing a computational environment comprises a processor (i.e., CPU) wherein processor time and memory layout such as random access memory (i.e., RAM) are systems considerations. For example, smaller computer systems offer inexpensive, fast processors and large memory and storage capabilities. In some embodiments, graphics processing units (GPUs) can be used. In some embodiments, hardware platforms for performing computational methods as described herein comprise one or more computer systems with one or more processors. In some embodiments, smaller computer are clustered together to yield a supercomputer network.

In some embodiments, computational methods as described herein are carried out on a collection of inter- or intra-connected computer systems (i.e., grid technology) which may run a variety of operating systems in a coordinated manner. For example, the CONDOR framework (University of Wisconsin-Madison) and systems available through United Devices are exemplary of the coordination of multiple stand-alone computer systems for the purpose dealing with large amounts of data. These systems may offer Perl interfaces to submit, monitor and manage large sequence analysis jobs on a cluster in serial or parallel configurations. One aspect of the disclosure is directed to a workflow module that may be integrated into existing workflows. In some embodiments, a workflow module may be a three-channel sequencing module and may be integrated into a NGS sequence analysis platform, for example the DRAGEN^{™} Bio-ID platform from Illumina.

### Chromenoquinoline Dyes of Formula (I)

One aspect of the disclosure relates to chromenoquinoline dyes of Formula (1), and salts and mesomeric forms thereof: 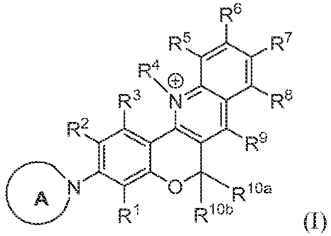
wherein ring A is a 4 to 10 membered heterocyclyl comprising at least one nitrogen atom, and ring A is optionally substituted with one or more R^{N};
each R^{N} is independently carboxyl, C₁-C₆ alkyl, substituted C₁-C₆ alkyl, C₁-C₆ alkoxy, substituted C₁-C₆ alkoxy, C₁-C₆ haloalkyl, C₁-C₆ haloalkoxy, (C₁-C₆ alkoxy)C₁-C₆ alkyl, -O(C₁-C₆ alkoxy)C₁-C₆ alkyl, optionally substituted amino, amino(C₁-C₆ alkyl), halo, cyano, hydroxy, hydroxy(C₁-C₆alkyl), nitro, sulfonyl, sulfo, sulfonate, S-sulfonamido, or N-sulfonamido;
each of R¹, R², R³, R⁵, R⁶, R⁷, R⁸, R⁹, R^{10a} and R^{10b} is independently H, C₁-C₆alkyl, substituted C₁-C₆alkyl, C₁-C₆alkoxy, substituted C₁-C₆alkoxy, C₂-C₆alkenyl, C₂-C₆alkynyl, C₁-C₆haloalkyl, C₁-C₆ haloalkoxy, (C₁-C₆alkoxy)C₁-C₆alkyl, -O-(C₁-C₆ alkoxy)C₁-C₆ alkyl, optionally substituted amino, amino(C₁-C₆ alkyl), halo, cyano, hydroxy, hydroxy(C₁-C₆ alkyl), nitro, sulfonyl, sulfo, sulfino, sulfonate, S-sulfonamido, N-sulfonamido, optionally substituted phenyl, optionally substituted 5 to 6 membered heteroaryl, optionally substituted C₃-C₇ cycloalkyl, or optionally substituted 4 to 7 membered heterocyclyl;
R⁴ is C₁-C₆ alkyl or substituted C₁-C₆ alkyl;
alternatively, R⁵ and R⁶ together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl,
alternatively, R⁶ and R⁷ together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl; and
alternatively, R⁷ and R⁸ together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl.

In some embodiments of the compound of Formula (I), the compound comprises a carboxyl group. In further embodiments, one of ring A, R⁴, R⁵, R⁶, R⁷ and R⁸ may comprise the carboxy group. In some other embodiments, a ring formed by R⁵ and R⁶ and the atoms to which they are attached may comprise the carboxyl group. In other embodiments, a ring formed by R⁶ and R⁷ and the atoms to which they are attached may comprise the carboxyl group. In other embodiments, a ring formed by R⁷ and R⁸ and the atoms to which they are attached may comprise the carboxyl group.

In some embodiments of the compound of Formula (1), ring A is 4 to 10 or 4 to 7 membered optionally substituted heterocyclyl comprising only one nitrogen atom. In other embodiments, ring A is 4 to 10 or 4 to 7 membered heterocyclyl comprising one or more nitrogen atom and one or more other heteroatoms (e.g., O or S). In further embodiments, ring A is 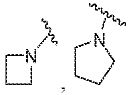 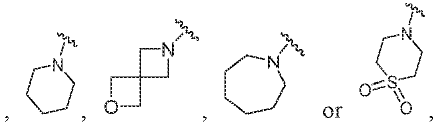 each optionally substituted with one R^{N}. In further embodiments, ring A is

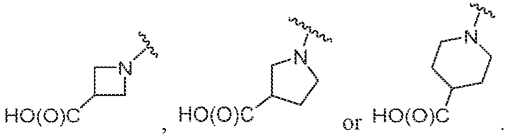

In some embodiments of the compound of Formula (I), R⁴ is C₁-C₆ alkyl or substituted C₁-C₆ alkyl. In one embodiment, R⁴ is methyl. In other embodiments, R⁴ is C₁-C₆ alkyl substituted with one or more substituents selected from the group consisting of carboxyl (-C(O)OH), carboxylate (-C(O)O⁻), sulfo (-SO₃H), sulfonate (-SO₃⁻), -C(O)OR^{a}, and -C(O)NR^{b}R^{c}, wherein R^{a} is optionally substituted C₁-C₆alkyl, optionally substituted C₆-C₁₀aryl, optionally substituted 5 to 10 membered heteroaryl, or optionally substituted C₃-C₇ cycloalkyl, and wherein each of R^{b} and R^{c} is independently H, optionally substituted C₁-C₆ alkyl, optionally substituted C₆-C₁₀ aryl, optionally substituted 5 to 10 membered heteroaryl, or optionally substituted C₃-C₇ cycloalkyl. In further embodiments, R⁴ is C₁-C₆ alkyl substituted with carboxyl, carboxylate, sulfo, sulfonate or -C(O)NR^{b}R^{c}, and wherein each R^{b} and R^{c} is independently C₁-C₆ alkyl substituted with carboxyl, carboxylate, -C(O)OR^{a}, sulfo or sulfonate.

In some embodiments of the compound of Formula (I), R¹ is H. In other embodiments, R¹ is -SO₃H or -SO₃⁻. In some embodiments, R² is H. In some embodiments, R³ is H.

In some embodiments of the compound of Formula (I), at least one of R⁵, R⁶, R⁷ and R⁸ is independently C₁-C₆ alkyl or C₁-C₆ alkoxy. In some instances, each of R⁵ and R⁷ is H, and each of R⁶ and R⁷ is methoxy. In other embodiments, at least one of R⁵, R⁶, R⁷ and R⁸ is substituted C₁-C₆ alkyl or substituted C₁-C₆ alkoxy, each substituted with carboxyl, carboxylate, sulfo, sulfonate, -C(O)OR³ or -C(O)NR^{b}R^{c}, and wherein each R^{b} and R^{c} is independently H or C₁-C₆ alkyl substituted with carboxyl, carboxylate, -C(O)OR^{a}, sulfo or sulfonate. In some instances, each of R⁵, R⁶ and R⁸ is H, and R⁷ is substituted C₁-C₆ alkyl or substituted C₁-C₆ alkoxy, each substituted with carboxyl, carboxylate, sulfo, sulfonate, -C(O)OR^{a} or -C(O)NR^{b}R^{c}. In still other embodiments, R⁶ and R⁷ together with the atoms to which they are attached form an optionally substituted 5 or 6 membered heterocyclyl, for example, optionally substituted 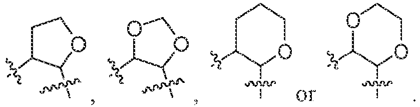 In other embodiments, R⁷ and R⁸ together with the atoms to which they are attached form an optionally substituted phenyl or six membered heteroaryl containing one or more nitrogen atoms. In one such embodiment, R⁷ and R⁸ together with the atoms to which they are attached form a phenyl. In other embodiment, R⁷ and R⁸ together with the atoms to which they are attached form a pyridyl.

In some embodiments of the compound of Formula (I), R⁹ is H, C₁-C₆ alkyl, or phenyl. In some embodiments, each of R^{10a} and R^{10b} is H.

In any embodiments of the compound of Formula (I), when a group is defined as a substituted C₁-C₆ alkyl, it may be a C₁, C₂, C₃, C₄, C₅ or C₆ alkyl (include but not limited to methyl, ethyl, isopropyl, n-propyl, n-butyl, 2-butyl, n-pentyl, 2-pentyl, n-hexyl, etc.) substituted with carboxyl, carboxylate, sulfo, sulfonate, -C(O)OR^{a} or -C(O)NR^{b}R^{c}. wherein R^{a} is C₁-C₆ alkyl optionally substituted with carboxyl, carboxylate, sulfo or sulfonate, and wherein each R^{b} and R^{c} is independently H or C₁-C₆ alkyl optionally substituted with carboxyl, carboxylate, -C(O)OR^{a}, sulfo or sulfonate.

Additional non-limiting examples of the compound of Formula (I) include: 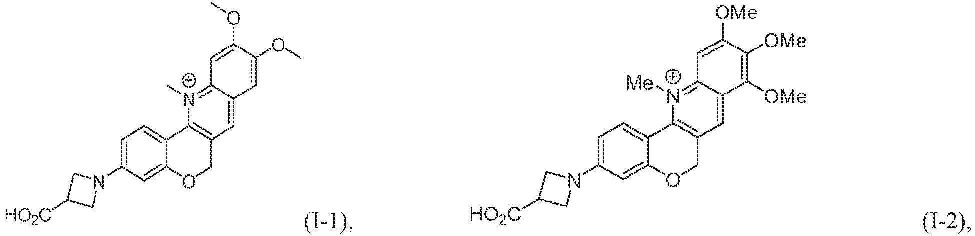 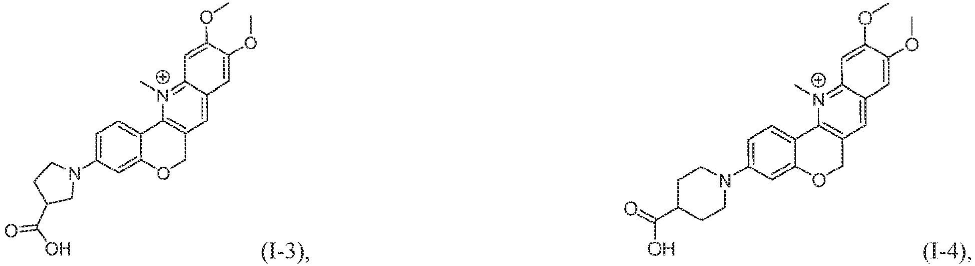 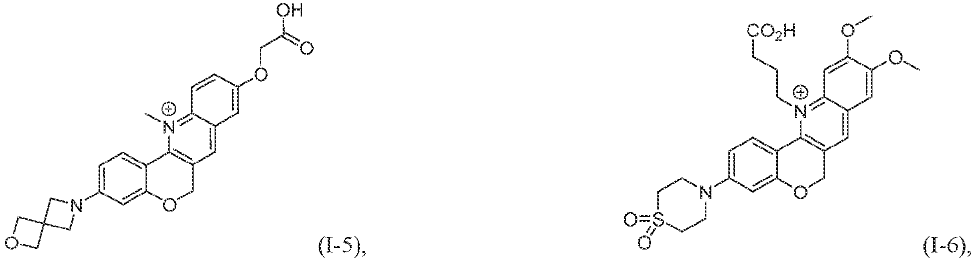 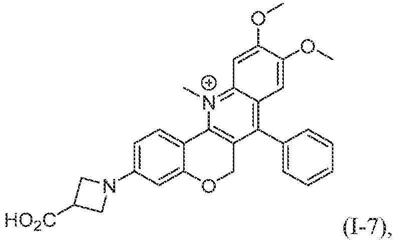 and salts and mesomeric forms thereof. For example, the salt form of compounds may each comprise an anion Z⁻. Non-limiting examples also include the carboxylated form (where the carboxylic acid group is deprotonated), and the corresponding C₁-C₆ alkyl carboxylic esters (such as methyl esters, ethyl esters isopropyl esters, and t-butyl esters formed from the carboxylic group of the compounds).

### Chromenoquinoline Dyes of Formula (IIa) or (IIb)

Another aspect of the disclosure relates to chromenoquinoline dyes of Formula (IIa) or (IIb), and salts and mesomeric forms thereof: 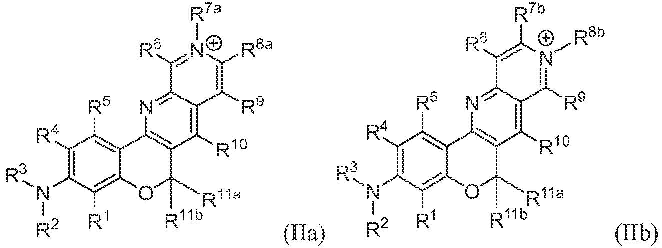
wherein each of R¹, R⁴, R⁵, R⁶, R^{7b}, R^{8a}, R⁹, R¹⁰, R^{11a} and R^{11b} is independently H, C₁-C₆alkyl, substituted C₁-C₆alkyl, C₁-C₆ alkoxy, substituted C₁-C₆ alkoxy, C₂-C₆ alkenyl, C₂-C₆ alkynyl, C₁-C₆haloalkyl, C₁-C₆haloalkoxy, (C₁-C₆alkoxy)C₁-C₆alkyl, -O(C₁-C₆alkoxy)C₁-C₆ alkyl, optionally substituted amino, amino(C₁-C₆alkyl), halo, cyano, hydroxy, hydroxy(C₁-C₆ alkyl), nitro, sulfonyl, sulfo, sulfino, sulfonate, S-sulfonamido, N-sulfonamido, optionally substituted phenyl, optionally substituted 5 to 6 membered heteroaryl, optionally substituted C₃-C₇ cycloalkyl, or optionally substituted 4 to 7 membered heterocyclyl;
each of R² and R³ is independently H, C₁-C₆alkyl, or substituted C₁-C₆alkyl, or R² and R³ together with the nitrogen atom to which they are attached form an optionally substituted 4 to 10 membered heterocyclyl comprising at least one nitrogen atom;
each of R^{7a} and R^{8b} is independently C₁-C₆alkyl or substituted C₁-C₆alkyl;
alternatively, R¹ and R² together with the atoms to which they are attached form an optionally substituted 5-10 membered heteroaryl or an optionally substituted 5-10 membered heterocyclyl;
alternatively, R³ and R⁴ together with the atoms to which they are attached form an optionally substituted 5-10 membered heteroaryl or an optionally substituted 5-10 membered heterocyclyl;
alternatively, R⁶ and R^{7b} together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl; and
alternatively, R^{8a} and R⁹ together with the atoms to which they are attached form an optionally substituted C₆-C₁₀ aryl, an optionally substituted 3-10 membered carbocyclyl, an optionally substituted 5-10 membered heteroaryl or an optionally substituted 3-10 membered heterocyclyl.

In some embodiments of the compound of Formula (IIa) or (IIb), the compound comprises a carboxyl group. In some embodiments of the compound of Formula (IIa), one of R² and R³, the 4 to 10 membered heterocyclyl formed by R², R³ and the nitrogen to which they are attached to, or R^{7a} may comprise a carboxyl group. In other embodiments of the compound of Formula (IIa), a ring formed by R^{8a} and R⁹ may comprise the carboxyl group. In some embodiments of the compound of Formula (IIb), one of R² and R³, the 4 to 10 membered heterocyclyl formed by R², R³ and the nitrogen to which they are attached to, or R^{8b} may comprise a carboxyl group. In other embodiments of the compound of Formula (IIb), a ring formed by R⁶ and R^{7b} may comprise the carboxyl group.

In some embodiments of the compound of Formula (IIa) or (IIb), R² is H and R³ is C₁-C₆alkyl or substituted C₁-C₆ alkyl. In other embodiments, each of R² and R³ is independently C₁-C₆ alkyl or substituted C₁-C₆ alkyl. For example, C₁-C₆ alkyl may be substituted with one or more substituents selected from the group consisting of carboxyl (-C(O)OH), carboxylate (-C(O)O⁻), sulfo (-SO₃H), sulfonate (-SO₃⁻), -C(O)OR^{a}, and -C(O)NR^{b}R^{c}, wherein R^{a} is optionally substituted C₁-C₆ alkyl, optionally substituted C₆-C₁₀ aryl, optionally substituted 5 to 10 membered heteroaryl, or optionally substituted C₃-C₇ cycloalkyl, and wherein each of R⁶ and R^{c} is independently H, optionally substituted C₁-C₆alkyl, optionally substituted C₆-C₁₀ aryl, optionally substituted 5 to 10 membered heteroaryl, or optionally substituted C₃-C₇ cycloalkyl. In further embodiments, C₁-C₆ alkyl may be substituted with carboxyl, carboxylate, sulfo, sulfonate or -C(O)NR^{b}R^{c}, and wherein each R^{b} and R^{c} is independently C₁-C₆ alkyl substituted with carboxyl, carboxylate, -C(O)OR^{a}, sulfo or sulfonate.

In some embodiments of the compound of Formula (IIa) or (IIb), the compound of Formula (IIa) or (IIb) is also represented by Formula (IIa-1) or (11b-1), a salt, or a mesomeric form thereof: 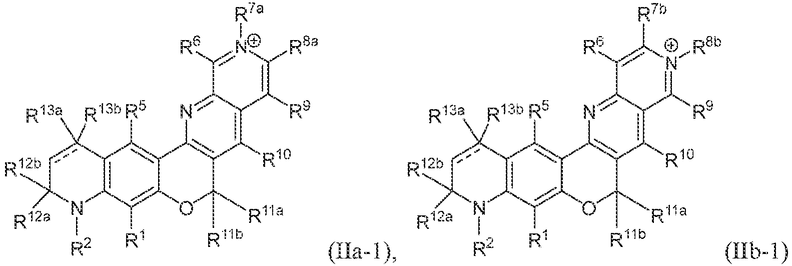
wherein each of R^{12a}, R^{12b}, R^{13a} and R^{13b} is independently H, C₁-C₆alkyl, substituted C₁-C₆ alkyl, C₁-C₆ alkoxy, C₂-C₆ alkenyl, C₂-C₆ alkynyl, C₁-C₆ haloalkyl, C₁-C₆ haloalkoxy, (C₁-C₆ alkoxy)C₁-C₆ alkyl, -O(C₁-C₆ alkoxy)C₁-C₆ alkyl, optionally substituted amino, amino(C₁-C₆ alkyl), halo, cyano, hydroxy, hydroxy(C₁-C₆ alkyl), nitro, sulfonyl, sulfo, sulfino, sulfonate, S-sulfonamido, or N-sulfonamido, and
the bond represented by a solid and dashed line -̅ -̅ -̅ -̅ -̅ is selected from the group consisting of a single bond and a double bond, provided that when -̅ -̅ -̅ -̅ -̅ is a double bond, then R^{13b} is absent.

In some embodiments of the compound of Formula (IIa-1) or (IIb-1), the bond represented by a solid and dashed line -̅ -̅ -̅ -̅ -̅ is a double bond. In some such embodiments, R^{13a} is H or C₁-C₆alkyl. In other embodiments, the bond represented by a solid and dashed line -̅ -̅ -̅ -̅ -̅ is a single bond. In some such embodiments, R^{13a} is H and R^{13b} is C₁-C₆ alkyl, or each of R^{13a} and R^{13b} is H. In some embodiments, each of R^{12a} and R^{12b} is H, or each of R^{12a} and R^{12b} is C₁-C₆ alkyl. In some embodiments, R² is C₁-C₆ alkyl or substituted C₁-C₆ alkyl. In further embodiments, R² is C₁-C₆ alkyl substituted with one or more substituents selected from the group consisting of carboxyl, carboxylate, sulfo, sulfonate, -C(O)OR^{a}, and -C(O)NR^{b}R^{c}, wherein R^{a} is optionally substituted C₁-C₆ alkyl, optionally substituted C₆-C₁₀ aryl, optionally substituted 5 to 10 membered heteroaryl, or optionally substituted C₃-C₇ cycloalkyl, and wherein each of R^{b} and R^{c} is independently H, optionally substituted C₁-C₆ alkyl, optionally substituted C₆-C₁₀ aryl, optionally substituted 5 to 10 membered heteroaryl, or optionally substituted C₃-C₇ cycloalkyl. In further embodiments, R² is C₁-C₆ alkyl substituted with carboxyl, carboxylate, sulfo, sulfonate or -C(O)NR^{b}R^{c}, and wherein each R^{b} and R^{c} is independently C₁-C₆alkyl substituted with carboxyl, carboxylate, -C(O)OR^{a}, sulfo or sulfonate.

In some embodiments of the compound of Formula (IIa), (IIa-1), (IIb) or (IIb-1), R¹ is H, sulfo or sulfonate. In other embodiments, R¹ and R² are joined together with the atoms to which they are attached to form an optionally substituted 6 membered heterocyclyl. In further embodiments, the 6 membered heterocyclyl contains only one nitrogen atom and is either unsubstituted or substituted with one or more C₁-C₆ alkyl.

In some other embodiments of the compound of Formula (IIa) or (IIb), the compound of Formula (IIa) or (IIb) is also represented by Formula (IIa-2) or (IIb-2), a salt, or a mesomeric form thereof: 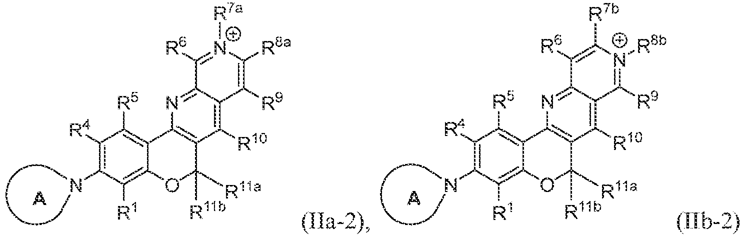
wherein ring A is a 4 to 10 membered heterocyclyl comprising at least one nitrogen atom, and ring A is optionally substituted with one or more R^{N}; and
each R^{N} is independently carboxyl, C₁-C₆alkyl, substituted C₁-C₆ alkyl, C₁-C₆ alkoxy, substituted C₁-C₆ alkoxy, C₁-C₆ haloalkyl, C₁-C₆ haloalkoxy, (C₁-C₆ alkoxy)C₁-C₆ alkyl, - O(C₁-C₆ alkoxy)C₁-C₆ alkyl, optionally substituted amino, amino(C₁-C₆ alkyl), halo, cyano, hydroxy, hydroxy(C₁-C₆ alkyl), nitro, sulfonyl, sulfo, sulfonate, S-sulfonamido, or N-sulfonamido.

In some embodiments of the compound of Formula (IIa-2) or (IIb-2), ring A is a 4 to 10 membered or 4 to 7 membered optionally substituted heterocyclyl comprising only one nitrogen atom. In other embodiments, ring A is 4 to 10 membered or 4 to 7 membered heterocyclyl comprising one or more nitrogen atom and one or more other heteroatoms (e.g., O or S). In further embodiments, ring A is 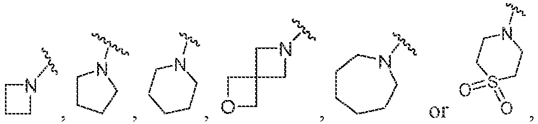 each optionally substituted with one R^{N}. In further embodiments, ring A is

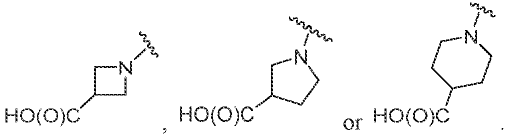

In some embodiments of the compound of Formula (IIa), (IIa-1), (IIa-2), (IIb), (IIb-1) or (IIb-2), each of R^{7a} and R^{8b} is independently C₁-C₆alkyl or substituted C₁-C₆alkyl. In some such embodiments, each of R^{7a} and R^{8b} is methyl or ethyl. In other embodiments, each of R^{7a} and R^{8b} is independently C₁-C₆alkyl substituted with one or more substituents selected from the group consisting of carboxyl, carboxylate, sulfo, sulfonate, -C(O)OR^{a}, and -C(O)NR^{b}R^{c}. In further embodiments, R^{a} is C₁-C₆ alkyl, and wherein each R^{b} and R^{c} is independently C₁-C₆ alkyl substituted with carboxyl, carboxylate, sulfo or sulfonate.

In some embodiments of the compound of Formula (IIa), (IIa-1), (IIa-2), (IIb), (IIb-1) or (IIb-2), each of R⁶, R^{7b}, R^{8a} and R⁹ is H. In other embodiments, R^{8a} and R⁹ together with the atoms to which they are attached form an optionally substituted phenyl or six membered heteroaryl containing one or more nitrogen atoms (e.g., pyridyl). In one embodiment, R^{8a} and R⁹ together with the atoms to which they are attached form a phenyl. In other embodiments, R⁶ and R^{7b} together with the atoms to which they are attached form an optionally substituted phenyl or six membered heteroaryl containing one or more nitrogen atoms (e.g., pyridyl). In one embodiment, R⁶ and R^{7b} together with the atoms to which they are attached form a phenyl.

In some embodiments of the compound of Formula (IIa), (IIa-1), (IIa-2), (IIb), (IIb-1) or (IIb-2), each of R^{11a} and R^{11b} is H.

In any embodiments of the compound of Formula (IIa), (IIa-1), (IIa-2), (IIb), (IIb-1) or (IIb-2), when a group is defined as a substituted C₁-C₆alkyl, it may be a C₁, C₂, C₃, C₄, C₅ or C₆ alkyl (include but not limited to methyl, ethyl, isopropyl, n-propyl, n-butyl, 2-butyl, n-pentyl, 2-pentyl, n-hexyl, etc.) substituted with carboxyl, carboxylate, sulfo, sulfonate, -C(O)OR^{a} or -C(O)NR^{b}R^{c}, wherein R^{a} is C₁-C₆ alkyl optionally substituted with carboxyl, carboxylate, sulfo or sulfonate, and wherein each R^{b} and R^{c} is independently H or C₁-C₆ alkyl optionally substituted with carboxyl, carboxylate, -C(O)OR^{a}, sulfo or sulfonate.

Additional non-limiting examples of the compound of Formula (IIa) or (IIb) include: 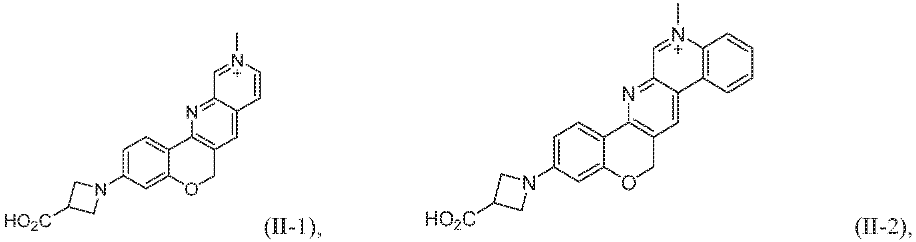 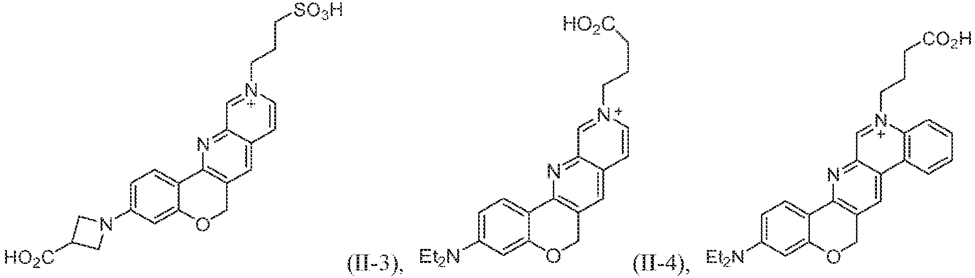 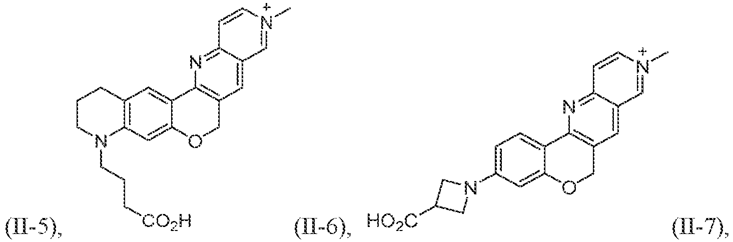 and salts and mesomeric forms thereof. For example, the salt form of compounds II-1 through 11-7 may each comprise an anion Z⁻. In other instances, those compounds with -SO₃H may be deprotonated as -SO₃⁻ so the compound as a whole is in neutral form.

### Cyclooctatetraene (COT) Photo-protecting Moieties

Another aspect of the present disclosure relates to the chromenoquinoline dyes of Formula (IIa), (IIa-1), (IIa-2), (IIb), (IIb-1) or (IIb-2) as described herein, where the dye is further modified by covalently attached to a photo-protecting cyclooctatetraene moiety via an amide bond, wherein the photo-protecting cyclooctatetraene moiety comprises the structure of formula (III): 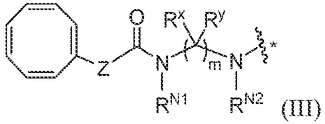
wherein Z is absent, optionally substituted C₂₋₆ alkenylene, or optionally substituted C₂₋₆ alkynylene;
each R^{x} and R^{y} is independently H, carboxyl, carboxylate, amino, sulfo, sulfonate, - C(O)OR^{a}, or -C(O)NR^{b}R^{c}. or C₁-C₆ alkyl substituted with amino, carboxyl, carboxylate, sulfo, sulfonate, -C(O)OR^{a}, or -C(O)NR^{b}R^{c},
each R^{N1} and R^{N2} is independently H or C₁-C₆ alkyl substituted with amino, carboxyl, carboxylate, sulfo, sulfonate, -C(O)OR^{a}, or -C(O)NR^{b}R^{c};
R^{a} is optionally substituted C₁-C₆ alkyl, optionally substituted C₆-C₁₀ aryl, optionally substituted 5 to 10 membered heteroaryl, or optionally substituted C₃-C₇ cycloalkyl;
each of R^{b} and R^{c} is independently H, optionally substituted C₁-C₆ alkyl, optionally substituted C₆-C₁₀ aryl, optionally substituted 5 to 10 membered heteroaryl, or optionally substituted C₃-C₇ cycloalkyl;
the carbon atom to which R^{x} and R^{y} are attached in 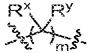 is optionally replaced with O, S, or N, provided that when said carbon atom is replaced with O or S, then R^{x} and R^{y} are both absent; when said carbon atom is replaced with N, then R^{y} is absent; and
m is an integral number between 1 and 10;
wherein the asterisk * indicates the attachment point of the photo-protecting cyclooctatetraene moiety to the carbonyl group of amide bond formed by the reaction of an amino group of the photo-protecting cyclooctatetraene moiety with the carboxyl group of the chromenoquinoline compounds described herein.

In some further embodiments, the photo-protecting COT moiety may comprise the following structures:

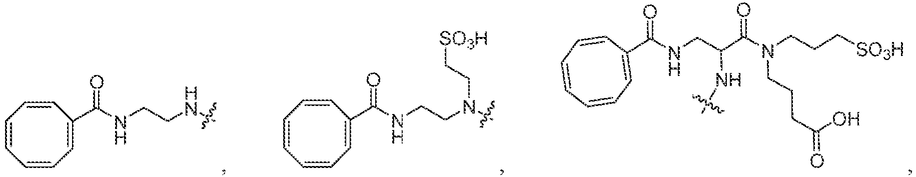

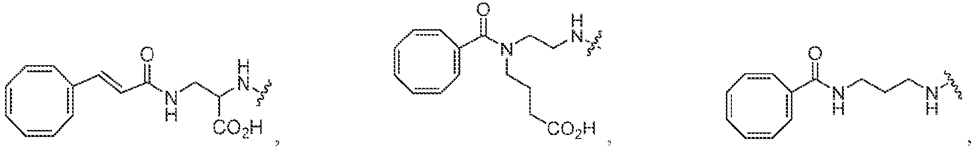

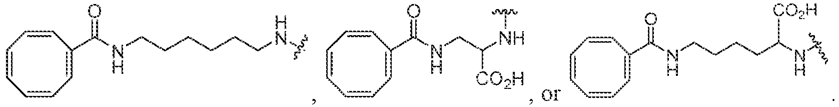

As a nonlimiting example, a COT moiety protected compound I-1 may have the following structure: 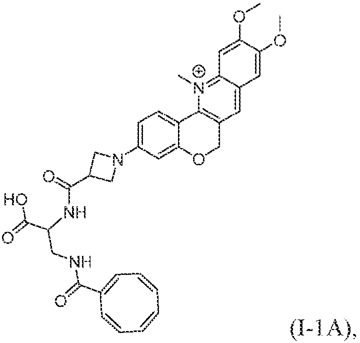 or a salt or mesomeric form thereof.

The COT moiety may be covalently attached to the chromenoquinoline dye described herein by reaction between a functional group of the chromenoquinoline dye described herein (e.g., a carboxyl group) and an amino group of a COT derivative to form an amide bond (where the carbonyl group of the amide bond is not shown). Alternatively, the chromenoquinoline dye described herein may have an amino group (instead of a carboxyl group) that forms the amide bond with a carboxyl group of the COT derivative.

### Labeled Nucleotides

According to an aspect of the disclosure, chromenoquinoline dye compounds described herein are suitable for attachment to substrate moieties, particularly comprising linker groups to enable attachment to substrate moieties. Substrate moieties can be virtually any molecule or substance to which the dyes of the disclosure can be conjugated, and, by way of non-limiting example, may include nucleosides, nucleotides, polynucleotides, carbohydrates, ligands, particles, solid surfaces, organic and inorganic polymers, chromosomes, nuclei, living cells, and combinations or assemblages thereof. The dyes can be conjugated by an optional linker by a variety of means including hydrophobic attraction, ionic attraction, and covalent attachment. In some aspect, the dyes are conjugated to the substrate by covalent attachment. More particularly, the covalent attachment is by means of a linker group. In some instances, such labeled nucleotides are also referred to as "modified nucleotides."

Some aspects of the present disclosure relate to a nucleotide labeled with a dye of Formula (IIa), (IIa-1), (IIa-2), (IIb), (IIb-1) or (IIb-2), or a salt of mesomeric form thereof as described herein, or a derivative thereof containing a photo-protecting moiety COT described herein. The labeled nucleotide may be attached to the dye compound disclosed herein via a carboxyl (-CO₂H) to form an amide bond. In some further embodiments, the carboxyl group may be in the form of an activated form of carboxyl group, for example, an amide or ester, which may be used for attachment to an amino or hydroxyl group of the nucleotide. The term "activated ester" as used herein, refers to a carboxyl group derivative which is capable of reacting in mild conditions, for example, with a compound containing an amino group. Non-limiting examples of activated esters include but not limited to p-nitrophenyl, pentafluorophenyl and succinimido esters.

For example, the chromenoquinoline dye compound of Formula (I) may be attached to the nucleotide via a carboxyl group of one of ring A, R⁴, R⁵, R⁶, R⁷ and R⁸, or a carboxyl group of a ring formed by R⁵ and R⁶ and the atoms to which they are attached, or a carboxyl group of a ring formed by R⁶ and R⁷ and the atoms to which they are attached, or a carboxyl group of a ring formed by R⁷ and R⁸ and the atoms to which they are attached.

In some such embodiments, R⁴ of Formula (I) comprises a -CO₂H or -(CH₂)₁₋₆-CO₂H and the attachment forms an amide moiety between the carboxyl functional group of R⁴ and the amino functional group of a nucleotide or a nucleotide linker. As one example, the labeled nucleotide may comprise the dye moiety of the following structure: 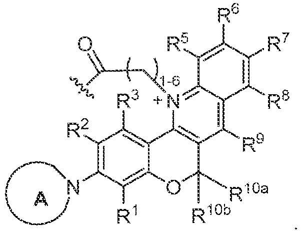 In other embodiments, ring A of Formula (1) comprises a -CO₂H or -(CH₂)₁₋₆-CO₂H and the attachment forms an amide using the -CO₂H group. For example, the labeled nucleotide may comprise the following dye moiety: 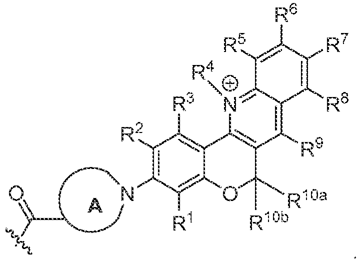 In other embodiments, R⁷ of Formula (I) comprises a -CO₂H or -(CH₂)₁₋₆-CO₂H and the attachment forms an amide using the -CO₂H group. For example, the labeled nucleotide may comprise the following dye moiety:

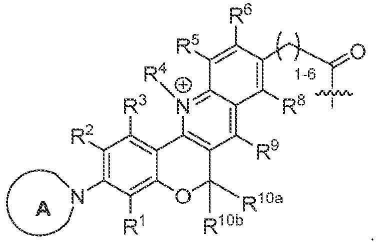

Similarly, the dye compound of Formula (IIa) may be attached to the nucleotide via a carboxyl group of one of R² and R³, the 4 to 10 membered heterocyclyl formed by R², R³ and the nitrogen to which they are attached to, or R^{7a}. In other embodiments, the compound of Formula (IIa) may be attached to the nucleotide via a carboxyl group of a ring formed by R^{8a} and R⁹. The dye of Formula (IIb) may be attached to the nucleotide via a carboxyl group of one of R² and R³, the 4 to 10 membered heterocyclyl formed by R², R³ and the nitrogen to which they are attached to, or R^{8b}. In other embodiments, the dye of Formula (IIb) may be attached to the nucleotide via a carboxyl group of a ring formed by R⁶ and R^{7b}. The attachment of the chromenoquinoline dyes is via an amide moiety formed between the carboxyl functional group of the chromenoquinoline dyes and an amino functional group of a nucleotide or a nucleotide linker. For example, R^{7a} of Formula (IIa) or R^{8b} of Formula (IIa) may comprise a -CO₂H or -(CH₂)₁₋₆-CO₂H and the attachment forms an amide using the -CO₂H group. Alternatively, R² of Formula (IIa-1) or (IIb-1) may comprise a -CO₂H or -(CH₂)₁₋₆-CO₂H and the attachment forms an amide using the -CO₂H group. In other instances, ring A of Formula (IIa-2) or (IIb-2) may comprise a -CO₂H or -(CH₂)₁₋₆-CO₂H and the attachment forms an amide using the -CO₂H group.

In some embodiments, the dye compounds may be covalently attached to nucleotides via the nucleotide base. In some such embodiments, the labeled nucleotide may have the dye attached to the C5 position of a pyrimidine base or the C7 position of a 7-deaza purine base, optionally through a linker moiety. For example, the nucleobase may be 7-deaza adenine, and the dye is attached to the 7-deaza adenine at the C7 position, optionally through a linker. The nucleobase may be 7-deaza guanine, and the dye is attached to the 7-deaza guanine at the C7 position, optionally through a linker. The nucleobase may be cytosine and the dye is attached to the cytosine at the C5 position, optionally through a linker. As another example, the nucleobase may be thymine or uracil and the dye is attached to the thymine or uracil at the C5 position, optionally through a linker.

### 3' Blocking Groups

The labeled nucleotide may also have a blocking group covalently attached to the ribose or deoxyribose sugar of the nucleotide. The blocking group may be attached at any position on the ribose or deoxyribose sugar. In particular embodiments, the blocking group is at the 3' OH position of the ribose or deoxyribose sugar of the nucleotide. Various 3' OH blocking group are disclosed in WO2004/018497 and WO2014/139596, For example, the blocking group may be azidomethyl (-CH₂N₃) or substituted azidomethyl (e.g., -CH(CHF₂)N₃ or CH(CH₂F)N₃), or allyl connecting to the 3' oxygen atom of the ribose or deoxyribose moiety. In some embodiments, the 3' blocking group is azidomethyl, forming 3'-OCH₂N₃ with the 3' carbon of the ribose or deoxyribose.

Additional 3' blocking groups are disclosed in U.S. Publication No. 2020/0216891 A1, Non-limiting examples of the 3' blocking group include: 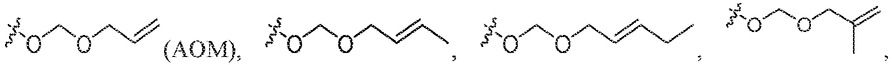 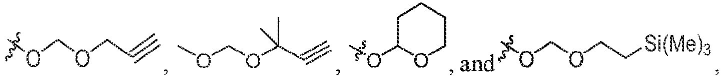 each covalently attached to the 3' carbon of the ribose or deoxyribose.

### Deprotection of the 3' Blocking Groups

In some embodiments, the 3' blocking group may be removed or deprotected by a chemical reagent to generate a free hydroxy group, for example, in the presence of a water soluble phosphine reagent. Non-limiting examples include tris(hydroxymethyl)phosphine (THMP), tris(hydroxyethyl)phosphine (THEP) or tris(hydroxylpropyl)phosphine (THP or THPP). 3'-acetal blocking groups described herein may be removed or cleaved under various chemical conditions. For 3' acetal blocking groups such as 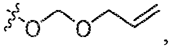 non-limiting cleaving condition includes a Pd(II) complex, such as Pd(OAc)₂ or allylPd(II) chloride dimer, in the presence of a phosphine ligand, for example tris(hydroxymethyl)phosphine (THMP), or tris(hydroxylpropyl)phosphine (THP or THPP). For those blocking groups containing an alkynyl group (e.g., an ethynyl), they may also be removed by a Pd(II) complex (e.g., Pd(OAc)₂ or allyl Pd(II) chloride dimer) in the presence of a phosphine ligand (e.g., THP or THMP).

### Palladium Cleavage Reagents

In some other embodiments, the 3' blocking group described herein such as AOM may be cleaved by a palladium catalyst. In some such embodiments, the Pd catalyst is water soluble. In some such embodiments, is a Pd(0) complex (e.g., Tris(3,3',3"-phosphinidynetris(benzenesulfonato)palladium(0) nonasodium salt nonahydrate). In some instances, the Pd(0) complex may be generated *in situ* from reduction of a Pd(II) complex by reagents such as alkenes, alcohols, amines, phosphines, or metal hydrides. Suitable palladium sources include Na₂PdCl₄, Pd(CH₃CN)₂Cl₂, (PdCl(C₃H₅))₂, [Pd(C₃H₅)(THP)]Cl, [Pd(C₃H₅)(THP)₂]Cl, Pd(OAc)₂, Pd(Ph₃)₄, Pd(dba)₂, Pd(Acac)₂, PdCl₂(COD), and Pd(TFA)₂. In one such embodiment, the Pd(0) complex is generated *in situ* from Na₂PdCl₄ or K₂PdCl₄. In another embodiment, the palladium source is allyl palladium(II) chloride dimer [(PdCl(C₃H₅))₂]. In some embodiments, the Pd(0) complex is generated in an aqueous solution by mixing a Pd(II) complex with a phosphine. Suitable phosphines include water soluble phosphines, such as THP, THMP, PTA, TCEP, bis(p-sulfonatophenyl)phenylphosphine dihydrate potassium salt, or triphenylphosphine-3,3',3"-trisulfonic acid trisodium salt.

In some embodiments, the Pd(0) is prepared by mixing a Pd(II) complex [(PdCl(C₃H₅))₂] with THP *in situ.* The molar ratio of the Pd(II) complex and the THP may be about 1:2, 1:3, 1:4, 1:5, 1.6, 1:7, 1:8, 1:9, or 1:10. In some further embodiments, one or more reducing agents may be added, such as ascorbic acid or a salt thereof (e.g., sodium ascorbate). In some embodiments, the cleavage mixture may contain additional buffer reagents, such as a primary amine, a secondary amine, a tertiary amine, a carbonate salt, a phosphate salt, or a borate salt, or combinations thereof. In some further embodiments, the buffer reagent comprises ethanolamine (EA), tris(hydroxymethyl)aminomethane (Tris), glycine, sodium carbonate, sodium phosphate, sodium borate, 2-dimethylethanolamine (DMEA), 2-diethylethanolamine (DEEA), N,N,N',N'-tetramethylethylenediamine(TEMED), or N,N,N',N'-tetraethylethylenediamine (TEEDA), or combinations thereof. In one embodiment, the buffer reagent is DEEA. In another embodiment, the buffer reagent contains one or more inorganic salts such as a carbonate salt, a phosphate salt, or a borate salt, or combinations thereof. In one embodiment, the inorganic salt is a sodium salt.

### Linkers

The dye compounds as disclosed herein may include a reactive linker group at one of the substituent positions for covalent attachment of the compound to a substrate or another molecule. Reactive linking groups are moieties capable of forming a bond (e.g., a covalent or noncovalent bond), in particular a covalent bond. In a particular embodiment the linker may be a cleavable linker. Use of the term "cleavable linker" is not meant to imply that the whole linker is required to be removed. The cleavage site can be located at a position on the linker that ensures that part of the linker remains attached to the dye and/or substrate moiety after cleavage. Cleavable linkers may be, by way of non-limiting example, electrophilically cleavable linkers, nucleophilically cleavable linkers, photocleavable linkers, cleavable under reductive conditions (for example disulfide or azide containing linkers), oxidative conditions, cleavable via use of safety-catch linkers and cleavable by elimination mechanisms. The use of a cleavable linker to attach the dye compound to a substrate moiety ensures that the label can, if required, be removed after detection, avoiding any interfering signal in downstream steps.

Useful linker groups may be found in PCT Publication No. WO2004/018493), examples of which include linkers that may be cleaved using water-soluble phosphines or water-soluble transition metal catalysts formed from a transition metal and at least partially water-soluble ligands. In aqueous solution the latter form at least partially water-soluble transition metal complexes. Such cleavable linkers can be used to connect bases of nucleotides to labels such as the dyes set forth herein.

Particular linkers include those disclosed in PCT Publication No. WO2004/018493 such as those that include moieties of the formulae 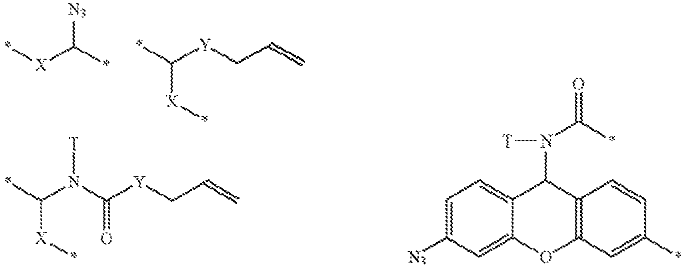 (wherein X is selected from the group comprising O, S, NH and NQ wherein Q is a C1-10 substituted or unsubstituted alkyl group, Y is selected from the group comprising O, S, NH and N(allyl), T is hydrogen or a C₁-C₁₀ substituted or unsubstituted alkyl group and * indicates where the moiety is connected to the remainder of the nucleotide or nucleoside). In some aspect, the linkers connect the bases of nucleotides to labels such as, for example, the dye compounds described herein.

Additional examples of linkers include those disclosed in U.S. Publication No. 2016/0040225 such as those include moieties of the formulae 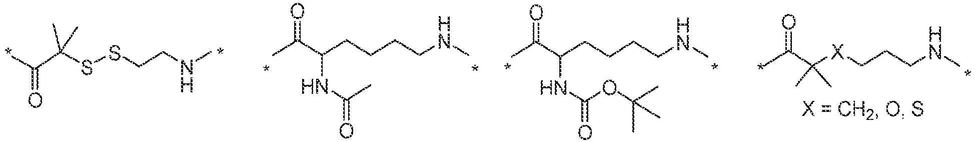 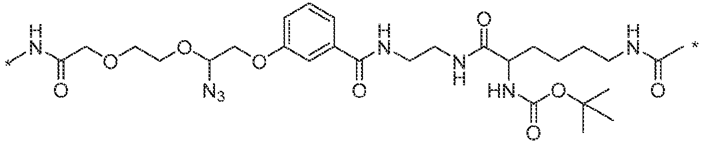 (wherein * indicates where the moiety is connected to the remainder of the nucleotide or nucleoside). The linker moieties illustrated herein may comprise the whole or partial linker structure between the nucleotides/nucleosides and the labels. The linker moieties illustrated herein may comprise the whole or partial linker structure between the nucleotides/nucleosides and the labels.

Additional examples of linkers include moieties of the formula: 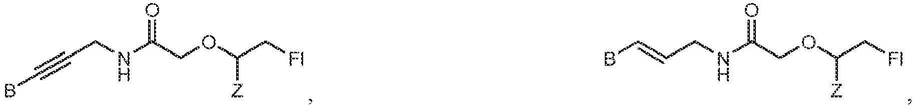 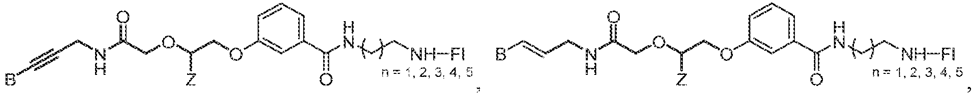 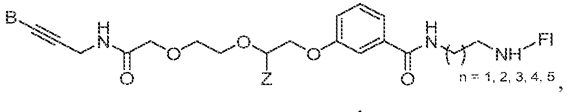 or 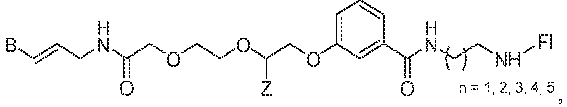 wherein B is a nucleobase; Z is -N₃ (azido), -O-C₁-C₆ alkyl, -O-C₂-C₆ alkenyl, or -O-C₂-C₆ alkynyl; and Fl comprises a dye moiety, which may contain additional linker structure. One of ordinary skill in the art understands that the dye compound described herein is covalently bounded to the linker by reacting a functional group of the dye compound (e.g., carboxyl) with a functional group of the linker (e.g., amino). In one embodiment, the cleavable linker comprises 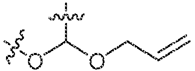 ("AOL" linker moiety) where Z is -O-allyl.

In particular embodiments, the length of the linker between a fluorescent dye (fluorophore) and a guanine base can be altered, for example, by introducing a polyethylene glycol spacer group, thereby increasing the fluorescence intensity compared to the same fluorophore attached to the guanine base through other linkages known in the art. Exemplary linkers and their properties are set forth in PCT Publication No. WO2007020457 The design of linkers, and especially their increased length, can allow improvements in the brightness of fluorophores attached to the guanine bases of guanosine nucleotides when incorporated into polynucleotides such as DNA. Thus, when the dye is for use in any method of analysis which requires detection of a fluorescent dye label attached to a guanine-containing nucleotide, it is advantageous if the linker comprises a spacer group of formula -((CH₂)₂O)ₙ-, wherein n is an integer between 2 and 50, as described in WO 2007/020457.

Nucleosides and nucleotides may be labeled at sites on the sugar or nucleobase. As known in the art, a "nucleotide" consists of a nitrogenous base, a sugar, and one or more phosphate groups. In RNA, the sugar is ribose and in DNA is a deoxyribose, i.e., a sugar lacking a hydroxy group that is present in ribose. The nitrogenous base is a derivative of purine or pyrimidine. The purines are adenine (A) and guanine (G), and the pyrimidines are cytosine (C) and thymine (T) or in the context of RNA, uracil (U). The C-1 atom of deoxyribose is bonded to N-1 of a pyrimidine or N-9 of a purine. A nucleotide is also a phosphate ester of a nucleoside, with esterification occurring on the hydroxy group attached to the C-3 or C-5 of the sugar. Nucleotides are usually mono, di- or triphosphates.

A "nucleoside" is structurally similar to a nucleotide but is missing the phosphate moieties. An example of a nucleoside analog would be one in which the label is linked to the base and there is no phosphate group attached to the sugar molecule.

Although the base is usually referred to as a purine or pyrimidine, the skilled person will appreciate that derivatives and analogues are available which do not alter the capability of the nucleotide or nucleoside to undergo Watson-Crick base pairing. "Derivative" or "analogue" means a compound or molecule whose core structure is the same as, or closely resembles that of a parent compound but which has a chemical or physical modification, such as, for example, a different or additional side group, which allows the derivative nucleotide or nucleoside to be linked to another molecule. For example, the base may be a deazapurine. In particular embodiments, the derivatives should be capable of undergoing Watson-Crick pairing. "Derivative" and "analogue" also include, for example, a synthetic nucleotide or nucleoside derivative having modified base moieties and/or modified sugar moieties. Such derivatives and analogues are discussed in, for example, Scheit, Nucleotide analogs (John Wiley & Son, 1980) and Uhlman et al., Chemical Reviews 90:543-584, 1990. Nucleotide analogues can also comprise modified phosphodiester linkages including phosphorothioate, phosphorodithioate, alkyl-phosphonate, phosphoranilidate, phosphoramidate linkages and the like.

A dye may be attached to any position on the nucleotide base, for example, through a linker. In particular embodiments, Watson-Crick base pairing can still be carried out for the resulting analog. Particular nucleobase labeling sites include the C5 position of a pyrimidine base or the C7 position of a 7-deaza purine base. As described above a linker group may be used to covalently attach a dye to the nucleoside or nucleotide.

In particular embodiments the labeled nucleotide or oligonucleotide may be enzymatically incorporable and enzymatically extendable. Accordingly, a linker moiety may be of sufficient length to connect the nucleotide to the compound such that the compound does not significantly interfere with the overall binding and recognition of the nucleotide by a nucleic acid replication enzyme. Thus, the linker can also comprise a spacer unit. The spacer distances, for example, the nucleotide base from a cleavage site or label.

Nucleosides or nucleotides labeled with the dyes described herein may have the formula: 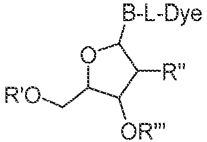 where Dye is a chromenoquinoline dye compound (label) moiety described herein (after covalent bonding between a functional group of the dye and a functional group of the linker "L"); B is a nucleobase, such as, for example uracil, thymine, cytosine, adenine, 7-deaza adenine, guanine, 7-deaza guanine, and the like; L is an optional linker which may or may not be present; R' can be H, or -OR' is monophosphate, diphosphate, triphosphate, thiophosphate, a phosphate ester analog, -O- attached to a reactive phosphorous containing group, or -O- protected by a blocking group; R" is H or OH; and R‴ is H, a 3' blocking group described herein, or -OR‴ forms a phosphoramidite. Where -OR‴ is phosphoramidite, R' is an acid-cleavable hydroxyl protecting group which allows subsequent monomer coupling under automated synthesis conditions. In some further embodiments, B comprises 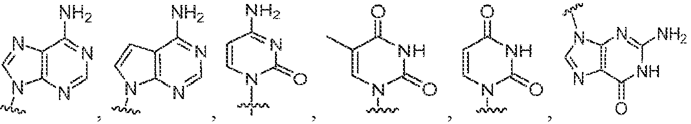 or 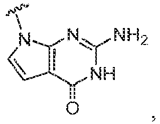 or optionally substituted derivatives and analogs thereof. In some further embodiments, the labeled nucleobase comprises the structure 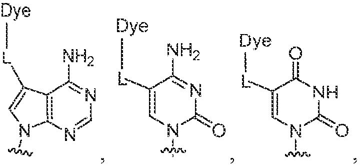 or

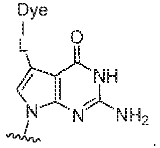

In a particular embodiment, the blocking group is separate and independent of the dye compound, i.e., not attached to it. Alternatively, the dye may comprise all or part of the 3'-OH blocking group. Thus R‴ can be a 3' OH blocking group which may or may not comprise the dye compound.

In yet another alternative embodiment, there is no blocking group on the 3' carbon of the pentose sugar and the dye (or dye and linker construct) attached to the base, for example, can be of a size or structure sufficient to act as a block to the incorporation of a further nucleotide. Thus, the block can be due to steric hindrance or can be due to a combination of size, charge and structure, whether or not the dye is attached to the 3' position of the sugar.

In still yet another alternative embodiment, the blocking group is present on the 2' or 4' carbon of the pentose sugar and can be of a size or structure sufficient to act as a block to the incorporation of a further nucleotide.

The use of a blocking group allows polymerization to be controlled, such as by stopping extension when a labeled nucleotide is incorporated. If the blocking effect is reversible, for example, by way of non-limiting example by changing chemical conditions or by removal of a chemical block, extension can be stopped at certain points and then allowed to continue.

In a particular embodiment, the linker (between dye and nucleotide) and blocking group are both present and are separate moieties. In particular embodiments, the linker and blocking group are both cleavable under the same or substantially similar conditions. Thus, deprotection and deblocking processes may be more efficient because only a single treatment will be required to remove both the dye compound and the blocking group. However, in some embodiments a linker and blocking group need not be cleavable under similar conditions, instead being individually cleavable under distinct conditions.

The disclosure also encompasses polynucleotides incorporating dye compounds. Such polynucleotides may be DNA or RNA comprised respectively of deoxyribonucleotides or ribonucleotides joined in phosphodiester linkage. Polynucleotides may comprise naturally occurring nucleotides, non-naturally occurring (or modified) nucleotides other than the labeled nucleotides described herein or any combination thereof, in combination with at least one modified nucleotide (e.g., labeled with a dye compound) as set forth herein. Polynucleotides according to the disclosure may also include non-natural backbone linkages and/or non-nucleotide chemical modifications. Chimeric structures comprised of mixtures of ribonucleotides and deoxyribonucleotides comprising at least one labeled nucleotide are also contemplated.

Non-limiting exemplary labeled nucleotides as described herein include: 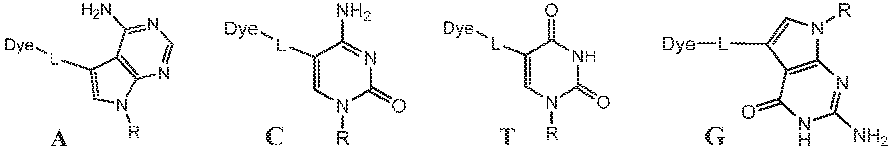 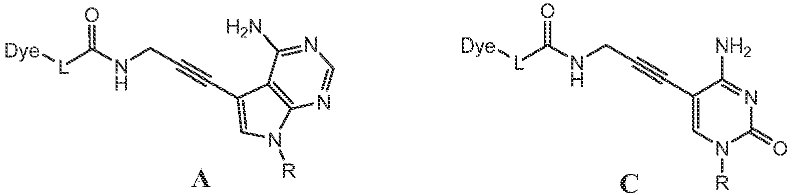 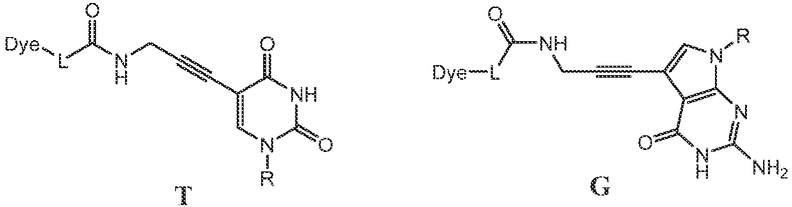 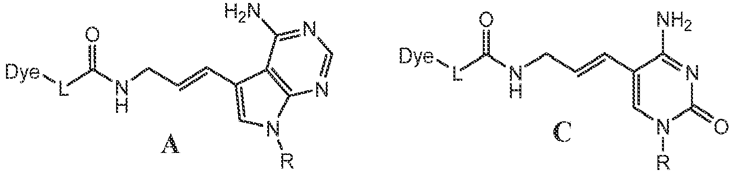 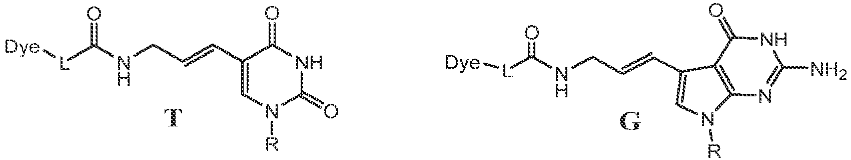 wherein L represents a linker and R represents a ribose or deoxyribose moiety as described above, or a ribose or deoxyribose moiety with the 5' position substituted with mono-, di- or triphosphates.

In some embodiments, non-limiting exemplary fluorescent dye conjugates are shown below: 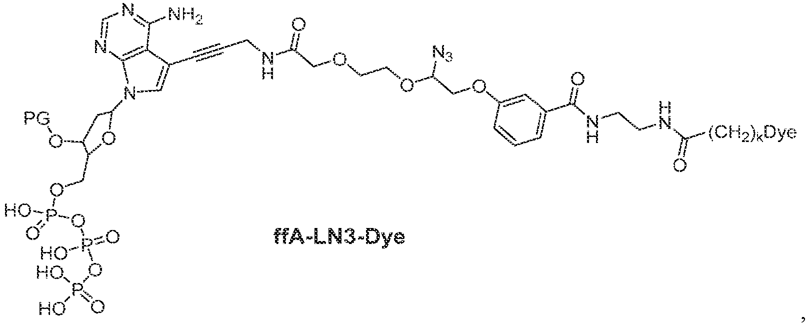 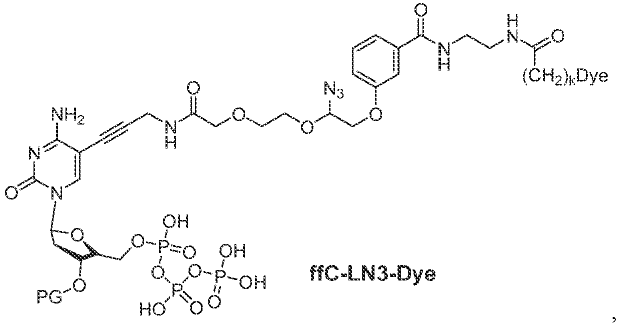 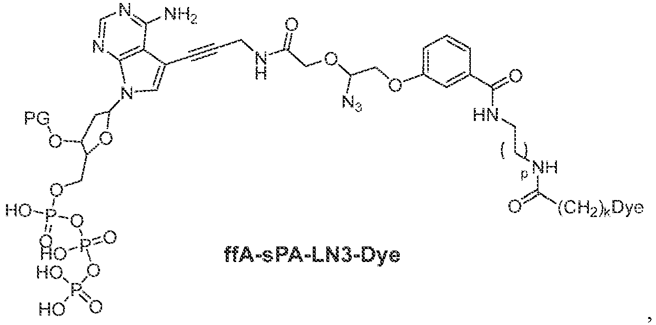 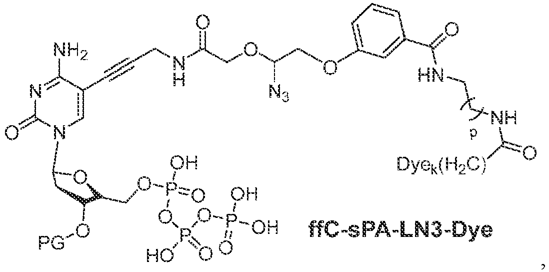 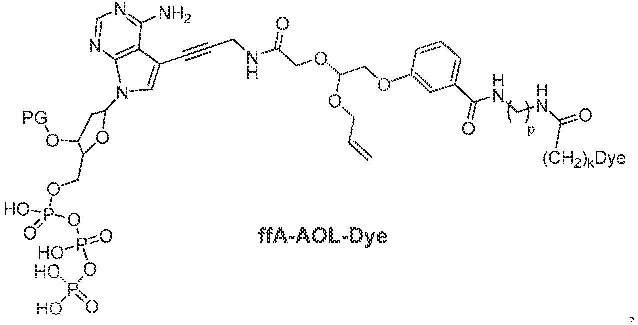 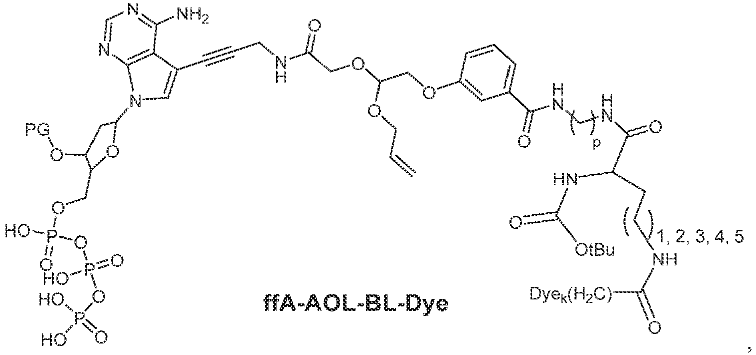 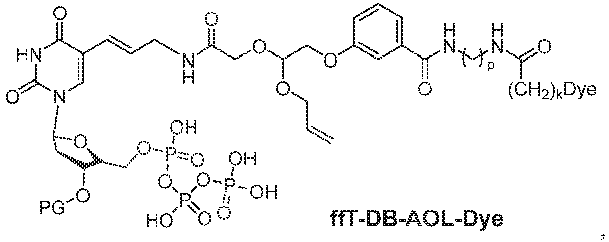 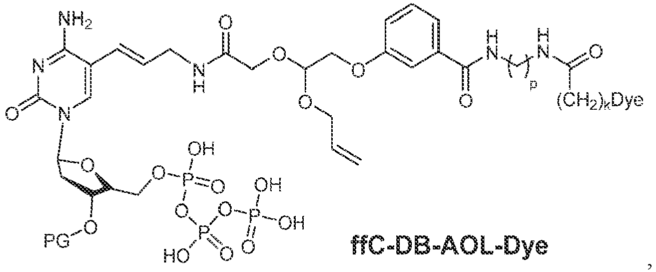 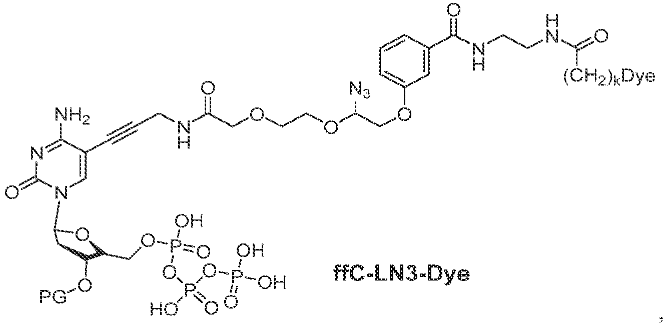 wherein PG stands for the 3' OH blocking groups described herein; p is an integer of 1, 2, 3, 4, 5, 6, 7, 8, 9, or 10; and k is 0, 1, 2, 3, 4, or 5. In one embodiment, -0-PG is AOM. In another embodiment, -0-PG is -O-azidomethyl. In one embodiment, k is 5. In some further embodiments, p is 1, 2 or 3; and k is 5. 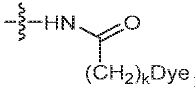 refers to the connection point of the Dye with the cleavable linker as a result of a reaction between an amino group of the linker moiety and the carboxyl group of the Dye. In any embodiments of the labeled nucleotide described herein, the nucleotide is a nucleotide triphosphate.

Additional aspects of the present disclosure relate to an oligonucleotide or polynucleotide comprising or incorporating a labeled nucleotide described herein. In some embodiments, the oligonucleotide or polynucleotide is hybridized to at least a portion of a target polynucleotide. In some embodiments, the target polynucleotide is immobilized on a solid support. In some further embodiments, the solid support comprises an array or a plurality of different immobilized target polynucleotides. In further embodiments, the solid support comprises a patterned flow cell. In further embodiments, the patterned flow cell comprises a plurality of nanowells. In further embodiments, the solid support comprises at least 5,000,000 spatially distinguishable sites/cm² that comprise multiple copies of target polynucleotides.

### EXAMPLES

Additional embodiments are disclosed in further detail in the following examples, which are not in any way intended to limit the scope of the claims.

### Example 1. Synthesis of chromenoquinoline dyes of Formula (I)

### Synthesis of precursors

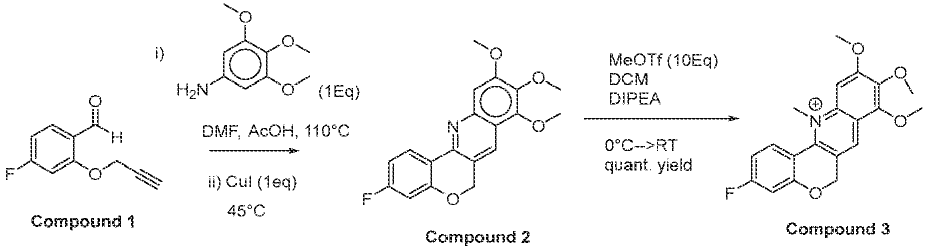

Compound 1 (0.5g, 2.81mmol), 3,4,5-trimethoxyaniline (0.514g, 2.81mmol) and DMF (5ml) were added to a 50ml round-bottomed flask fitted with stir bar, condenser and nitrogen line. Acetic acid (82µl, 1.40mmol) was added and the flask was heated at 810°C (hotplate temperature) for four hours. The reaction was cooled to 45°C and copper iodide (0.534g, 2.81mmol) was added as a solid. The reaction was stirred at 45°C overnight. The reaction was cooled to RT, filtered through a sintered glass funnel and purified by normal phase column chromatography to afford Compound 2. Yield: 0.47mmol, 17% yield. LC-MS (ESI): (positive ion) m/z 1042 (M+H⁺). 1H NMR (300 MHz, CDCl₃) δ 8.44 (s, 1H), 8.09 (s, 1H), 7.34 (s, 1H), 6.86 (td, J = 8.5, 2.5 Hz, 1H), 6.73 (dd, J = 9.7, 2.5 Hz, 1H), 5.36 (d, J = 1.0 Hz, 2H), 4.08 (s, 3H), 4.04 (s, 3H), 3.99 (s, 3H).

Compound 2 (160mg, 0.47mmol) was placed in a 25ml 1-neck round-bottomed flask with anhydrous DCM (5ml) and a stirbar. The reaction was cooled to 0°C under nitrogen using an ice bath.Methyl triflate (MeOTf, 530µl, 4.7mmol) was added dropwise *via* needle and syringe. DIPEA (180µl, 1mmol) was added while the reaction was stirred at 0°C. After addition the reaction was allowed to warm to RT. The reaction was stirred for four hours at RT. The reaction mixture was mixed with approx. 100ml DCM and washed with 0.5% triethylamine solution in water (100ml), brine (100ml), and water (100ml). The aqueous fractions were re-extracted with DCM. The combined DCM fractions were dried over MgSO₄, filtered, and dried *in vacuo.* Purification by normal phase column chromatography afforded Compound 3. Quantitative yield (167mg). 1H NMR (300 MHz, DMSO-d6) δ 9.05 (s, 1H), 8.23 (dd, J = 8.9, 6.3 Hz, 1H), 7.52 (s, 1H), 7.38 - 7.26 (m, 2H), 5.46 (s, 2H), 4.54 (s, 3H), 4.20 (s, 3H), 4.12 (s, 3H), 3.98 (s, 3H).

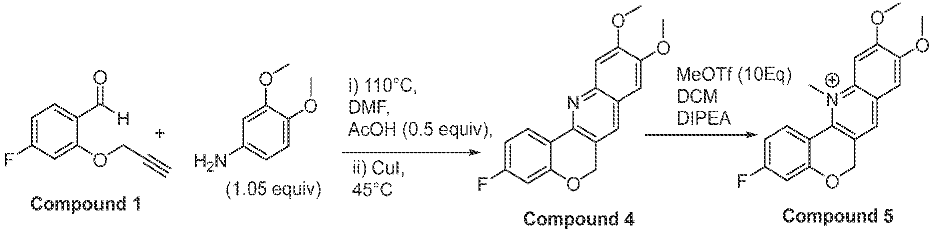

Compound 1 (1g, 5.61mmol) and 3,4-dimethoxyaniline (0.903g, 5.89mmol) were mixed with DMF (10ml) and acetic acid (162µl, 2.81mmol) in a 100ml 1-neck RBF fitted with stirbar and nitrogen line. The reaction was heated at 810°C for two hours. The reaction was cooled to 45°C and copper iodide (0.534g, 2.81mmol) was added as a solid. The reaction was stirred at 45°C overnight. The reaction was then cooled to RT and filtered through a sintered glass funnel. The filtrate was diluted with DCM (200ml) and ammonium chloride (pH10, 200ml) was added. Separated the DCM layer and dried over MgSO4 before drying *in vacuo.* The dried material purified by normal phase column chromatography to afford Compound 4. Yield: 420mg, 24% yield. ¹H NMR (300 MHz, DMSO) δ 8.32 (dd, J = 8.7, 6.8 Hz, 1H), 8.01 (s, 1H), 7.40 (s, 1H), 7.32 (s, 1H), 7.00 (td, J = 8.7, 2.6 Hz, 1H), 6.93 (dd, J = 10.2, 2.6 Hz, 1H), 5.43 (s, 2H), 3.93 (d, J = 15.1 Hz, 6H). Compound 5 was prepared following similar procedure described in the synthesis of Compound 3.

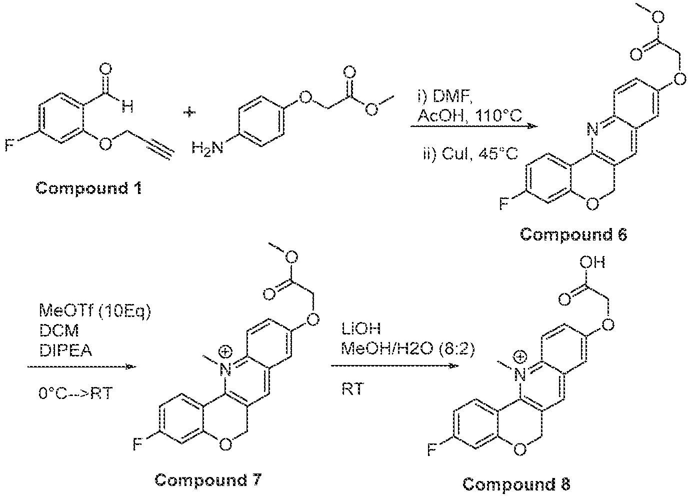

Compound 6 was prepared following similar procedure as described in the synthesis of Compound 2. Compound 7 was prepared following similar procedure as described in the synthesis of Compound 3. Lithium hydroxide (25mg, 1060µmol) was dissolved in a mixture of MeOH/H₂O (8:2, 4ml). The LiOH solution was added to a solution of Compound 6 (75mg, 212µmol) in MeOH/H₂O (1ml) in a 1-neck round-bottomed flask. Stirred at RT until there was no starting material remaining. Purified the crude aqueous mixture by reverse-phase afforded Compound 8. Yield: 15µmol (7% yield). ¹H NMR (300 MHz, DMSO) δ 8.87 (d, J = 6.3 Hz, 2H), 8.41 (d, J = 9.7 Hz, 1H), 8.35 (d, J = 9.7 Hz, 1H), 8.26 (dd, J = 9.0, 6.3 Hz, 1H), 7.73 (ddd, J = 21.0, 9.6, 2.9 Hz, 2H), 7.61 (d, J = 2.9 Hz, 1H), 7.51 (d, J = 2.9 Hz, 1H), 7.37 - 7.22 (m, 3H), 6.98 - 6.87 (m, 1H), 6.66 (td, J = 8.5, 2.5 Hz, 1H), 5.44 (s, 2H), 4.57 (s, 3H), 4.39 (m, 2H), 4.19 (s, 3H).

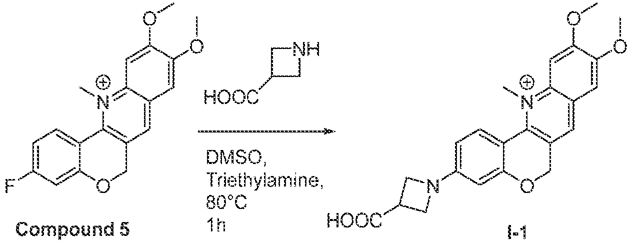

Compound 5 (98mg, 300µmol) and azetidine-3-carboxylic acid (152mg, 1.5mmol) were placed in a 25ml 1-neck round-bottomed flask fitted with stirbar and nitrogen line. DMSO (5ml) and triethylamine (20µl) were added to the flask and the mixture was heated with stirring at 80°C for one hour. A bright orange solid precipitate formed in the reaction mixture. The reaction mixture was diluted with acetonitrile (5ml) before filtering off the precipitate using a sintered glass funnel. The filtered material was washed with water (5mlx5) before being dried under high vacuum overnight to afford I-1. LC-MS (ESI): (positive ion) m/z 308 (M+H⁺). ¹H NMR (300 MHz, TFA-d) δ 8.74 (s, 1H), 8.35 (d, J = 8.0 Hz, 1H), 7.74 (d, J = 8.6 Hz, 2H), 7.66 (s, 1H), 5.52 (s, 2H), 5.30 (dt, J = 32.8, 10.5 Hz, 4H), 4.81 (s, 3H), 4.34 (d, J = 32.6 Hz, 7H). Yield: 207µmol (69%).

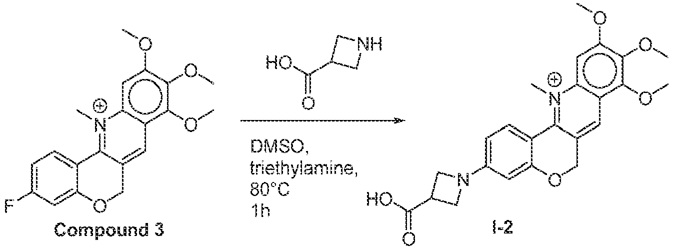

Compound 3 (71mg, 200µmol) and azetidine-3-carboxylic acid (101mg, 1.0mmol) were placed in a 25ml 1-neck round-bottomed flask fitted with stirbar and nitrogen line. DMSO (5ml) and triethylamine (20µl) were added to the flask and the mixture was heated with stirring at 80°C for one hour to afford I-2 and purified by prep-HPLC. LC-MS (ESI): (positive ion) m/z 338 (M+H⁺). ¹H NMR (300 MHz, TFA-d) δ 9.07 (s, 1H), 8.31 (d, J = 8.3 Hz, 1H), 7.69 (d, J = 9.2 Hz, 1H), 7.42 (s, 1H), 5.49 (s, 2H), 5.25 (dt, J = 32.9, 10.2 Hz, 4H), 4.72 (s, 3H), 4.45 - 4.18 (m, 9H). Yield: 134µmol (67%).

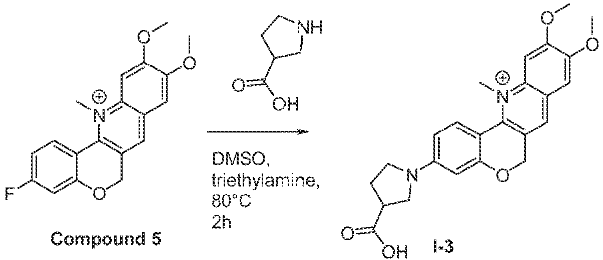

Compound 5 (12mg, 36µmol) and pyrrolidine-3-carboxylic acid (21mg, 184µmol) were placed in a 10ml 1-neck round-bottomed flask fitted with stirbar and nitrogen line. DMSO (1ml) and triethylamine (10µl) were added to the flask and the mixture was heated with stirring at 80°C for two hours to afford 1-3, which was purified by prep-HPLC. LC-MS (ESI): (positive ion) m/z 322 (M+H⁺). Yield: 36µmol (quantitative).

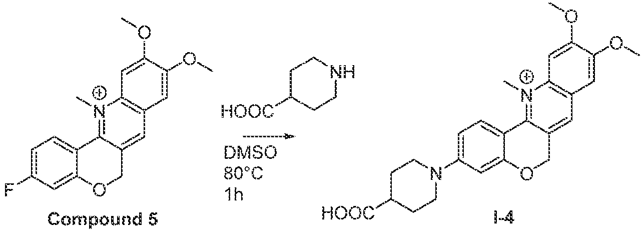

Compound 5 (16mg, 50µmol) and isonipecotic acid (32mg, 250µmol) were placed in a 10ml 1-neck round-bottomed flask fitted with stirbar and nitrogen line. DMSO (1ml) and triethylamine (10µl) were added to the flask and the mixture was heated with stirring at 80°C for one hour to afford I-4 which was purified by prep-HPLC. LC-MS (ESI): (positive ion) m/z 336 (M+H⁺). Yield: 22.3µmol (45%).

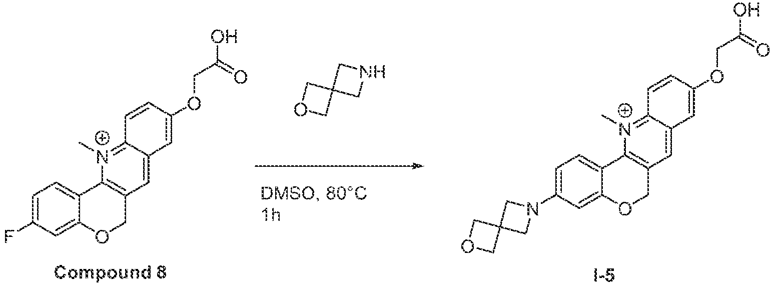

Compound 8 (20mg, 59µmol) and 2-oxa-6-azaspiro[3.3]heptane (29mg, 294µmol) were placed in a 10ml 1-neck round-bottomed flask fitted with stirbar and nitrogen line. DMSO (1ml) and triethylamine (10µl) were added to the flask and the mixture was heated with stirring at 80°C for one hour to afford I-5, which was purified by prep-HPLC. LC-MS (ESI): (positive ion) m/z 320 (M+H⁺).
Yield: 13.8µmol (23%).

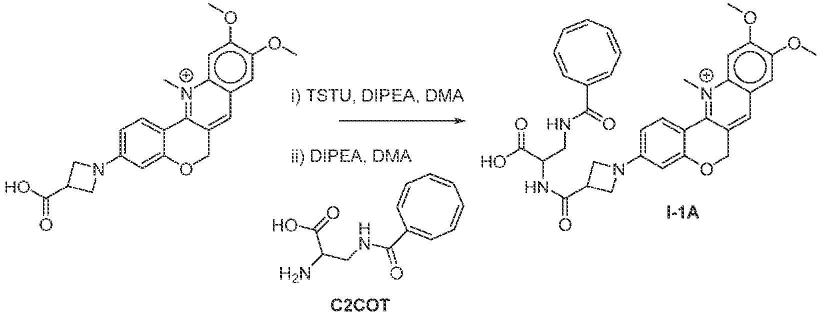

1-1 (28mg, 68µmol), TSTU (31mg, 102µmol) and DMA (5ml) were placed in a 50ml 1-neck round-bottomed flask fitted with nitrogen line and stirbar. DIPEA (35µl, 204µmol) was added, and the reaction was stirred at RT for 30mins. Once activation was confirmed by UPLC and LCMS, C2COT (24mg, 105µmol) was added to the reaction mixture as a solid. The reaction was left stirring overnight at RT to afford I-1A, which was purified by prep-HPLC. LC-MS (ESI): (positive ion) m/z 624 (M+H⁺). 1H NMR (300 MHz, TFA-d) δ 8.72 (s, 1H), 8.31 (d, J = 8.4 Hz, 1H), 7.79 - 7.68 (m, 2H), 7.63 (s, 1H), 7.10 (s, 1H), 6.28 (d, J = 11.3 Hz, 1H), 6.04 (s, 2H), 5.95 (s, 3H), 5.49 (s, 2H), 5.24 (t, J = 13.0 Hz, 3H), 5.18 (s, 1H), 5.11 (s, 1H), 4.78 (s, 3H), 4.35 (s, 3H), 4.27 (s, 3H), 4.16 (s, 2H), 1.49 (d, J = 7.6 Hz, 1H). Yield: 51µmol (75%).

### Example 2. General synthesis of chromenoquinoline dyes labeled nucleotides and spectral properties

The chromenoquinoline dye of Formula (I) (0.020 mmol) was coevaporated with 2x 2 mL of anhydrous N,N'-dimethylformamide (DMF), then dissolved in 2 mL of anhydrous *N,N'-*dimethylacetamide (DMA). *N*,*N*-diisopropylethylamine (28.4 µL, 0.863 mmol) was added, followed by *N*,*N*,*N*',*N*'-tetramethyl-*O*-(*N*-succinimidyl)uronium tetrafluoroborate as 0.1M solution in anhydrous DMA (TSTU, 932 µL, 0.023 mmol). The reaction was stirred under nitrogen at RT for 30 minutes. In the meantime, an aqueous solution of the 2'-deoxyadenosine triphosphates-linker (0.01 mmol) was evaporated to dryness under reduced pressure and resuspended in 900 µL of 0.1 M triethylammonium bicarbonate (TEAB) solution in water. The activated chromenoquinoline dye solution was added to the triphosphate and the reaction was stirred at RT for 18 hours. The crude product was purified firstly by ion-exchange chromatography on DEAE-Sephadex A25 (25 g). The fractions containing the triphosphate were pooled and the solvent was evaporated to dryness under reduced pressure.

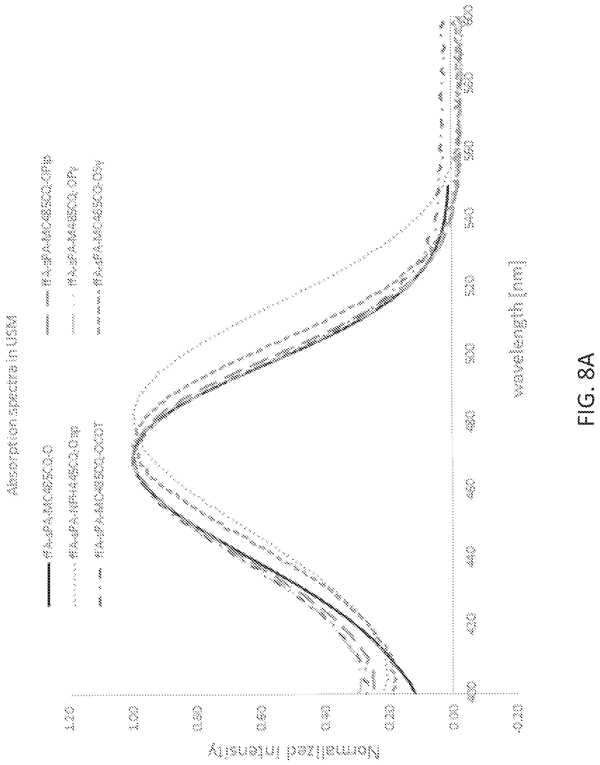

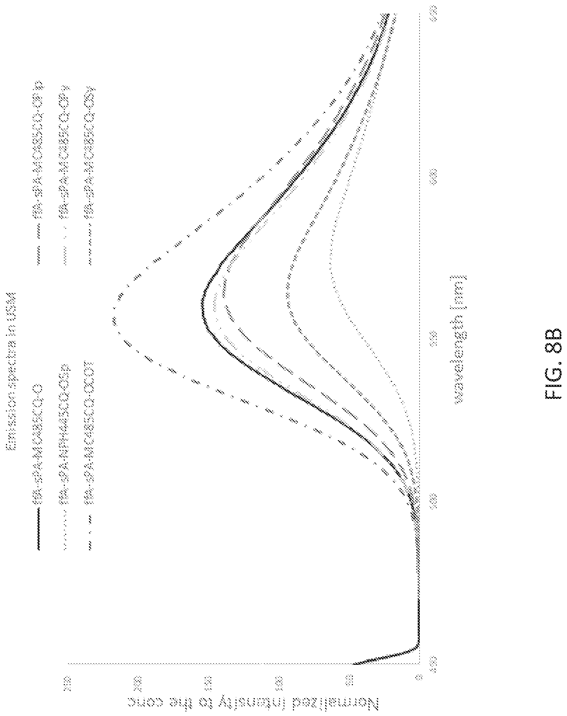

The spectral properties of several fully functionalized A nucleotides (ffAs) conjugated with the chromenoquinoline dyes described herein were characterized. FIG. 8A illustrates the fluorescent absorption spectra of ffA nucleotides conjugated with chromenoquinoline dyes I-1 through I-5 and I-1A as a 2 µM solution in Universal Scan Mix (USM, 1 M Tris pH 7.5, 0.05% TWEEN, 20 mM sodium ascorbate, 10 mM ethyl gallate). FIG. 8B show the fluorescence emission spectra of ffA nucleotide conjugated with chromenoquinoline dyes I-1 through I-5 and I-1A acquired using 350 nm as excitation wavelength in USM The spectra were acquired on an Agilent Cary 800 UV-Vis Spectrophotometer and on a Cary Eclipse Fluorescence Spectrophotometer, using quartz or plastic cuvettes. It was observed that the ffAs labeled with the chromenoquinoline dyes I-1 through I-5 and I-1A all had long Stokes shift of over 800 nm.

### Example 3. Sequencing experiments on Illumina MiSeq^{®} instrument

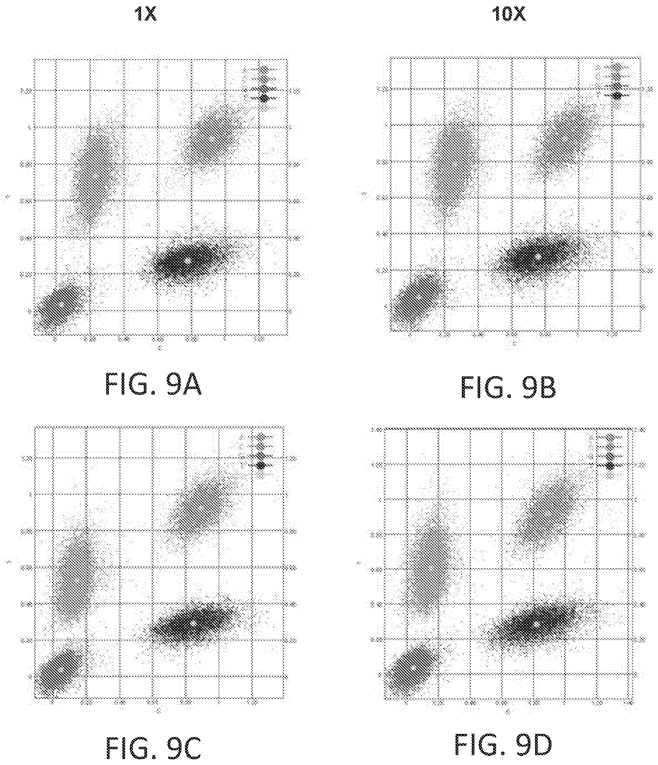

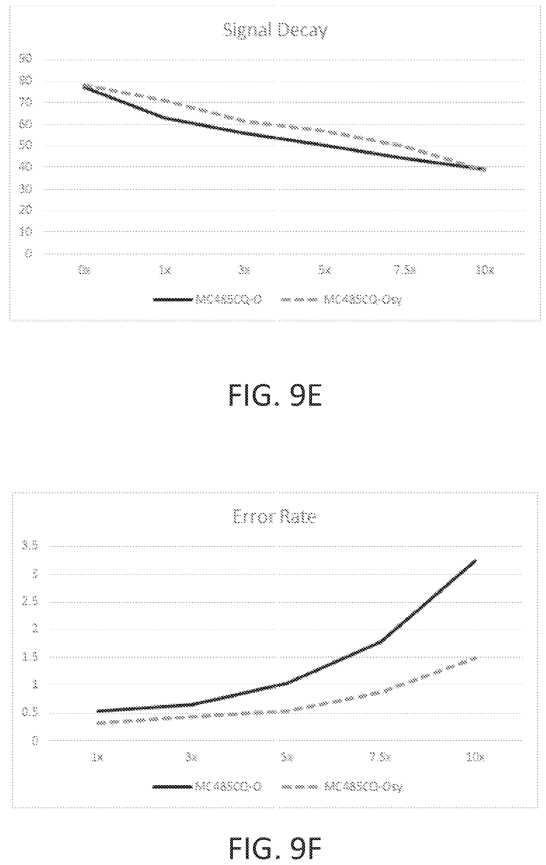

The ffAs labeled with the chromenoquinoline dyes described herein were tested on an Illumina MiSeq^{®} instrument under the 1Ex-2Ch mode, which had been set up to take images with a blue excitation light (~ 350 nm). Images were taken simultaneously through collection channels which are in blue (372-520nm) and green (540-640nm).The incorporation mix used in each of these experiments contained the following five ffNs; an ffA labeled with a chromenoquinoline dye of Formula (1), an ffC excitable with blue light at 350 nm (for example ffC-linker-coumarin dye A), an ffT excitable with blue light at 450nm (e.g., ffT-linker-coumarin dye B), an ffT labeled with a known green dye (AF550POPOS0), and an unlabeled ffG (dark ffG) in 50 mM glycine buffer, pH 9.6, 50 mM NaCl, 1 mM EDTA, 0.2% CHAPS, 4 mM MgSO₄ and a DNA polymerase. FIG. 9A and 9B were scatterplots obtained at cycle 26 when the blue laser dosage was at 1X and 10X respectively (longer exposure time was used to increase blue laser dosage). The incorporation mix included the following ffNs: ffA-sPA-I-1 (2 µM), ffC-sPA-coumarin dye A (2 µM), ffT-LN3-coumarin dye B (0.7 µM), ffT-LN3-AF550POPOSO (1.3 µM) and dark G (2 µM). FIG. 9C and 9D were scatterplots obtained at cycle 26 when the blue laser dosage was at 1X and 10X respectively. The incorporation mix included the following ffNs: ffA-sPA-I-2 (2 µM), ffC-sPA-coumarin dye A (2 µM), ffT-LN3-coumarin dye B (0.7 µM), fff-LN3-AF550POPOSO (1.7 µM), and dark G (2 µM). FIGs. 9E and 9F demonstrate the signal decay and error rate of the same sequencing run at 851 cycles using the ffN sets with either ffA-sPA-I-1 or ffA-sPA-I-2 respectively, where different dosages of blue laser at 1x, 3x, 5x, 7.5x and 10x were assigned to different areas of the same flow cell.

Coumarin dye A is disclosed in U.S. Publication No. 2018/0094140, having the structure moiety 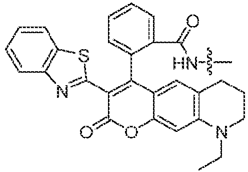 when conjugated with the ffC. Coumarin dye B is disclosed in U.S. Publication No. 2020/0277670 A1, having the structure moiety 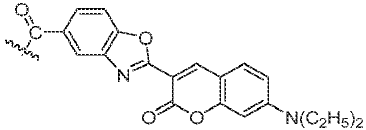 when conjugated with the ffC. AF550POPOS0 is disclosed in U.S. Publication No. 2018/0282791 A1, having the structure moiety 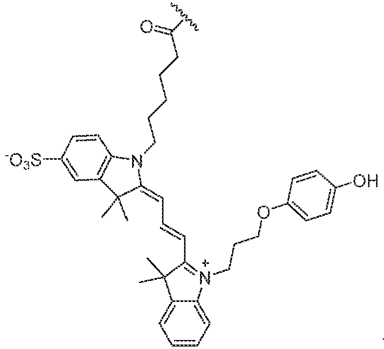 when conjugated with the ffT.

FIGs. 9A-9D indicated the ffN set with the chromenoquinoline dye labeled ffAs provided excellent quality scatterplots with good cloud separation even when the blue laser dosage was increased to 10x. FIGs. 9E and 9F also demonstrated excellent sequencing metrics with slight increase in signal decay and error rate when blue laser dosage was increased from 1x to 10x. The phasing values were 0.077 to 0.848 for using ffN set with ffA-sPA-I-1 and 0.070 to 0.825 for using ffN set with ffA-sPA-I-2. The average prephasing values, which was not light dependent, were 0.25 and 0.42 respectively.

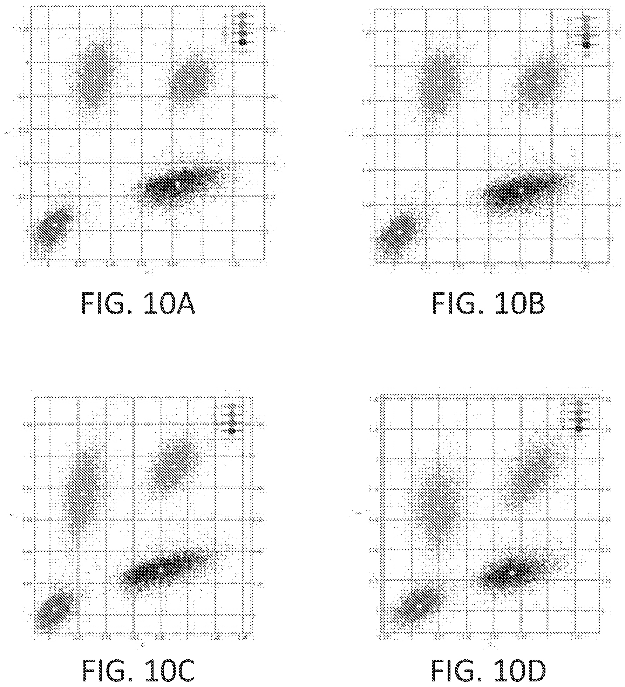

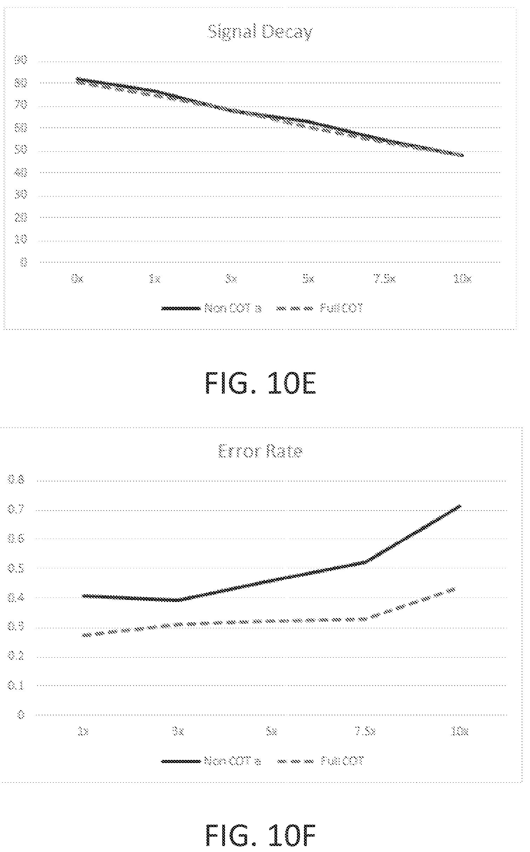

FIG. 10A and 10B were scatterplots obtained at cycle 26 when the blue laser dosage was at 1x and 10x respectively. The incorporation mix included the following full COT ffNs: ffA-sPA-I-1A (2 µM), ffC-sPA-coumarin dye A-COT (2 µM), ffT-LN3-coumarin dye B-COT (0.7 µM), ffT-LN3-AF550POPOSO (1.3 µM) and dark G (2 µM). FIG. 10C and 10D were scatterplots obtained at cycle 26 when the blue laser dosage was at 1x and 10x respectively. The incorporation mix included the following ffNs: ffA-sPA-I-1 (2 µM), ffC-sPA-coumarin dye A-COT (2 µM), ffT-LN3-coumarin dye B-COT (0.7 µM), ffT-LN3-AF550POPOSO (1.7 µM), and dark G (2 µM). FIGs. 10E and 10F demonstrate the signal decay and error rate of the same sequencing run at cycle 851 cycle using the ffN sets with either ffA-sPA-I-1A or ffA-sPA-I-1 respectively, where different dosages of blue laser at 1x, 3x, 5x, 7.5x and 10x were assigned to different areas of the same flow cell.

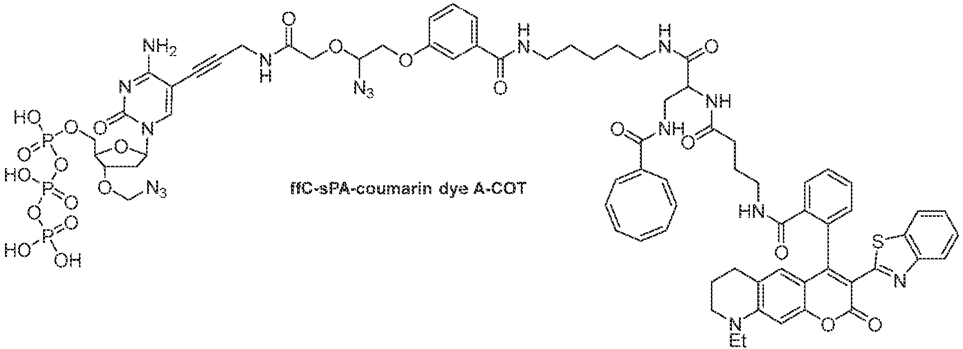

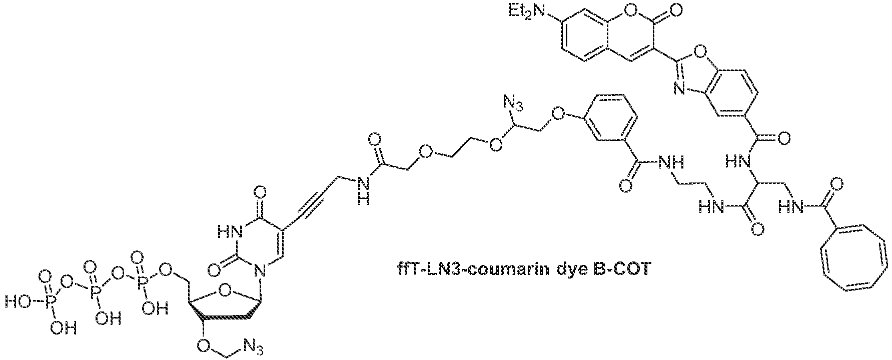

The sequencing metrics shown in FIGs. 10E and 10F suggest that a better error rate was achieved when COT protected dye I-1A was used to label ffA, as compared to the ffN set with ffA labeled with I-1 when the blue laser dosage was increased from 1x to 10x. However, the signal decay was not substantially distinguishable. The phasing values were 0.087 to 0.847 for using ffN set with ffA-sPA-I-1A and 0.094 to 0.851 for using ffN set with ffA-sPA-I-1. The average prephasing values, which was not light dependent, were 0.07 and 0.098 respectively.

## Claims

1. A method of sequencing clusters of labeled polynucleotides bound to a flowcell, comprising:
exciting the clusters at a first excitation wavelength and detecting fluorescent emissions at a first detection wavelength from a first labeled nucleotide,
exciting the clusters at a second excitation wavelength and detecting fluorescent emissions at a second detection wavelength from a second labeled nucleotide,exciting the clusters at the first excitation wavelength and detecting fluorescent emissions at the second detection wavelength from a third labeled nucleotide,
determining clusters that did not emit fluorescent emissions after excitation at the first or second excitation wavelengths to detect the presence of a fourth unlabeled nucleotide, and
determining the nucleotide sequence of the polynucleotides based on the detected fluorescent emissions;
wherein the first excitation wavelength and the second excitation wavelength are different wavelengths; and wherein the first detection wavelength and the second detection wavelength are different wavelengths.

2. The method of claim 1, wherein the first labeled nucleotide, the second labeled nucleotide, and the third labeled nucleotide are each labeled with a single fluorescent dye.

3. The method of any of claims 1 or 2, wherein the third labeled nucleotide is labeled with a long Stokes shift dye, wherein the long Stokes shift dye has a Stokes shift ranging from about 300 meV to 850 meV.

4. The method of any of claims 1 or 2, wherein the third labeled nucleotide is labeled with a long Stokes shift dye, wherein the long Stokes shift dye has an emission shifted by about 50 nm to 150 nm.

5. The method of claim 4, wherein the long Stokes shift dye is excited at about 300 nm.

6. The method of any of claims 1 or 2, wherein the third labeled nucleotide is labeled with a chromenoquinoline dye.

7. The method of any of claims 1 or 2, wherein the first labeled nucleotide is labeled with dye NR455BoC.

8. The method of any of claims 1 or 2, wherein the second labeled nucleotide is labeled with dye NR550S0.

9. The method of any of claims 1 or 2, wherein the third labeled nucleotide is labeled with dye MC485CQ-O.

10. The method of any of claims 1 to 9, wherein the first detection wavelength is imaged by a first charge coupled device, and the second detection wavelength is imaged by a second charge coupled device.

11. The method of any of claims 1 or 2, wherein exciting at the first excitation wavelength and detecting fluorescent emissions at the first emission wavelength is performed separately from exciting at the first excitation wavelength and detecting fluorescent emissions at the second emission wavelength.

12. The method of any of claims 1 or 2, wherein exciting at the first excitation wavelength and detecting fluorescent emissions at the first emission wavelength is performed at the same time as exciting at the first excitation wavelength and detecting fluorescent emissions at the second emission wavelength.

13. The method of any of claims 1 or 2, wherein the detecting fluorescent emissions from the first labeled nucleotide, the second labeled nucleotide and the third labeled nucleotide are performed using a Time Delay Integration (TDI) imaging method.

14. A system for sequencing polynucleotides bound to a flowcell, comprising:
a machine-readable memory, and
a processor configured to execute machine-readable instructions, which, when executed by the processor, cause the system to perform steps according to any of claims 1 to 13.

15. A non-transitory computer-readable medium storing a polynucleotide sequencing program including instructions that, when executed by a processor, causes a polynucleotide sequencing apparatus, to perform steps according to any of claims 1 to13.

## Patentansprüche

1. Verfahren zum Sequenzieren von Clustern markierter Polynukleotide, die an eine Durchflusszelle gebunden sind, umfassend:
Anregen der Cluster auf einer ersten Anregungswellenlänge und Nachweisen von Fluoreszenzemissionen auf einer ersten Nachweiswellenlänge von einem ersten markierten Nukleotid,
Anregen der Cluster auf einer zweiten Anregungswellenlänge und Nachweisen von Fluoreszenzemissionen auf einer zweiten Nachweiswellenlänge von einem zweiten markierten Nukleotid, Anregen der Cluster auf der ersten Anregungswellenlänge und Nachweisen von Fluoreszenzemissionen auf der zweiten Nachweiswellenlänge von einem dritten markierten Nukleotid,
Bestimmen von Clustern, die nach Anregung auf der ersten oder zweiten Anregungswellenlänge keine Fluoreszenzemissionen emittiert haben, um die Anwesenheit eines vierten unmarkierten Nukleotids nachzuweisen, und
Bestimmen der Nukleotidsequenz der Polynukleotide auf der Grundlage der nachgewiesenen Fluoreszenzemissionen;
wobei die erste Anregungswellenlänge und die zweite Anregungswellenlänge unterschiedliche Wellenlängen sind; und wobei die erste Nachweiswellenlänge und die zweite Nachweiswellenlänge unterschiedliche Wellenlängen sind.

2. Verfahren nach Anspruch 1, wobei das erste markierte Nukleotid, das zweite markierte Nukleotid und das dritte markierte Nukleotid jeweils mit einem einzigen Fluoreszenzfarbstoff markiert sind.

3. Verfahren nach einem der Ansprüche 1 oder 2, wobei das dritte markierte Nukleotid mit einem Farbstoff mit langer Stokes-Verschiebung markiert ist, wobei der Farbstoff mit langer Stokes-Verschiebung eine Stokes-Verschiebung im Bereich von etwa 300 meV bis 850 meV aufweist.

4. Verfahren nach einem der Ansprüche 1 oder 2, wobei das dritte markierte Nukleotid mit einem Farbstoff mit langer Stokes-Verschiebung markiert ist, wobei der Farbstoff mit langer Stokes-Verschiebung eine um etwa 50 nm bis 150 nm verschobene Emission aufweist.

5. Verfahren nach Anspruch 4, wobei der Farbstoff mit langer Stokes-Verschiebung bei etwa 300 nm angeregt wird.

6. Verfahren nach einem der Ansprüche 1 oder 2, wobei das dritte markierte Nukleotid mit einem Chromenochinolin-Farbstoff markiert ist.

7. Verfahren nach einem der Ansprüche 1 oder 2, wobei das erste markierte Nukleotid mit dem Farbstoff NR455BoC markiert ist.

8. Verfahren nach einem der Ansprüche 1 oder 2, wobei das zweite markierte Nukleotid mit dem Farbstoff NR550S0 markiert ist.

9. Verfahren nach einem der Ansprüche 1 oder 2, wobei das dritte markierte Nukleotid mit dem Farbstoff MC485CO-O markiert ist.

10. Verfahren nach einem der Ansprüche 1 bis 9, wobei die erste Nachweiswellenlänge durch eine erste ladungsgekoppelte Vorrichtung abgebildet wird und die zweite Nachweiswellenlänge durch eine zweite ladungsgekoppelte Vorrichtung abgebildet wird.

11. Verfahren nach einem der Ansprüche 1 oder 2, wobei das Anregen auf der ersten Anregungswellenlänge und das Nachweisen von Fluoreszenzemissionen auf der ersten Emissionswellenlänge getrennt vom Anregen auf der ersten Anregungswellenlänge und dem Nachweisen von Fluoreszenzemissionen auf der zweiten Emissionswellenlänge durchgeführt wird.

12. Verfahren nach einem der Ansprüche 1 oder 2, wobei das Anregen auf der ersten Anregungswellenlänge und das Nachweisen von Fluoreszenzemissionen auf der ersten Emissionswellenlänge zur gleichen Zeit wie das Anregen auf der ersten Anregungswellenlänge und das Nachweisen von Fluoreszenzemissionen auf der zweiten Emissionswellenlänge durchgeführt wird.

13. Verfahren nach einem der Ansprüche 1 oder 2, wobei das Nachweisen von Fluoreszenzemissionen aus dem ersten markierten Nukleotid, dem zweiten markierten Nukleotid und dem dritten markierten Nukleotid unter Verwendung eines Time Delay Integration (TDI)-Bildgebungsverfahrens durchgeführt wird.

14. System zum Sequenzieren von Polynukleotiden, die an eine Durchflusszelle gebunden sind, umfassend:
einen maschinenlesbaren Speicher, und
einen Prozessor, der konfiguriert ist, um maschinenlesbare Anweisungen auszuführen, die, wenn sie von dem Prozessor ausgeführt werden, das System veranlassen, Schritte nach einem der Ansprüche 1 bis 13 durchzuführen.

15. Nicht-transitorisches computerlesbares Medium, das ein Polynukleotid-Sequenzierungsprogramm speichert, das Anweisungen beinhaltet, die, wenn sie von einem Prozessor ausgeführt werden, eine Polynukleotid-Sequenzierungseinrichtung veranlassen, Schritte nach einem der Ansprüche 1 bis 13 durchzuführen.

## Revendications

1. Procédé de séquençage de groupes de polynucléotides marqués liés à une cellule de flux, comprenant :
l'excitation des groupes à une première longueur d'onde d'excitation et la détection d'émissions fluorescentes à une première longueur d'onde de détection à partir d'un premier nucléotide marqué,
l'excitation des groupes à une seconde longueur d'onde d'excitation et la détection d'émissions fluorescentes à une seconde longueur d'onde de détection à partir d'un deuxième nucléotide marqué, l'excitation des groupes à la première longueur d'onde d'excitation et la détection d'émissions fluorescentes à la seconde longueur d'onde de détection à partir d'un troisième nucléotide marqué,
la détermination de groupes qui n'émettaient pas d'émissions fluorescentes après excitation aux première ou seconde longueurs d'onde d'excitation afin de détecter la présence d'un quatrième nucléotide non marqué, et
la détermination de la séquence nucléotidique des polynucléotides sur la base des émissions fluorescentes détectées ;
dans lequel la première longueur d'onde d'excitation et la seconde longueur d'onde d'excitation sont des longueurs d'onde différentes ; et dans lequel la première longueur d'onde de détection et la seconde longueur d'onde de détection sont des longueurs d'onde différentes.

2. Procédé selon la revendication 1, dans lequel le premier nucléotide marqué, le deuxième nucléotide marqué et le troisième nucléotide marqué sont chacun marqués avec un seul colorant fluorescent.

3. Procédé selon l'une quelconque des revendications 1 ou 2, dans lequel le troisième nucléotide marqué est marqué avec un colorant à long décalage de Stokes, dans lequel le colorant à long décalage de Stokes présente un décalage de Stokes allant d'environ 300 meV à 850 meV.

4. Procédé selon l'une quelconque des revendications 1 ou 2, dans lequel le troisième nucléotide marqué est marqué avec un colorant à long décalage de Stokes, dans lequel le colorant à long décalage de Stokes présente une émission décalée d'environ 50 nm à 150 nm.

5. Procédé selon la revendication 4, dans lequel le colorant à long décalage de Stokes est excité à environ 300 nm.

6. Procédé selon l'une quelconque des revendications 1 ou 2, dans lequel le troisième nucléotide marqué est marqué avec un colorant chroménoquinoléine.

7. Procédé selon l'une quelconque des revendications 1 ou 2, dans lequel le premier nucléotide marqué est marqué avec le colorant NR455BoC.

8. Procédé selon l'une quelconque des revendications 1 ou 2, dans lequel le deuxième nucléotide marqué est marqué avec le colorant NR550S0.

9. Procédé selon l'une quelconque des revendications 1 ou 2, dans lequel le troisième nucléotide marqué est marqué avec le colorant MC485CQ-O.

10. Procédé selon l'une quelconque des revendications 1 à 9, dans lequel la première longueur d'onde de détection est imagée par un premier dispositif à couplage de charge, et la seconde longueur d'onde de détection est imagée par un second dispositif à couplage de charge.

11. Procédé selon l'une quelconque des revendications 1 ou 2, dans lequel l'excitation à la première longueur d'onde d'excitation et la détection d'émissions fluorescentes à la première longueur d'onde d'émission sont effectuées séparément de l'excitation à la première longueur d'onde d'excitation et de la détection d'émissions fluorescentes à la seconde longueur d'onde d'émission.

12. Procédé selon l'une quelconque des revendications 1 ou 2, dans lequel l'excitation à la première longueur d'onde d'excitation et la détection d'émissions fluorescentes à la première longueur d'onde d'émission sont effectuées en même temps que l'excitation à la première longueur d'onde d'excitation et la détection d'émissions fluorescentes à la seconde longueur d'onde d'émission.

13. Procédé selon l'une quelconque des revendications 1 ou 2, dans lequel la détection d'émissions fluorescentes du premier nucléotide marqué, du deuxième nucléotide marqué et du troisième nucléotide marqué est effectuée à l'aide d'un procédé d'imagerie par intégration à délai temporel (TDI).

14. Système de séquençage de polynucléotides liés à une cellule de flux, comprenant :
une mémoire lisible par machine, et
un processeur configuré pour exécuter des instructions lisibles par machine qui, lorsqu'elles sont exécutées par le processeur, amènent le système à effectuer des étapes selon l'une quelconque des revendications 1 à 13.

15. Support non transitoire lisible par ordinateur stockant un programme de séquençage de polynucléotides incluant des instructions qui, lorsqu'elles sont exécutées par un processeur, amènent un appareil de séquençage de polynucléotides à effectuer des étapes selon l'une quelconque des revendications 1 à 13.
